# EUROPEAN PATENT SPECIFICATION

(11) **EP 4 454 293 B1**
(45) Date of publication and mention of the grant of the patent: **05.08.2026**
(21) Application number: 22910327.0
(22) Date of filing: 20.12.2022
(51) Int. Cl.: H04R 25/00, A61N 1/05, A61N 1/36, A61N 1/37, A61N 1/39, A61N 1/32, A61N 1/362, A61N 1/375

(54) **ADVANCED RETENTION MAGNET SYSTEM FOR MEDICAL DEVICE**
VERBESSERTES HALTEMAGNETSYSTEM FÜR MEDIZINISCHE VORRICHTUNG
SYSTÈME D'AIMANT DE RÉTENTION AVANCÉ POUR DISPOSITIF MÉDICAL

(30) Priority: 20.12.2021 US 202163291682 P
(43) Date of publication of application: 30.10.2024
(73) Proprietor: Cochlear Limited, Macquarie University, New South Wales 2109 (AU)
(72) Inventor: FUNG, Wilson, Macquarie University, New South Wales 2109 (AU); POWELL, Anthony, Macquarie University, New South Wales 2109 (AU); DIOLASO, Irene Tsimos, Macquarie University, New South Wales 2109 (AU); LEIGH, Charles Roger Aaron, Macquarie University, New South Wales 2109 (AU); MCINNES, Timothy, Macquarie University, New South Wales 2109 (AU)
(74) Representative: Grünecker Patent- und Rechtsanwälte PartG mbB
(86) International application number: PCT/IB2022/062541
(87) International publication number: WO 2023/119156

(56) References cited:
- US-A1- 2011 224 756
- US-A1- 2012 296 155
- US-A1- 2014 343 626
- US-A1- 2016 317 810
- US-A1- 2018 369 586
- US-A1- 2020 114 151
- US-A1- 2021 058 718
- US-A1- 2021 235 209

## Description

### BACKGROUND

US 2021/235209 A1 discloses an external portion of an auditory prosthesis which includes an external magnet that interacts with an implantable magnet to hold the external portion against the skin. Additional elements disposed within portions of the auditory prosthesis are utilized to redirect the magnetic flux.
US 2020/114151 A1 discloses a cochlear implant including a magnet assembly. The magnet assembly has a spine and at least one magnet that is secured to the spine.
US 2011/224756 A1 discloses an implantable magnet that can freely turn in response to an external magnetic field.
Medical devices have provided a wide range of therapeutic benefits to recipients over recent decades. Medical devices can include internal or implantable components/devices, external or wearable components/devices, or combinations thereof (e.g., a device having an external component communicating with an implantable component). Medical devices, such as traditional hearing aids, partially or fully-implantable hearing prostheses (e.g., bone conduction devices, mechanical stimulators, cochlear implants, etc.), pacemakers, defibrillators, functional electrical stimulation devices, and other medical devices, have been successful in performing lifesaving and/or lifestyle enhancement functions and/or recipient monitoring for a number of years.

The types of medical devices and the ranges of functions performed thereby have increased over the years. For example, many medical devices, sometimes referred to as "implantable medical devices," now often include one or more instruments, apparatus, sensors, processors, controllers or other functional mechanical or electrical components that are permanently or temporarily implanted in a recipient. These functional devices are typically used to diagnose, prevent, monitor, treat, or manage a disease/injury or symptom thereof, or to investigate, replace or modify the anatomy or a physiological process. Many of these functional devices utilize power and/or data received from external devices that are part of, or operate in conjunction with, implantable components.

### SUMMARY

A device according to the invention includes the features defined in claim 1.

Embodiments include the features defined in the dependent claims.

### BRIEF DESCRIPTION OF THE DRAWINGS

FIG. 1 depicts a partial perspective view of a percutaneous bone conduction device worn on a recipient.
FIG. 2 depicts a cross-sectional schematic view of a passive transcutaneous bone conduction device worn on a recipient.
FIG. 2A depicts a top view of an exemplary embodiment.
FIG. 2B depicts a side view of an exemplary embodiment.
FIG. 2C depicts a side view of an exemplary embodiment.
FIG. 2D depicts an alternate technology to which some teachings herein are applicable.
FIG. 3A depicts a partial cross-sectional schematic view of a passive transcutaneous bone conduction device worn on a recipient.
FIG. 3B depicts a partial cross-sectional schematic view of a passive transcutaneous bone conduction device utilizing magnet groups, worn on a recipient.
FIGs. 4A and 4B are perspective views of an exemplary magnets.
FIG. 5A is a perspective view of an exemplary magnets.
FIGs. 5B-6C variously depict exemplary magnets.
FIG. 7A depicts an exemplary magnet apparatus in magnetic communication with an external magnet.
FIGs. 7B-D depict an exemplary magnet apparatus.
FIGs. 8A-B, 9, 10, 11, 14 and 15 depict exemplary magnet apparatuses.
FIGs. 12 and 12A and 12B depict an exemplary magnet apparatus in magnetic communication with an external magnet.
FIG. 13 depicts an exemplary magnet apparatus.
FIGs. 16 and 17 and 19 depict exemplary magnet apparatuses.
FIG. 18 depicts a framework for evaluating magnet rotation.
FIG. 20 is an algorithm for an exemplary embodiment.
FIGs. 21-26B present exemplary arrangements of magnetic bodies.
FIGs. 27-35 depict exemplary magnet apparatuses.

### DETAILED DESCRIPTION

Merely for ease of description, the techniques presented herein are sometimes described herein with reference to an illustrative medical device, namely a cochlear stimulator, and in other instances, a cochlear implant. However, it is to be appreciated that the techniques presented herein may also be used with a variety of other medical devices that, while providing a wide range of therapeutic benefits to recipients, patients, or other users, may benefit from setting changes based on the location of the medical device. For example, the techniques presented herein may be used with other hearing prostheses, including acoustic hearing aids, bone conduction devices, middle ear auditory prostheses, direct acoustic stimulators, other electrically simulating auditory prostheses (e.g., auditory brain stimulators), etc. Some embodiments include the utilization of the teachings herein to treat an inner ear of a recipient that has and/or utilizes one or more of these devices. The techniques presented herein may also be used with vestibular devices (e.g., vestibular implants), visual devices (i.e., bionic eyes), sensors, pacemakers, drug delivery systems, defibrillators, functional electrical stimulation devices, catheters, seizure devices (e.g., devices for monitoring and/or treating epileptic events), sleep apnea devices, electroporation, etc. In further embodiments, the techniques presented herein may be used with air purifiers or air sensors (e.g., automatically adjust depending on environment), hospital beds, identification (ID) badges/bands, or other hospital equipment or instruments.

The teachings detailed herein can be implemented in sensory prostheses, such as hearing implants specifically, and neural stimulation devices in general. Other types of sensory prostheses can include retinal implants. Accordingly, any teaching herein with respect to a sensory prosthesis corresponds to a disclosure of utilizing those teachings in / with a hearing implant and in / with a retinal implant, unless otherwise specified, providing the art enables such. Moreover, with respect to any teachings herein, such corresponds to a disclosure of utilizing those teachings with all of or parts of a cochlear implant, cochlear stimulator, a bone conduction device (active and passive transcutaneous bone conduction devices, and percutaneous bone conduction devices) and a middle ear implant, providing that the art enables such, unless otherwise noted. To be clear, any teaching herein with respect to a specific sensory prosthesis corresponds to a disclosure of utilizing those teachings in / with any of the aforementioned hearing prostheses, and *vice versa.* Corollary to this is at least some teachings detailed herein can be implemented in somatosensory implants and/or chemosensory implants. Accordingly, any teaching herein with respect to a sensory prosthesis corresponds to a disclosure of utilizing those teachings with/in a somatosensory implant and/or a chemosensory implant.

Thus, merely for ease of description, the first illustrative medical device is a hearing prosthesis. Any techniques presented herein described for one type of hearing prosthesis or any other device disclosed herein corresponds to a disclosure of another embodiment of using such teaching with another device (and/or another type of hearing device including other types of bone conduction devices (active transcutaneous and/or passive transcutaneous), middle ear auditory prostheses (particularly, the EM vibrator / actuator thereof), direct acoustic stimulators), etc. The techniques presented herein can be used with implantable / implanted microphones (where such is a transducer that receives vibrations and outputs an electrical signal (effectively, the reverse of an EM actuator), whether or not used as part of a hearing prosthesis (e.g., a body noise or other monitor, whether or not it is part of a hearing prosthesis) and/or external microphones. The techniques presented herein can also be used with vestibular devices (e.g., vestibular implants), sensors, seizure devices (e.g., devices for monitoring and/or treating epileptic events, where applicable), and thus any disclosure herein is a disclosure of utilizing such devices with the teachings herein (and *vice versa*)*,* providing that the art enables such. The teachings herein can also be used with conventional hearing devices, such as telephones and ear bud devices connected MP3 players or smart phones or other types of devices that can provide audio signal output, that use an EM transducer. Indeed, the teachings herein can be used with specialized communication devices, such as military communication devices, factory floor communication devices, professional sports communication devices, etc.

By way of example, any of the technologies detailed herein which are associated with components that are implanted in a recipient can be combined with information delivery technologies disclosed herein, such as for example, devices that evoke a hearing percept, to convey information to the recipient. By way of example only and not by way of limitation, a sleep apnea implanted device can be combined with a device that can evoke a hearing percept so as to provide information to a recipient, such as status information, etc. In this regard, the various sensors detailed herein and the various output devices detailed herein can be combined with such a non-sensory prosthesis or any other nonsensory prosthesis that includes implantable components so as to enable a user interface, as will be described herein, that enables information to be conveyed to the recipient, which information is associated with the implant.

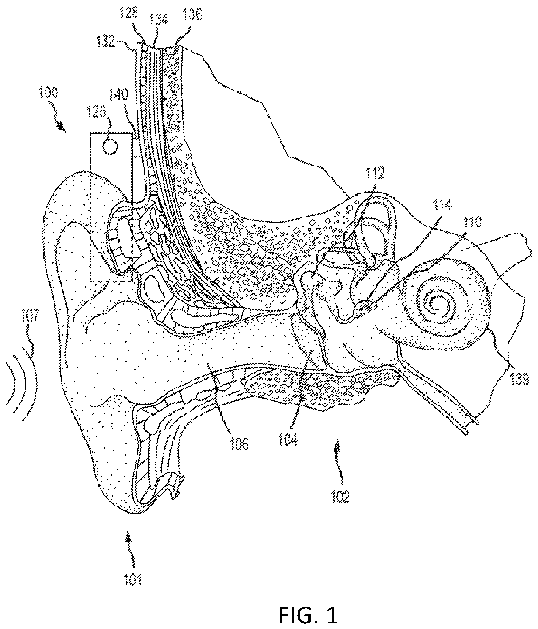

FIG. 1 depicts a partial perspective view of a percutaneous bone conduction device 100 positioned behind outer ear 101 of the recipient and includes a sound input element 126 to receive sound signals 107. The sound input element 126 can be a microphone, telecoil, or similar. In the present example, sound input element 126 can be located, for example, on or in bone conduction device 100, or on a cable extending from bone conduction device 100. Also, bone conduction device 100 includes a sound processor (not shown), a vibrating electromagnetic actuator and/or various other operational components.

More particularly, sound input device 126 converts received sound signals into electrical signals. These electrical signals are processed by the sound processor. The sound processor generates control signals that cause the actuator to vibrate. In other words, the actuator converts the electrical signals into mechanical force to impart vibrations to skull bone 136 of the recipient.

Bone conduction device 100 further includes coupling apparatus 140 to attach bone conduction device 100 to the recipient. In the example of FIG. 1A, coupling apparatus 140 is attached to an anchor system (not shown) implanted in the recipient. An exemplary anchor system (also referred to as a fixation system) can include a percutaneous abutment fixed to the recipient's skull bone 136. The abutment extends from skull bone 136 through muscle 134, fat 128, and skin 132 so that coupling apparatus 140 can be attached thereto. Such a percutaneous abutment provides an attachment location for coupling apparatus 140 that facilitates efficient transmission of mechanical force.

It is noted that sound input element 126 can include devices other than a microphone, such as, for example, a telecoil, etc. In an exemplary embodiment, sound input element 126 can be located remote in a BTE device (not shown) supported by the ear and in communication with the bone conduction device 100 via a cable. Alternatively, sound input element 126 can be subcutaneously implanted in the recipient, or positioned in the recipient's ear canal or positioned within the pinna. Sound input element 126 can also be a component that receives an electronic signal indicative of sound, such as, from an external audio device. For example, sound input element 126 can receive a sound signal in the form of an electrical signal from an MP3 player or a smartphone electronically connected to sound input element 126.

The sound processing unit of the auditory prosthesis processes the output of the sound input element 126, which is typically in the form of an electrical signal. The processing unit generates control signals that cause an associated actuator to vibrate. These mechanical vibrations are delivered by an external portion of the auditory prosthesis 100, as described below.

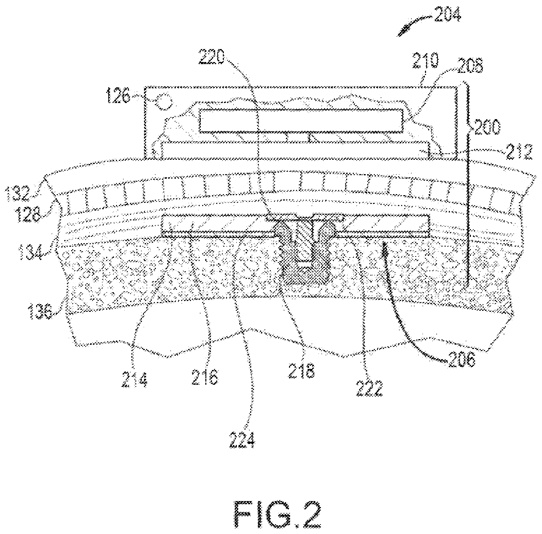

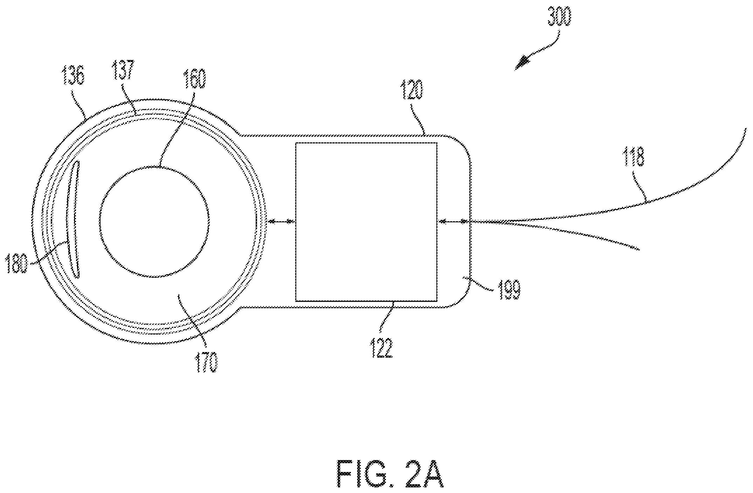

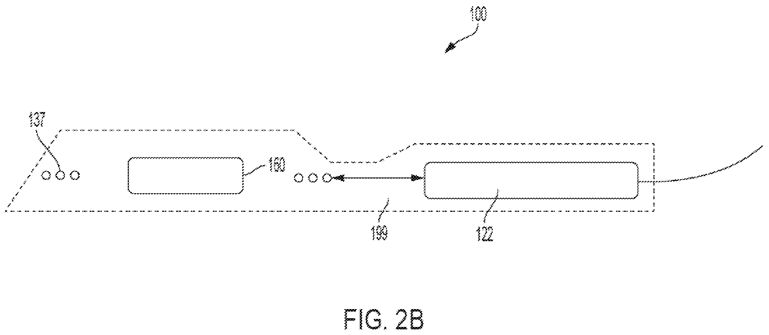

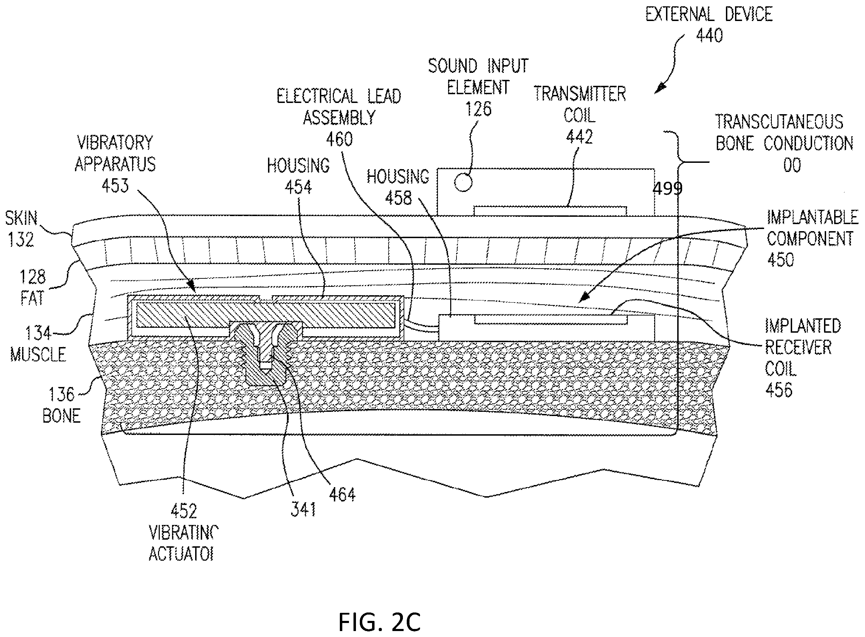

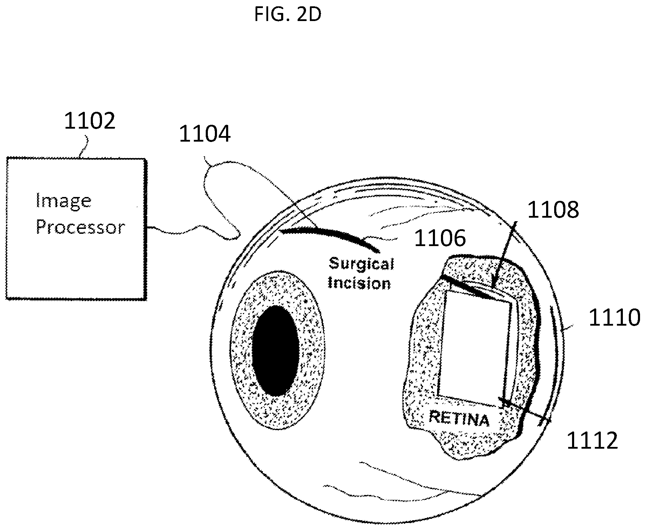

FIG. 2 depicts an example of a transcutaneous bone conduction device 200 that includes an external portion 204 and an implantable portion 206. The transcutaneous bone conduction device 200 of FIG. 2 is a passive transcutaneous bone conduction device in that a vibrating actuator 208 is located in the external portion 204. Vibrating actuator 208 is located in housing 210 of the external component, and is coupled to plate 212. Plate 212 can be in the form of a permanent magnet, a group of magnets, and/or in another form that generates and/or is reactive to a magnetic field, or otherwise permits the establishment of magnetic attraction between the external portion 204 and the implantable portion 206 sufficient to hold the external portion 204 against the skin of the recipient. Magnetic attraction can be further enhanced by utilization of a magnetic implantable plate 216. A single external magnet 212 of a first polarity and a single implantable magnet 216 of a second polarity, are depicted in FIG. 2. In alternative embodiments, two magnets in both the external portion 204 and implantable portion 206 can be utilized. In a further alternative embodiment, the plate 212 can include an additional plastic or biocompatible housing (not shown) that encapsulates plate 212 and contacts the skin of the recipient.

The vibrating actuator 208 is a device that converts electrical signals into vibration. In operation, sound input element 126 converts sound into electrical signals. Specifically, the transcutaneous bone conduction device 200 provides these electrical signals to vibrating actuator 208, or to a sound processor (not shown) that processes the electrical signals, and then provides those processed signals to vibrating actuator 208. The vibrating actuator 208 converts the electrical signals into vibrations. Because vibrating actuator 208 is mechanically coupled to plate 212, the vibrations are transferred from the vibrating actuator 208 to plate 212. Implantable plate assembly 214 is part of the implantable portion 206, and is made of a ferromagnetic material that can be in the form of a permanent magnet, that generates and/or is reactive to a magnetic field, or otherwise permits the establishment of a magnetic attraction between the external portion 204 and the implantable portion 206 sufficient to hold the external portion 204 against the skin 132 of the recipient. Additional details regarding the magnet groups that can be utilized in both the external portion 204 and the implantable portion 206 are described in more detail herein. Accordingly, vibrations produced by the vibrating actuator 208 of the external portion 204 are transferred from plate 212 across the skin 132 to implantable plate 216 of implantable plate assembly 214. This can be accomplished as a result of mechanical conduction of the vibrations through the skin 132, resulting from the external portion 204 being in direct contact with the skin 132 and/or from the magnetic field between the two plates 212, 216. These vibrations are transferred without a component penetrating the skin 132, fat 128, or muscular 134 layers.

As can be seen, the implantable plate assembly 214 is substantially rigidly attached to bone fixture 220 in this embodiment. Implantable plate assembly 214 includes through hole 220 that is contoured to the outer contours of the bone fixture 218, in this case, a bone screw that is secured to the bone 136 of the skull. This through hole 220 thus forms a bone fixture interface section that is contoured to the exposed section of the bone fixture 218. In an exemplary embodiment, the sections are sized and dimensioned such that at least a slip fit or an interference fit exists with respect to the sections. Plate screw 222 is used to secure implantable plate assembly 214 to bone fixture 218. As can be seen in FIG. 2, the head of the plate screw 222 is larger than the hole through the implantable plate assembly 214, and thus the plate screw 222 positively retains the implantable plate assembly 214 to the bone fixture 218. In certain embodiments, a silicone layer 224 is located between the implantable plate 216 and bone 136.

FIG. 2A depicts an exemplary high-level diagram of another exemplary prosthesis including an implantable component 300 of a so-called cochlear implant system, which can be a totally implantable system or a system with an external component (sound processor, RF antenna, microphone, etc. - more on this below) and the implantable component 300, looking downward from outside the skull towards the skull. As can be seen, implantable component 300 includes a magnet apparatus 160 that is surrounded by a coil 137 that is in two-way communication (although in some instances, the communication is one-way) with a stimulator unit 122, which in turn is in communication with the electrode assembly 118. This is basically a classic implantable component of a so-called cochlear implant.

Still with reference to FIG. 2A, it is noted that the stimulator unit 122, and the magnet apparatus 160 are located in a body made of an elastomeric material 199, such as by way of example only and not by way of limitation, silicone. Hereinafter, the elastomeric material 199 of the body will be often referred to as silicone. However, it is noted that any reference to silicone herein also corresponds to a reference to any other type of component that will enable the teachings detailed herein and/or variations thereof, such as, by way of example and not by way of limitation only, bio-compatible rubber, etc.

As can be seen in FIG. 2A, the housing made of elastomeric material 199 includes a slit 180 (not shown in FIG. 2B, as, in some instances, the slit is not utilized, and in other instances, the slit is located elsewhere - more on this below). In some variations, the slit 180 has utilitarian value in that it can enable insertion and/or removal of the magnet apparatus 160 from the body made of elastomeric material 199, such as for MRI treatment. The magnet apparatus is surrounded by silicone 170 of the silicone body 199, and the silicone holds the magnet apparatus in place, and also supports the coil.

It is noted that magnet apparatus 160 is presented in a conceptual manner. In this regard, it is noted that in at least some instances, the magnet apparatus 160 is an assembly that includes a magnet surrounded by a biocompatible coating. Still further by way of example, magnet apparatus 160 is an assembly where the magnet is located within a container having interior dimensions generally corresponding to the exterior dimensions of the magnet, although in other embodiments, this is not the case. This container can be hermetically sealed, thus isolating the magnet in the container from body fluids of the recipient that penetrate the housing (the same principle of operation occurs with respect to the aforementioned coated magnet). In an exemplary embodiment, this container permits the magnet to revolve or otherwise move relative to the container, as is known in the art. Additional details of the container will be described below. In this regard, it is noted that while sometimes the term magnet is used as shorthand for the phrase magnet apparatus, and thus any disclosure herein with respect to a magnet also corresponds to a disclosure of a magnet apparatus according to the aforementioned embodiments and/or variations thereof and/or any other configuration that can have utilitarian value according to the teachings detailed herein.

With reference now to FIG. 2B it is noted that the outlines of the silicone body made from elastomeric material 199 are presented in dashed line format for ease of discussion. In an exemplary embodiment, silicone or some other elastomeric material fills the interior within the dashed line, other than the other components of the implantable device (e.g., plates, magnet, stimulator, etc.). That said, in an alternative embodiment, silicone or some other elastomeric material substantially fills the interior within the dashed lines other than the components of the implantable device (e.g., there can be pockets within the dashed line in which no components and no silicone are located).

It is noted that FIGs. 2A and 2B are conceptual figures presented for purposes of discussion. Commercial embodiments corresponding to these FIGs. can be different from that depicted in the figures.

FIG. 2C depicts an exemplary embodiment of a transcutaneous bone conduction device 499 according to another embodiment that includes an external device 440 and an implantable component 450. The transcutaneous bone conduction device 499 of FIG. 2C is an active transcutaneous bone conduction device in that the vibrating actuator 452 (which can be an electromagnetic actuator, or a piezoelectric actuator, etc.) is located in the implantable component 450. Specifically, a vibratory element in the form of vibrating actuator 452 is located in housing 454 of the implantable component 450. In an exemplary embodiment, much like the vibrating actuator 342 described above with respect to transcutaneous bone conduction device 300, the vibrating actuator 452 is a device that converts electrical signals into vibration.

External component 440 includes a sound input element 126 that converts sound into electrical signals. Specifically, the transcutaneous bone conduction device 499 provides these electrical signals to vibrating actuator 452, or to a sound processor (not shown) that processes the electrical signals, and then provides those processed signals to the implantable component 450 through the skin of the recipient via a magnetic inductance link. **In** this regard, a transmitter coil 442 of the external component 440 transmits these signals to implanted receiver coil 456 located in housing 458 of the implantable component 450. Components (not shown) in the housing 458, such as, for example, a signal generator or an implanted sound processor, then generate electrical signals to be delivered to vibrating actuator 452 via electrical lead assembly 460. The vibrating actuator 452 converts the electrical signals into vibrations.

The vibrating actuator 452 is mechanically coupled to the housing 454. Housing 454 and vibrating actuator 452 collectively form a vibratory apparatus 453. The housing 454 is substantially rigidly attached to bone fixture 341.

As with the embodiments above, the external device 440 is held against the skin via magnetic attraction between a ferromagnetic body in the external device 440 and the implantable component 450, such as in the implanted receiver coil apparatus 456.

FIG. 2D presents an exemplary embodiment of a neural prosthesis in general, and a retinal prosthesis and an environment of use thereof, in particular. In some embodiments of a retinal prosthesis, a retinal prosthesis sensor-stimulator 1108 is positioned proximate the retina 1110. In an exemplary embodiment, photons entering the eye are absorbed by a microelectronic array of the sensor-stimulator 1108 that is hybridized to a glass piece 1112 containing, for example, an embedded array of microwires. The glass can have a curved surface that conforms to the inner radius of the retina. The sensor-stimulator 108 can include a microelectronic imaging device that can be made of thin silicone containing integrated circuitry that convert the incident photons to an electronic charge.

An image processor 1102 is in signal communication with the sensor-stimulator 1108 via cable 1104 which extends through surgical incision 1106 through the eye wall (although in other embodiments, the image processor 1102 is in wireless communication with the sensor-stimulator 1108). In an exemplary embodiment, the image processor 1102 is analogous to the sound processor / signal processors of the auditory prostheses detailed herein, and in this regard, any disclosure of the latter herein corresponds to a disclosure of the former in an alternate embodiment. The image processor 1102 processes the input into the sensor-stimulator 108, and provides control signals back to the sensor-stimulator 1108 so the device can provide processed and output to the optic nerve. That said, in an alternate embodiment, the processing is executed by a component proximate to or integrated with the sensor-stimulator 1108. The electric charge resulting from the conversion of the incident photons is converted to a proportional amount of electronic current which is input to a nearby retinal cell layer. The cells fire and a signal is sent to the optic nerve, thus inducing a sight perception.

The retinal prosthesis can include an external device disposed in a Behind-The-Ear (BTE) unit or in a pair of eyeglasses, or any other type of component that can have utilitarian value. The retinal prosthesis can include an external light / image capture device (e.g., located in / on a BTE device or a pair of glasses, etc.), while, as noted above, in some embodiments, the sensor-stimulator 1108 captures light / images, which sensor-stimulator is implanted in the recipient. In an exemplary embodiment, there is a transcutaneous communication coil that is held against a skin of a recipient via magnetic attraction to communication with an implanted component, which implanted component provides the stimulation to evoke a sight precept. In an embodiment, the teachings herein regarding magnetic attraction are utilized in such.

In the interests of compact disclosure, any disclosure herein of a microphone or sound capture device corresponds to an analogous disclosure of a light / image capture device, such as a charge-coupled device. Corollary to this is that any disclosure herein of a stimulator unit which generates electrical stimulation signals or otherwise imparts energy to tissue to evoke a hearing percept corresponds to an analogous disclosure of a stimulator device for a retinal prosthesis. Any disclosure herein of a sound processor or processing of captured sounds or the like corresponds to an analogous disclosure of a light processor / image processor that has analogous functionality for a retinal prosthesis, and the processing of captured images in an analogous manner. Indeed, any disclosure herein of a device for a hearing prosthesis corresponds to a disclosure of a device for a retinal prosthesis having analogous functionality for a retinal prosthesis. Any disclosure herein of fitting a hearing prosthesis corresponds to a disclosure of fitting a retinal prosthesis using analogous actions. Any disclosure herein of a method of using or operating or otherwise working with a hearing prosthesis herein corresponds to a disclosure of using or operating or otherwise working with a retinal prosthesis in an analogous manner.

The teachings detailed herein can be used in any of the embodiments disclosed above and/or in other medical devices.

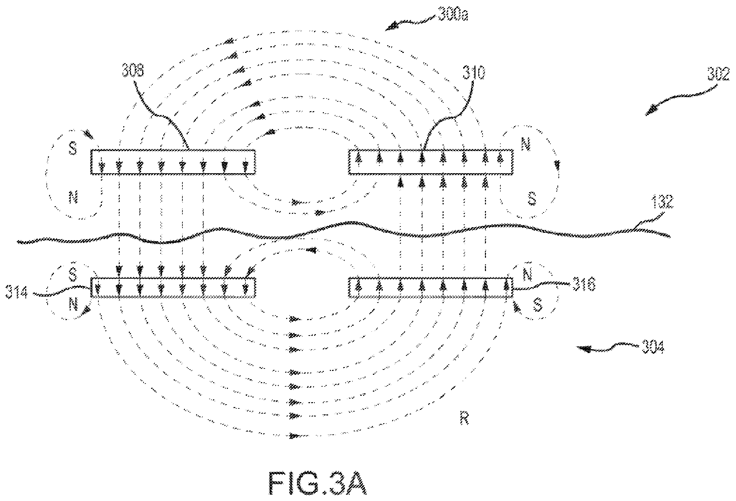

FIG. 3A depicts a partial cross-sectional schematic view of a magnet group 300a of an exemplary transcutaneous bone conduction device (or a headpiece for a transcutaneous communication inductance coil, in an alternate embodiment) for a recipient R. Only skin 132 of the recipient R is depicted for clarity. The bone conduction device includes external magnet portions 302 and an implantable magnet portions 304. These are depicted without any of the other components seen for purposes of clarity. Each of the external group 302 and the implantable group 304 include reciprocal groups of magnets that form a transcutaneous coupling between those groups 302, 304, via a closed magnetic circuit. Other components in the external portion and the implantable portion e.g., housings, sound processing components, batteries, microphones, actuators, anchors, etc., are described above, but not depicted in FIG. 3A. The external group 302 includes a plurality of external magnets 308, 310. **In** this embodiment, magnet 308 has a magnetization direction (e.g., as defined by the north and south poles thereof) that extends into the skin 132 of the recipient R, while magnet 310 has a magnetization direction that extends away from the skin 132. As such, these magnetization directions are substantially parallel and opposed to each other. In the illustrated example, the implantable portion 304 also includes two magnets 314, 316. Magnet 314 has a magnetization direction that is both substantially parallel to and harmonized with the magnetization direction of magnet 308, while magnet 316 has a magnetization direction that is both substantially parallel to and harmonized with the magnetization direction of magnet 310. The magnets 314, 316 can be disposed in a housing.

Magnetic flux generated by the magnets 308, 310, 314, 316 is also depicted in FIG. 3A. The magnetic field, and especially stray portions thereof, can interfere with the operation of the inductance communication coils that extend about the magnets in a device such as an active transcutaneous bone conduction device, or a cochlear implant, etc., that utilizes such to communicate with components implanted in the recipient, or the sound processor in general or other components disposed in the external portion. The performance of the vibrating actuator (if electromagnetic), such as in the case of a passive transcutaneous bone conduction device, which is typically in close proximity to the external magnets, can also be worsened by stray magnetic fields penetrating the actuator, thus reducing sensitivity and causing distortion.

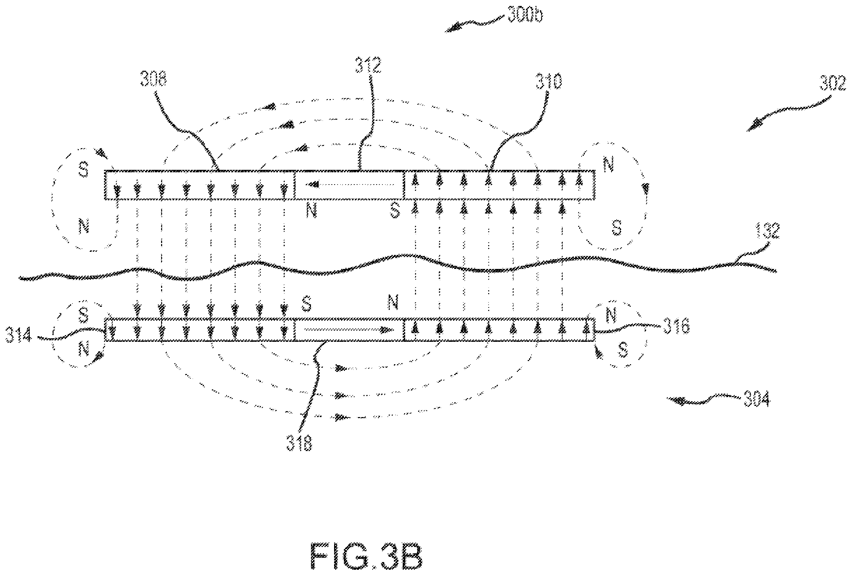

FIG. 3B depicts a partial cross-sectional schematic view of magnets 300b for a device such as, for example, a transcutaneous bone conduction device for a recipient R. This arrangement 300b utilizes additional magnets 312, 318, that have the utilitarian effect of reducing stray magnetic fields and otherwise improve performance. Utilization of magnets 312 and 318 can reduce interferences and further improve functionality of the auditory prosthesis or other device associated therewith. The magnetization direction of magnet 312 is substantially parallel and opposed to magnetization direction of magnet 318. Both of these magnetization directions are substantially parallel to the skin 132. The magnetic components 312, 318 divert the magnetic flux as depicted in FIG. 3B, to reduce the stray magnetic fields, thus correcting or minimizing the above-identified and other issues. Regardless of the number of magnets used, arranging the magnets 312, 318 such that the magnetization directions are in a circuit that defines a substantially continuous magnetic flux path in the medical device. In other words, the magnets 312, 318 create a shortcut for flux on that side of the medical device. As such, each of magnets 308, 310, 312, 314, 316, and 318 define a localized section of the flux path. By creating the circuit of magnetization direction, the magnetic flux is distributed asymmetrically on opposing sides of the medical device. This asymmetrical distribution, in practical terms, results in the retention force on one side of the magnets (e.g., 308 and 310) being increased and the magnetic interference on the other being reduced. Retention force is increased because the depicted arrangement of the magnets produces a flux concentration proximate the skin 132. In the depicted example, magnetic retention force proximate the skin 132 is increased, while magnetic interference away from the skin (e.g., where the sound processor, vibrating actuator, and other components are located) is decreased.

Each magnet in each magnet group generates its own magnetic field (as will be detailed below, an exemplary embodiment is such that portions 308, 312 and 310 are portions of a monolithic magnet. Together, magnets 308, 310, 312, 314, 316, and 318 form a magnet group (and generate a group magnetic field), although subsets of these magnets (e.g., magnets 308, 310, 312 in the external portion 302; and magnets 314, 316, 318 in the implantable portion 304) can also form magnet groups (and their own group magnetic fields). Moreover, the magnets in each magnet group need not be physically separate components, but can be a unitary part having different magnetization directions, which can be accomplished by the magnetization process. The effect on the magnetic field is depicted in FIG. 3B, where the field is channeled through the magnet 312, so as to reduce stray magnetic flux. Of course, magnet 318 channels the field so the stray flux generated by the implantable magnets 314, 316 is also reduced.

Magnets having differing form factors and magnetization directions are contemplated. For example, magnets that are diametrically magnetized and magnets that are axially magnetized are contemplated for applications such as bone conduction devices, to maintain a low profile of the auditory prosthesis. In the depicted embodiment, magnets 308, 310, 314, and 316 are axially magnetized so as to have a magnetization direction normal to a transcutaneous interface (i.e., the interface between the external portion and the implantable portion). The magnets 312, 318 are magnetized through the width so as to have a magnetization direction transverse to the magnetization direction of magnets 308, 310, 314, and 316. In examples where a unitary magnet is used, the unitary magnet can be magnetized such that portions thereof are diametrically magnetized, while other portions thereof are axially magnetized. Moreover, each magnet of a given magnet group can physically contact magnets proximate thereto so as to form a continuous flux path within the medical device (or the implanted component), if desired. Other configurations are contemplated and described in more detail below.

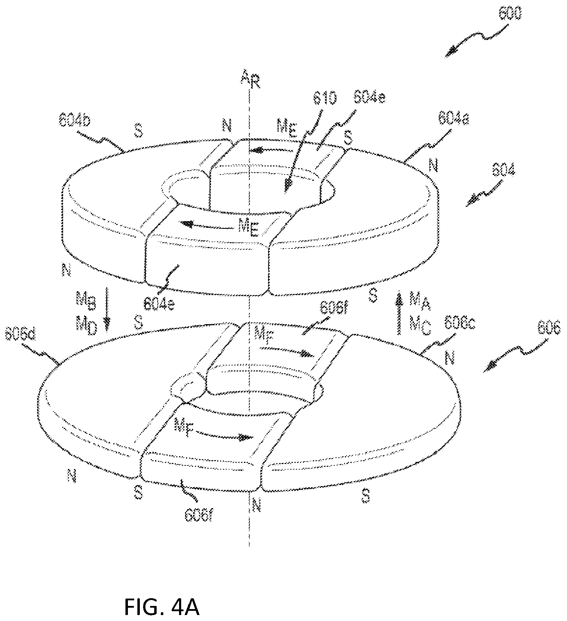

FIG. 4A is a perspective view of a magnet group 600 in accordance with one example of the technology. External magnet group includes magnets 604a and 604b, each having an arced form factor with two straight ends or edges, which magnets are axially magnetized. External magnet group 604 also includes a third magnet 604e, disposed between the ends of magnets 604a and 604b. In the depicted example, the third magnet 604e is in two parts, and, in that regard, can be considered to be two discrete magnets, disposed between different ends of magnets 604a and 604b. In other examples, magnet 604e can be configured as a single part, typically defining a gap 610 therein for receipt of a fixation screw 222 (as depicted in FIG. 2). Magnetization direction M_{E} is depicted, again, in a simplified form as a single vector substantially orthogonal to magnetization directions M_{A}, M_{B}. This magnetization direction M_{E} indicates that the north pole N of magnet 604e is disposed proximate magnet 604b, while the south pole S is disposed proximate magnet 604a. By orienting the poles as such, magnetic flux of the first magnet 604a is diverted more directly to the second magnet 604b, via the third magnet 604e. Similarly, magnet group 606 also includes a third magnet 606f, disposed between magnets 606c and 606d. In the depicted example, magnet 606f is in two parts, but in other examples, magnet 606f can be configured as a single part. Magnetization direction M_{F} is depicted, again, in a simplified form as a single vector substantially orthogonal to magnetization directions M_{C}, M_{D}. This magnetization direction M_{F} indicates that the north pole N of magnet 606f is disposed proximate magnet 606c, while the south pole S is disposed proximate magnet 606d. By orienting the poles as such, magnetic flux of the first magnet 606d is diverted more directly to the second magnet 606c, via the third magnet 606f. It should be noted that the magnetization directions M_{E} and M_{F} are both substantially parallel and opposed to each other.

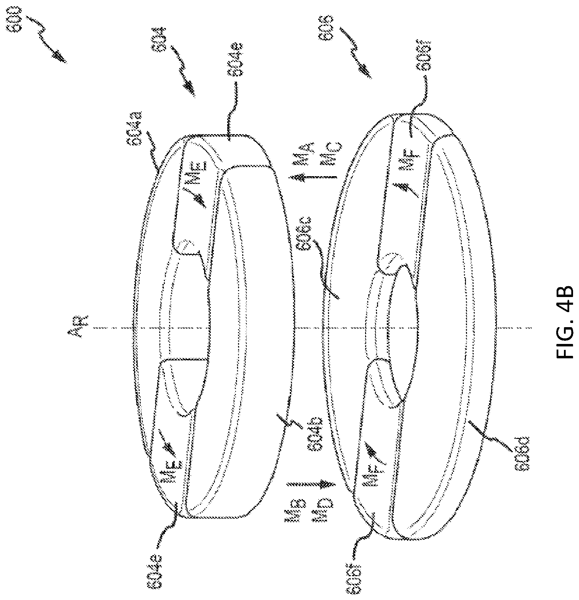

FIG. 4B is a perspective view of the magnet group 600' of FIG. 4A from a different angle. The components are generally numbered consistently with the components of FIG. 4A, and not all elements thereof are necessarily described further.

The magnets 604a, 604b, 604e of the external magnet group are disposed in a circuit that defines a substantially continuous flux path through the external component. Magnetic flux is channeled along the flux path following the magnetization direction of the respective magnets: from the first end magnet 604a, through the intermediate third magnet 604e, to the second end magnet 604b. This reduces the incidence of stray magnetic flux adjacent the magnet 604e.

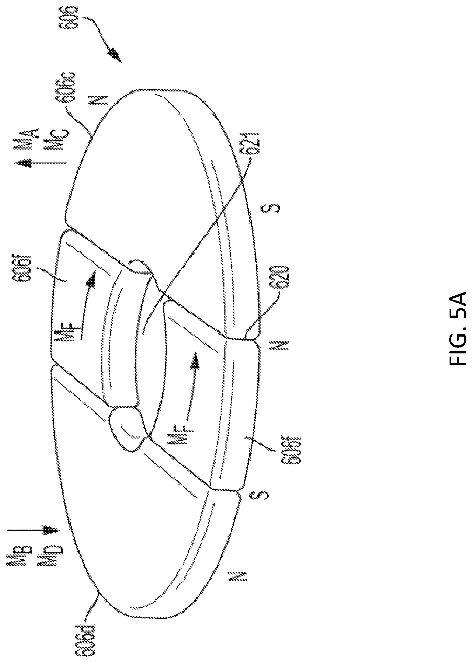

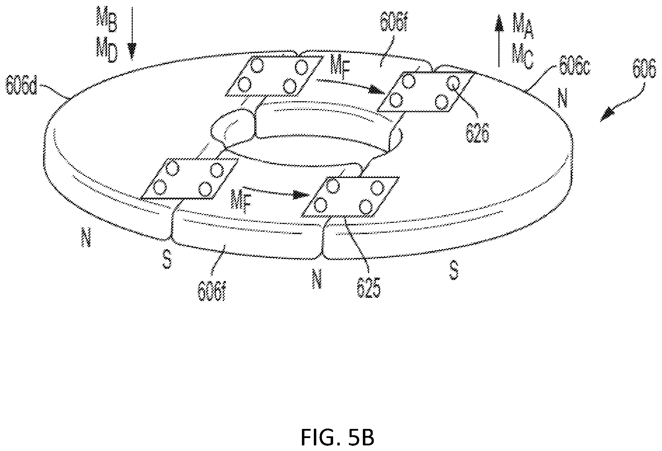

Figure 4A presents two separate elements 606f that are separated by a space. In an exemplary embodiment, the two separate elements are connected to one another by the magnet portions 606d and 606c, consistent with the embodiment of figure 4A. In an exemplary embodiment, adhesive or the like is utilized between the interfacing surfaces of the magnet portions 606d and 606c, and the respective corresponding portions of 606f. Figure 5A presents an exemplary location where adhesive 620 can be located at the facing surfaces of the respective magnet portions. FIG. 5B depicts another exemplary embodiment that utilizes plates 625 to hold the magnet elements 606f to the magnet portions 606c and 606d. In an exemplary embodiment, these can be plastic plates while in other embodiments these can be metallic plates. In an exemplary embodiment, the plates are bolted or screwed to the respective magnets utilizing bolts/screws 626, while in other embodiments, adhesive is utilized to glue the plates to the magnets. In an exemplary embodiment, there can be plates 625 located on the opposite sides (not shown). The bolts can extend all the way through to the opposite side plates connecting everything together. Any arrangement that can secure the plates to the magnet elements can be utilized in at least some exemplary embodiments.

In an exemplary embodiment, the magnet group is configured such that the first magnet portion, the second magnet portion and the third magnet portion establish a device such that the first portion and the third portion are contiguous, and the second portion and the third portion are contiguous. In an exemplary embodiment, a cross-section of the magnet group lying on a plane perpendicular to a longitudinal axis of the magnet group contains only the gap for the hole 621, while, with respect to other embodiments that will be described below, there are no gaps. In some embodiments, the magnet group is configured such that the first magnet portion, the second magnet portion and the third magnet portion are portions that are solid portions In an exemplary embodiment, the magnet group is configured such that the first magnet portion, the second magnet portion and the third magnet portion are portions that have solid cross-sections when taken on a plane perpendicular to a longitudinal axis of the magnet group, and the second magnetic field extends normal to the first and third magnetic fields, and the second magnet portion extends from one side of the group to an opposite side of the group.

In an exemplary embodiment, there are only the three portions that make up the magnet group. In an exemplary embodiment, there are only 2, 3, 4, 5, or 6 portions that make up the magnet group.

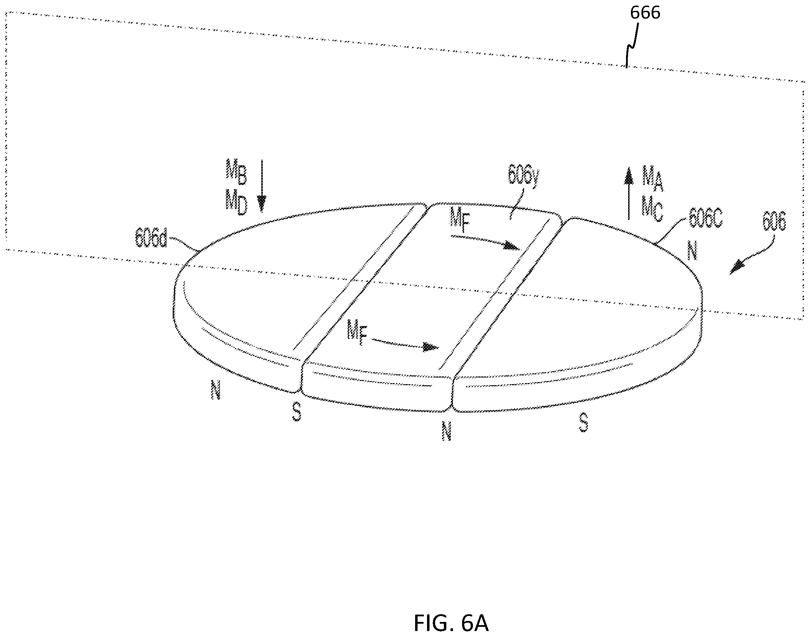

The above said, in some embodiments, there is no hole through the magnet(s). Figure 6A shows an exemplary embodiment of the magnet group where the third magnet portion 606y is a solid body without any through holes. Such an embodiment can be utilized with, for example, the implantable components where the devices that are utilized to fix the implantable component to the recipient in general, and to bone in particular, are located away from the magnet. Indeed, in an exemplary embodiment, the embodiment of figure 6A could be utilized in a device where there is no component per se that fixes the apparatus to the recipient. By way of example only and not by way of limitation, an implantable portion of a cochlear implant could be located in a recipient in a manner without any true positive retention of the implantable portion to the skull. Instead, in an exemplary embodiment, the pressure between the skin in the skull can be utilized to hold the implantable component in place, or at least the receiver stimulator thereof. In an exemplary embodiment, an excavation of the like in the skull can be utilized to hold the receiver stimulator in the lateral plane and the skin over the receiver stimulator can be utilized to hold the receiver stimulator into that excavation.

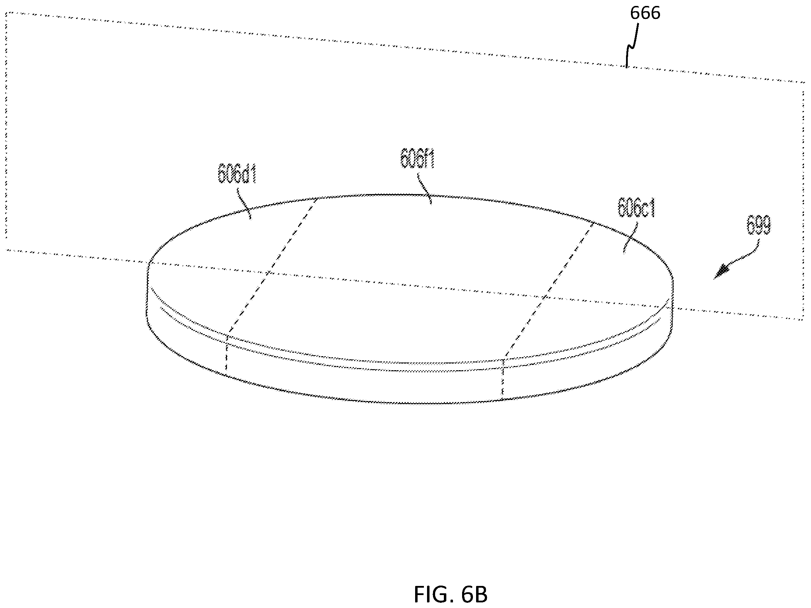

While the embodiments associated with some figures above have been described in terms of three separate magnet portions establishing the magnet group, other embodiments utilize a monolithic single magnet and/or a magnet that combines at least two of the portions into a monolithic component. In this regard, figure 6B depicts an exemplary alternate embodiment of a magnet portion group that comprises magnet portion 606d1, which corresponds to the first magnet portion detailed above and otherwise has the functionality of magnet 606d. The magnet group also comprises magnet portion 606c1, which corresponds to the second magnet portion detailed above and otherwise has the functionality of magnet 606c. In the middle is the magnet portion 606f1, which corresponds to the third magnet portion detailed above and otherwise has the functionality of magnet 606f. In an exemplary embodiment, the magnet group 699 is a monolithic disk that has a circular outer circumference and a height of less than, more than or equal to 0.1, 0.2, 0.3, 0.4, 0.5, 0.6, 0.7, 0.8, 0.9, 1.0, 1.1, 1.2, 1.3, 1.4, 1.5. 1.6. 1.7, 1.8. 1.9, 2.0, 2.1, 2.2, 2.3, 2.4, 2.5, 2.6. 2.7, 2.8, 2.9, 3.0, 3.5, 4, 4.5, 5, 5.5, 6, 7, 8, 9, or 10 or 11 or 12 or more mm or any value or range of values therebetween in 0.01 mm increments. The diameter can less than greater than or equal to 5, 5.5, 6, 6.5, 7, 7,5, 8, 8.5, 9, 9.5, 10, 11, 12, 13, 14, 15, 16, 17, 18, 19, 20, 21, 22, 23, 24, 25, 26, 27, 28, 29, or 30 mm or any value or range of values therebetween in 0.01 mm increments. This can also be the case for the embodiments above as well.

While the embodiment of figure 6B is presented as a monolithic disk without any through hole for the screw 222, in an alternate embodiment, there can be a through hole.

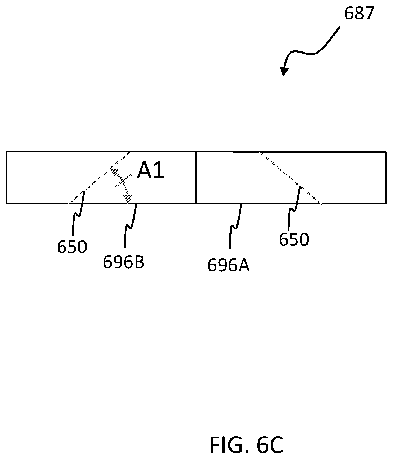

Figure 6C presents an exemplary cross-section of magnet 687 according to an exemplary variation of the apparatus of figure 6A taken through plane 666, which is a plane that lies on the longitudinal axis of magnet 606 / 687, and is taken through the central portion of magnet 606d and the central portion of magnet 606c (and through the central portion of magnet portion 606y), albeit with respect to this embodiment of FIG. 6C. Here, this is a four (4) pole magnet 666 (magnet group 666). In this embodiment, there are two separate magnet bodies - the bodies established by portions 696A and 696B (hereinafter, we use the phrase "magnet" to cover a single body and a plurality of magnet bodies that collectively form a magnet group). Figure 6C depicts the thickest sections (length from the longitudinal axis) of magnet portions 606d and 606c. Also shown in figure are the theoretical poles boundary line 650. This is the hypothetical line that divides the north pole from the south pole; everything on one side of the line is the hypothetical magnetic monopole north, and everything on the opposite side of the line is the hypothetical magnetic monopole south. As seen, in this exemplary embodiment, the surface area of magnet 696B is a north pole and faces the south pole of magnet portion 696A. Thus, the boundary line 650 establishes the magnets with a half south pole and half north pole. Here, the theoretical poles boundary lines 650 are canted. In an embodiment, the angle A1 from the bottom planar surface of the disk magnet (FIG. 6C is a cross-section of a disk magnet) can be 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69 or 70 degrees or any value or range of values therebetween in 0.1 degree increments (e.g., 27, 55, 33.3 to 66.6, etc.). In an embodiment, the magnet is symmetric in the plane shown (each magnet is a mirror image of the other).

Magnet 687 can be utilized as the implantable magnet in any of the embodiments described above and/or below. Moreover, the magnets of the various figures can be used as the magnet of the external component, and embodiments include, for example, a system with an implanted device and an external device, respectively having the various magnets disclosed herein in an arrangement such that the external device is attracted to the implanted device when the external device is worn against the head of the human. While focus on some embodiments will sometimes be described in terms of the utilization of magnet 687 in the implantable component of a medical device. It is noted that other embodiments can utilize other types of magnets such as those described herein and other types as well, and will often be described in terms of such. It is noted that any of the disclosure described above does not constitute structure corresponding to the innovative features of the present invention, but instead provides a framework for those teachings as will be described below. Accordingly, "means for" language does not cover those descriptions per se without one or more features of the below. That is not to say that the above is not used with "means for." That is to say that, for example, a means for providing magnetic retention of an external component would not cover apparatus 160, but would cover apparatus 760 as will now be described.

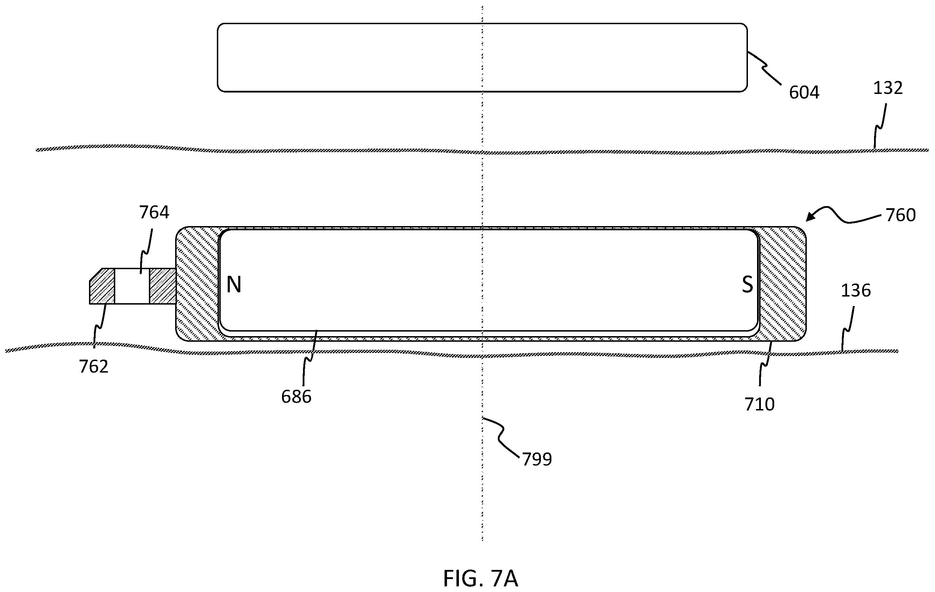

And with respect to figure 7A, there can be seen a magnet apparatus 760 that is configured to be utilized inside a human body and otherwise implanted in the human body. In an exemplary embodiment, the functionality of the magnet apparatus 760 corresponds to that of the magnet apparatus 160 detailed above. Here, magnet apparatus 760 includes a housing 710 that houses a diametrically magnetized planar disk magnet 686 (this is presented as a single body that has a North-South pole 90 degrees from the longitudinal axis 799 - embodiments can instead utilize the magnet 687 of FIG. 6C). Magnet apparatus 760 includes a tab 762 including a through bore 764. In an exemplary embodiment, this has utilitarian value with respect to enabling a hook or the like that is passed through the above-noted slot in the silicone body to talk into the bore 764 to enable the magnet apparatus 760 to be extracted from the housing body. The magnet apparatus can be considered a cassette that can be removed from the rest of the implant for MRI purposes (and later replaced, or a new one can be placed in the implant) without having to explant the remainder of the implant.

Not shown in figure 7A is the silicone body and other components of the implanted component. Also not shown in figure 7A is the external component. In reality, a silicone body could surround the magnet apparatus 760 (thus supporting the magnet apparatus above the surface of bone 136). Also in reality, structure of the external component would be supporting the external magnet 604.

The housing 710 hermetically isolates the magnet 686 that is located inside the interior cavity of the housing 710. The magnet 710 is depicted as being in contact with the housing wall at the top of the housing 710. This is owing to the magnetic attraction between the magnet 604 of the external component and the magnet 686. In this embodiment, the cavity of the housing 710 is dimensionally larger in all dimensions then the dimensions of the magnet 686. This means that there is a space between at least one longitudinal side of the magnet (top or bottom with respect to the view of figure 7A) and a space between the lateral side of the magnet (at least with respect to the cross-section shown in figure 7A - in reality, if one side was contacting the sidewall of the housing, because the cross-section of the cavity is circular, there would be only line contact in the vertical direction in a purely dimensionally stable structure).

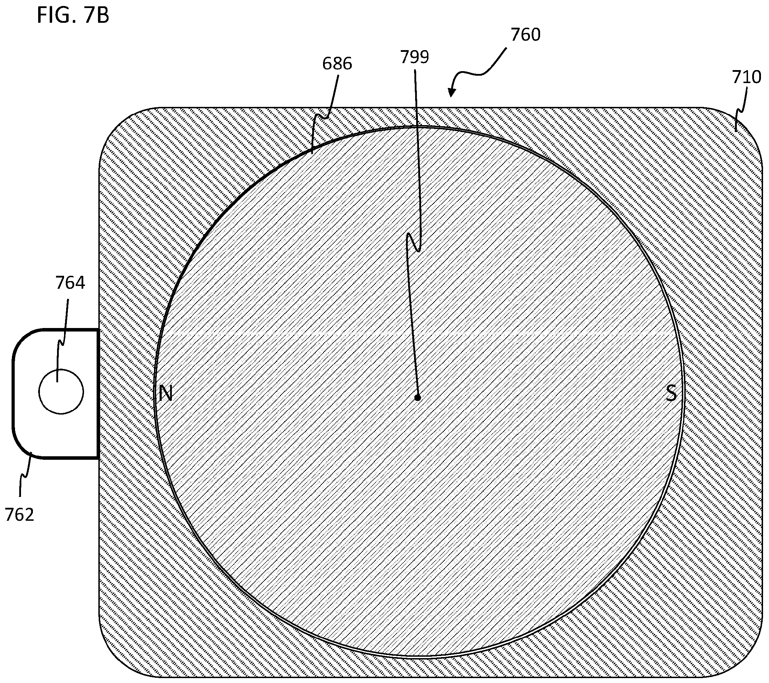

Figure 7B depicts a top view of the magnet apparatus 760. As can be seen, there is a slight space between the magnet 686 and the sidewall of the housing 710. With respect to the position of the magnet depicted in figure 7B, there is a space all the way around the outer periphery of the magnet, but again, the magnet can be located to the left for example, thus creating line contact at one point in effectively doubling the width of the space opposite such. And referring back to figure 7A, because the magnet 686 is being pulled upward by the magnet 604, there is no space the top of the magnet, but double the space at the bottom.

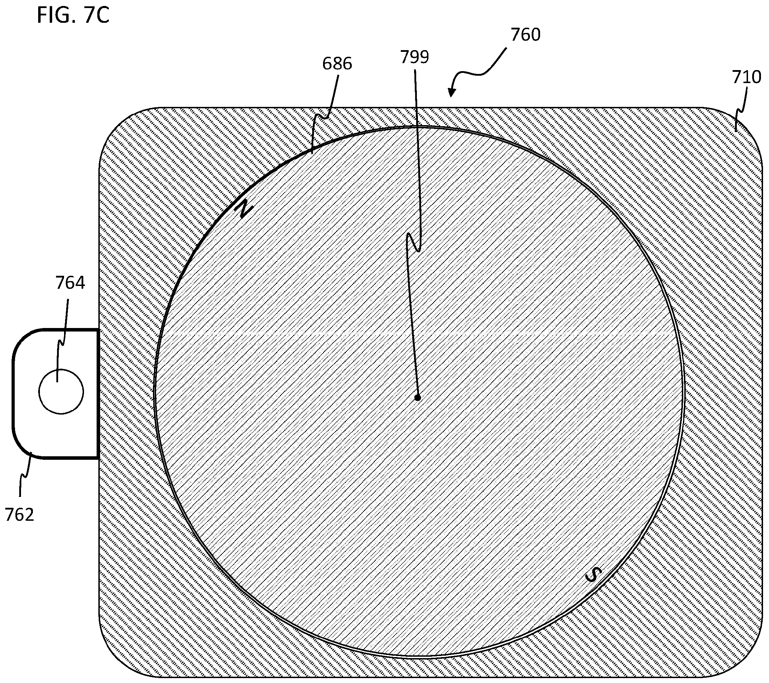

In this exemplary embodiment, the magnet apparatus 760 is configured to enable the magnet 686 to rotate about the longitudinal axis of the magnet 799. This is depicted by way of example only and not by way of limitation in figure 7C by comparison to figure 7B. In an exemplary embodiment, the magnet 604 is also diametrically magnetized or otherwise has a magnetic flux that is not purely axial. Accordingly, depending on the orientation about the longitudinal axis of the disk magnet 604, the poles of the implanted magnet may not align unless the magnet rotates or otherwise is rotatable. In this exemplary embodiment, the magnet can rotate within the cavity of the housing 710 owing to the forces applied to the implanted magnet 686 from the external magnet 604. In an exemplary embodiment, the poles may not necessarily align perfectly even after the rotation of the implanted magnet 686, but will align sufficiently so as to provide sufficient retention between the actual component in the implanted component. The ability of the magnet to rotate can have utilitarian value such as when the magnet is exposed to an MRI procedure magnetic field, thus making the device MRI compliant, at least if the rotation allows the implanted magnet to align with the magnetic field of an MRI machine, embodiments include methods according to such and include an MRI compatible implant. It can also have utilitarian value with respect to allowing an external device with a non-rotating magnet, such as is the case with respect to some embodiments) to be attached to the head in the correct orientation.

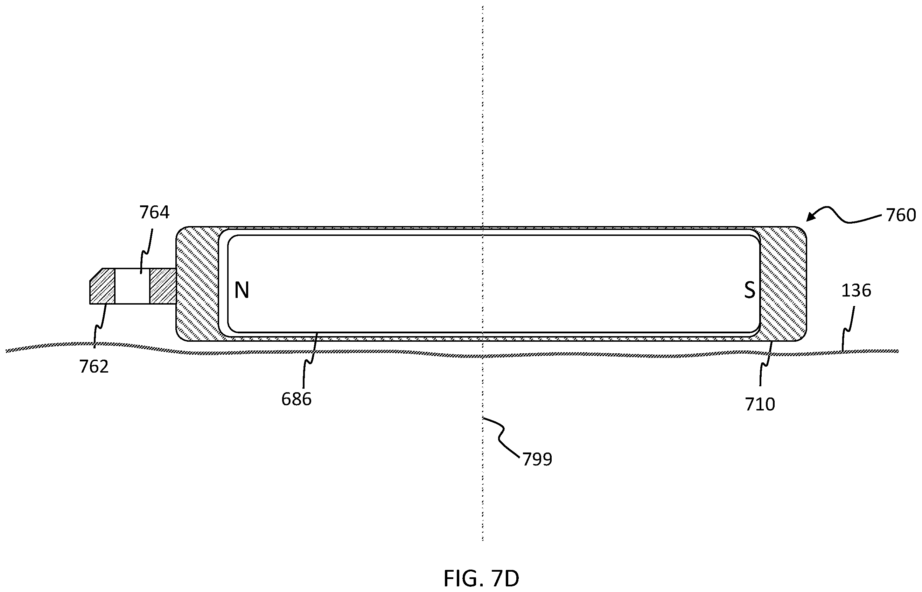

In embodiments of figure 7A and variations thereof, a top portion of the housing 710 is made of a magnetic material, such as a ferromagnetic material. In an embodiment, the magnetic material is not magnetized. This can enable the magnet 686 to be retained in the position shown in figure 7A after the external component is removed and otherwise after the external magnet 604 no longer magnetically interacts with the implanted magnet 686. In an alternate embodiment, a bottom portion of the housing is made of a magnetic material, which magnetic material may or may not be magnetized, and this can enable the magnet 686 to be retained at the bottom of the cavity in the housing as opposed to the top (the configuration shown in FIG. 7A). In an exemplary embodiment, the entire housing can be made of a magnetic material, and thus the magnet 686 can be retained at the bottom of the housing or the top of the housing or even against the side of the housing as shown in figure 7D. Here, it can be seen that there is a space between the magnet and the bottom housing wall, and the magnet and the top housing wall. In this exemplary embodiment, the sidewall of the housing could be made of a magnetic material, while in an alternate embodiment, the entire housing could be made of a magnetic material.

The above said, the magnetic material could itself have a magnetization applied to the material so that it either attracts or repels the implant magnet. (The magnetization could be simple, such as two poles, or could be a flat magnet with repeating N/S poles. Any arrangement that can have utilitarian value to enable the teachings herein can be used. As will be detailed below, the material can be in the hermetic magnet casing, or outside the magnetic casing, or both, and all can be hermetically isolated from the environment. To be clear, the magnetic material (such as the shim disclosed herein) used to establish retention / attraction of the implanted manet to the housing can be magnetized or not magnetized. Any material that can be used to establish a magnetic attraction between the implant magnet and the housing or other portion of the implant can be used in some embodiments. A ferromagnetic material that is not magnetized can be used. Non-ferromagnetic material that can establish magnetic attraction can be used in some embodiments, providing that the art enables such.

In view of the above, it can be seen that in some exemplary embodiments, there are magnet apparatuses that are configured to retain the magnet thereof within a specific location within the housing thereof, while enabling the magnet to move. In this regard, by way of example only and not by way of limitation, embodiments where the magnet is normally held at the bottom of the housing owing to magnetic attraction between the magnet 686 and the housing (bottom wall of the housing) when the external component is not magnetically coupled to the implantable component, when the external component is brought into magnetic coupling with the implantable component so as to hold the external component proximate the implanted component, and so as to, for example, align the external communication coil with the implanted communication coil utilizing the magnetic polarities of the magnets, the magnet 686 could be pulled away from the bottom of the housing up to contact with the top of the housing. Upon removal of the external component from the person and thus the elimination of magnetic communication between the external magnet and implanted magnet, the magnetic attraction of the magnet 686 to the magnet material of the bottom of the housing would pull the magnet 686 downward (at least if the top wall was not a magnetic material, for example). It is briefly noted that the terms upward and downward are described with respect to the orientation shown in the figures. In reality, during use, the aforementioned upward and downward movements would in fact be from side to side because the cochlear implant and/or the active transcutaneous bone conduction device implant, or more specifically, the receiver coils and thus the magnets thereof, are typically located on the side of the head between the skull and the skin, and thus would be oriented 90° from the orientation shown in FIG. 7A and 7D. (The orientation about the longitudinal axis of the magnet / magnet apparatus could be any angle, but typically, the tab would point to the rear of the head as the slit of the implant would be facing towards the rear.)

The embodiments above have focused on the concept of at least a portion of the housing 710 of the magnet apparatus being made of a magnetic material, magnetized or otherwise. In an alternate embodiment, the housing 710 is not made of a magnetic material. Instead, the housing is made of a polymer such as PEEK or the like. In an embodiment, no part of the housing is made of a magnetic material. In an exemplary embodiment, no part of the housing is made of a metal material or otherwise includes a metal material. In an embodiment, the housing is made of titanium or portions of the housing can be made of titanium, at least nonmagnetic titanium alloy (in the event that a titanium alloy that is magnetic can be devised). In an exemplary embodiment, the tab 762 can be made of metal, or a grommet reinforcing hole 764 could be made of metal. In an exemplary embodiment, the tab is not metal. Indeed, in an exemplary embodiment, all outer portions of the magnet apparatus, and/or portions that could be exposed to body fluids, are made of nonmagnetic material or otherwise do not include magnetic material.

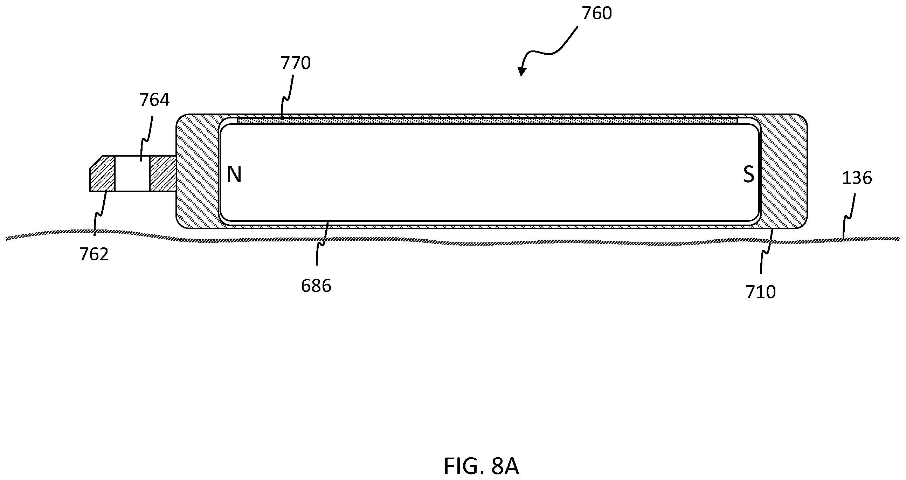

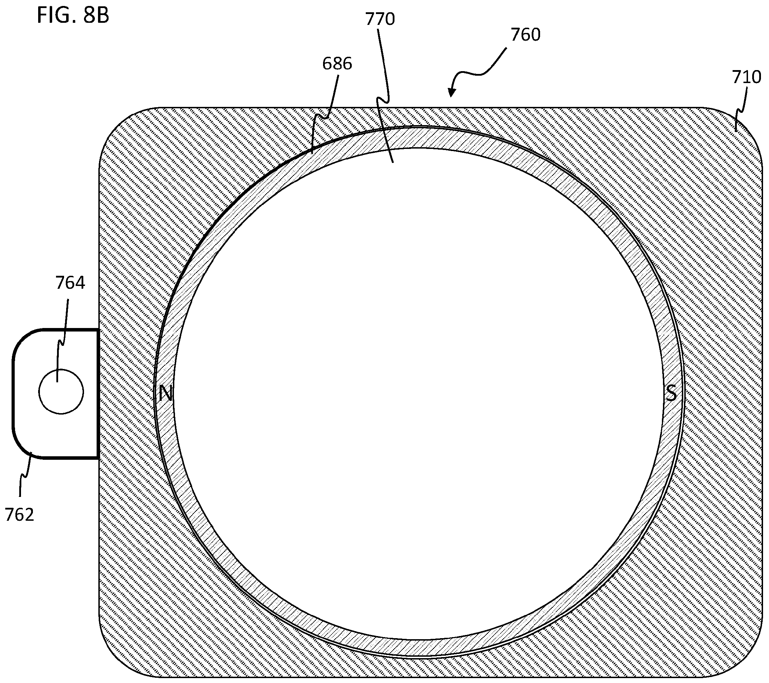

In an exemplary embodiment, the magnet apparatus could include a body made of a magnetic material, such as shim 770 as shown in figure 8A. In an exemplary embodiment, shim 770 is a flat disk as can be seen in figure 8A and 8B (the latter a top view of FIG. 8A). As seen, an outer diameter of the shim 770 is less than the outer diameter of the magnet 686, although in other embodiments the diameter can be the same or the former's diameter could be larger than the latter. In an embodiment, the shim is a low reluctance material. The shim can be soft iron. In an embodiment, the shim is a magnetized component (it can be a permanent magnet). In another embodiment, the shim is not a magnet and/or is not magnetized (more on this below).

The shim can be a plate or a sheet of metal. A shim or body can be cut from a sheet, or segmented patterns could be made by adhering pre-cut forms (e.g., punched, laser cut, wire-cut, etc.), or could be made by selective material removal (e.g., laser ablation, chemical etching, machining, etc.). The bodie(s) of the magnetic material components can be fabricated according to any utilitarian manner. The bodies can be machined from a block of magnetic material to obtain the desired thicknesses and/or shapes.

In the embodiment shown in figure 8A, the body 770 is located inside the cavity along with the magnet 686. In the embodiment shown in figure 8A, the magnet 686 is in direct contact with the body 770. It is noted that in some embodiments, a lubricant could be utilized within the cavity or otherwise be located on the surfaces of the magnet and/or the shim and/or the walls of the housing so as to create a lower friction environment so as to enhance rotation or otherwise enable movement. As used herein, a pure fluid that may be interposed between two bodies where at least one body is attracted to the other body via magnetism does not prevent the two bodies from being considered in direct contact. This is opposed to a solid that might be interposed between the two bodies or a substance that has properties of both solid and a fluid feature. Further, if the magnet 686 is coated with paint for example, or with Teflon, for example, where the paint is solidified, the magnet 686 would still be in direct contact with the shim because it is simply a coating. The same if the shim was painted or coated. Also, if the shim was coated with a lubricant that remains fluid, there would be direct contact between the shim and the magnet providing there is nothing else is between the shim and the magnet. Put another way, a coated or painted magnet is still a magnet.

FIG. 8A thus shows the magnet 686 in direct contact with the shim 770 (although as seen below, in other embodiments, the magnet is separated by a distance from the shim). In an embodiment, there is a Teflon coating on the side of the shim 770 facing the magnet. The shim 770 is adhesively bonded to the upper wall of the housing. An alternate embodiment, the shim can be welded to the upper wall of the housing. The point is, in this embodiment, the shim is directly exposed to the magnet 686 and in the absence of external magnetic forces or otherwise large-scale accelerations that would overcome the magnetic force/magnetic attraction between the magnet 686 in the shim 770, the magnet 686 will be held against the shim 770. Thus, there will be a space or gap below the magnet 686 and the bottom wall of the housing 710.

In an embodiment, the shim (which is used herein as a proxy for any magnetic material arrangement that has utilitarian value - any disclosure of a shim corresponds to an alternate disclosure of a magnetic plate or a magnetic body) could be embedded into the housing. Alternatively, the magnetic material could be coated onto the raw titanium material of the housing wall (or other material of the housing wall) before the housing is manufactured. The material could be coated on after the housing wall at issue is formed. Possible processes of placing / securing the magnetic material include resistance welding, laser welding, sputtering (physical vapor deposition), chemical coating, explosive welding, ultrasonic welding, adhesives).

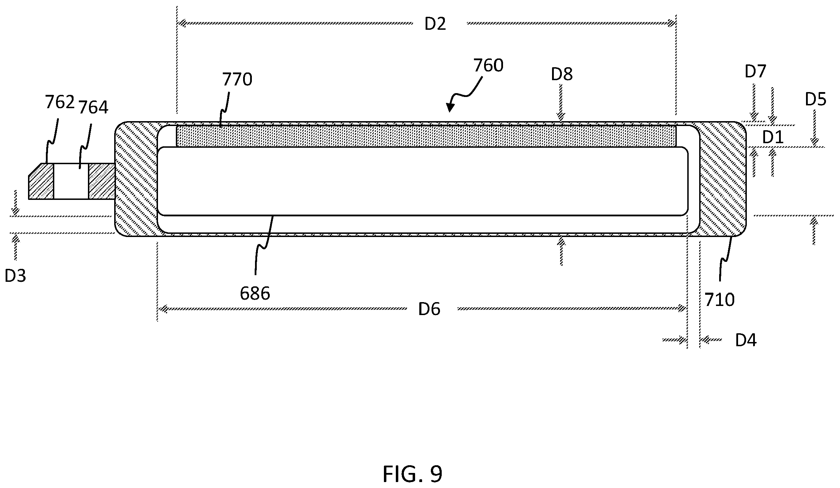

Figure 9 presents a version of the embodiment of figure 8A except with more exaggerated details for the purposes of explanation. In this regard, the embodiment of figure 9 is the same as the embodiment of figure 8A. Values D6 and D5 (diameter and height of the magnet, respectively) can be any of the values detailed above with respect to the disk magnets. That is, for example, the components of the magnet 686 (which can be a monolithic body or made of two or more components (e.g., the 4 pole magnet can be two separate bodies magnetically attracted to each other and/or adhesively or mechanically connected to each other - e.g., the 4 pole magnet of 686 of FIG. 6C, which can be separate bodies 696A and 696B) that has a circular outer circumference and a height. The height (D5) can be less than, more than, or equal to 0.5, 0.6, 0.7, 0.8, 0.9, 1.0, 1.1, 1.2, 1.3, 1.4, 1.5. 1.6. 1.7, 1.8. 1.9, 2.0, 2.1, 2.2, 2.3, 2.4, 2.5, 2.6. 2.7, 2.8, 2.9, 3.0, 3.5, 4, 4.5, 5, 5.5, 6, 7, 8, 9, 10, 11, or 12 mm, or any value or range of values therebetween in 0.01 mm increments. The diameter (D6) can less than greater than or equal to 5, 5.5, 6, 6.5, 7, 7,5, 8, 8.5, 9, 9.5, 10, 11, 12, 13, 14, 15, 16, 17, 18, 19, 20, 21, 22, 23, 24, 25, 26, 27, 28, 29, or 30 mm, or any value or range of values therebetween in 0.01 mm increments. This can also be the case for the embodiments above as well.

Values for D1 (the thickness of the shim) can be less than, more than or equal to 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 125, 150, 175, 200, 225, 250, 275, 300, 325, 350, 375, 400, 450, 500, 550, 600, 650, 700, 750, 700, 850, 900, 950, or 1000 microns, or any value or range of values therebetween in 0.1 micron increments, depending on the retention that is desired and/or depending on the size / strength of the magnet 686. Values for D7 (the housing wall thickness - shown is the top thickness, but the bottom can also be one of the values of D7 (the top need not be the same thickness as the bottom) can be any of the values for D1, starting at 20 microns (the values need not be the same - we are simply reusing the values in the interests of textual economy), as well as 1.0, 1.1, 1.2, 1.3, 1.4 or 1.5 mm, with any value or range of values in the increments for D7. The values of D3 can be any of the values of D7 (again, they need not be the same). Values for D8 (the maximum thickness of the magnet apparatus) can be 0.5, 0.75, 1, 1.25, 1.5, 1.75, 2, 2.25, 2.5, 2.75, 3, 4, 5, 6, 7, 8, 9, 10, 11, 12, 13, 14, or 15 mm, or any values or range of values therebetween in 0.1 mm increments. Values for D2 can be any of the values of D6 (again, they need not be the same). D2 can be bigger than D6, or smaller. And as we detail below, embodiments can used shims that are non-circular (with respect to the top view). In such embodiments, D2 can be the maximum diameter. Moreover, there can be a plurality of D2 values (one for the maximum diameter, one for the minimum diameter (e.g., elliptical), for non-square rectangle, one for the long side and one for the short side, for a triangle, one D2 could be the height and one D2 could be the base, etc.). And note that in some embodiments, the values herein are values where a given component will fall within or be larger.

And we note that while embodiments herein are described mostly in terms of a round / circular magnet, disk shape magnet, other shapes can be used. For example, a pill shaped magnet and/or a racetrack shaped magnet can be used. A triangular, rectangular, elliptical shaped magnet can be used. Embodiments typically have at least one flat surface. In some embodiments, the surface has a maximum diameter of at least 50, 55, 60, 65, 70, 75, 80, 85, 90, 95 or 100% of the maximum diameter of the magnet.

FIG. 9 shows the magnet located to one side of the cavity. Thus, when magnet 686 is fully against the sidewall of the housing, D4 is any of the values detailed for D3 (again, they need not be the same). The height of the cavity is thus D3 plus D5 plus D1, and the width of the cavity (diameter of the cavity) is thus D6 plus D4. In an exemplary embodiment, D6 is between (including inclusive) about 8 to 14 mm, D5 is between (again inclusive) of about 1 to 3 mm, D4 can be between about 0.05 mm and 1 mm (when the magnet is all the way to the left wall). D1 is between about 5 to 75 microns, D3 is between about 50 to about 750 microns. D7 is about 50 to 250 microns (and the bottom wall thickness can also be 50 to 250 microns - the bottom wall need not be the same as the top wall). D2 can be between 7 and 13 mm, or 1 and 13 mm.

Thus, it can be seen that in some embodiments, there is a clearance between an interior of the housing and the magnet in a direction of a longitudinal axis of the housing and/or the magnet, and/or in a direction normal to the longitudinal axis (the lateral direction) of the housing and/or the magnet. In an embodiment, this can enable (or more accurately, result in) movement in the longitudinal direction and/or in the lateral direction, respectively. Embodiments herein can utilize magnetic attraction in the various directions to limit / restrict movement in those directions (and/or directions normal to the various directions) that would or might otherwise exist because of the respective clearances (wherein limit/restrict includes preventing movement, at least under certain circumstances (such as under certain acceleration regimes)). In an embodiment, the clearance is all the way around the magnet when an interior of the housing and the magnet are concentric with one another, and the clearance has values with respect to the concentric arrangement less than and/or equal to and/or greater than 10, 9, 8, 7, 6, 5, 4, 3, 2, 1.5, 1, 0.9, 0.8, 0.7, 0.6, 0.5, 0.4, 0.3 or 0.2 % of the diameter of the interior of the housing measured normal to the longitudinal axis (both the diameter and the value). In an embodiment, the clearance between the magnet (including a coated magnet - a "magnet" covers the apparatus that is a magnet, which can be coated with a metal or some other material, and thus the clearance would be measured from the outer surface of the coating) and the opposite surface of the housing assembly, such as the housing wall, as measured along the longitudinal axis, when the magnet is located on one side of the housing assembly with respect to direction along the longitudinal axis (e.g., against one wall and away from the opposite wall), when the magnet is concentric as noted in the preceding sentence, can be less than and/or equal to and/or greater than 10, 9, 8, 7, 6, 5, 4, 3, 2, 1.5, 1, 0.9, 0.8, 0.7, 0.6, 0.5, 0.4, 0.3 or 0.2 % of the diameter of the interior of the housing measured normal to the longitudinal axis of the housing (both the clearance and the diameter).

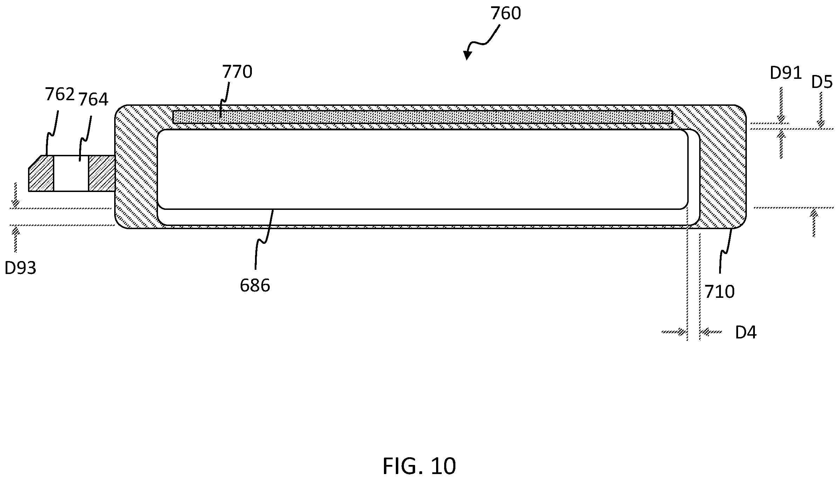

FIG. 10 presents another embodiment that utilizes a magnetic body 770. However, here, the body 770 is embedded in the housing wall (the upper housing wall). In an exemplary embodiment, the housing can be moulded about the body 770. Alternatively, the housing wall can include a hollow into which the body 770 is inserted, and then a cover can be placed over the hollow. In an exemplary embodiment, the cover can simply be filled with a material that cures and provides a seal or otherwise secures the shim 770 in place. In an embodiment, a pocket could be made for the shim, which is then sealed with adhesive tape, that also acts as a low friction surface. Or a shell could be welded over the pocket in another embodiment.

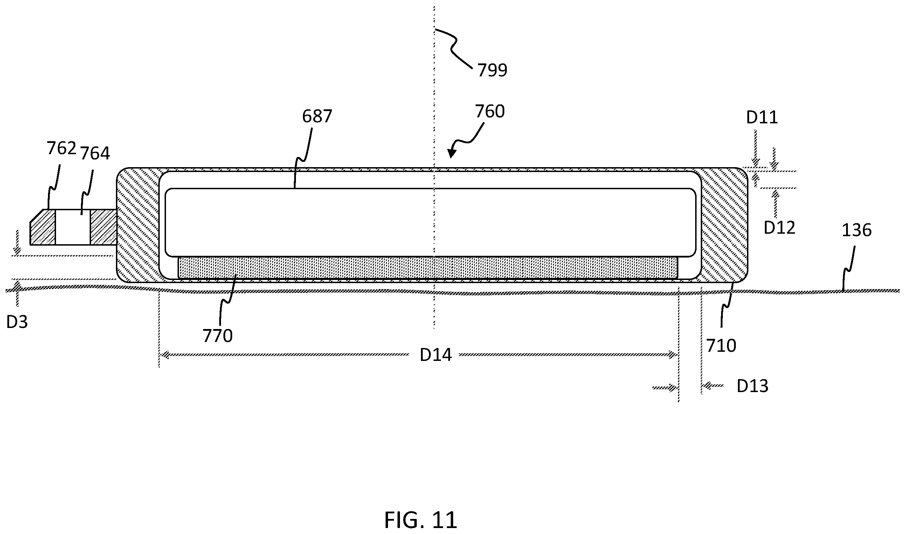

FIG. 11 presents another exemplary embodiment where the shim 770 is inside the cavity of the housing 710 and is in direct contact with the magnet 687 (here, the magnet of FIG. 6C is presented - again, any magnet that can have utilitarian value can be utilized providing that the art enables such). The shim 770 can be bonded to the bottom wall. Some features of the embodiment of FIG. 11 are that D11 can be any of the values of D7 above. D12 can be less than, more than or equal to 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 125, 150, 175, 200, 225, 250, 275, 300, 325, 350, 375, 400, 450 or 500 microns, or any value or range of values therebetween in 0.1 micron increments. Indeed, in may respects, as long as D12 is greater than zero so that the magnet does not jam (it can rotate), such embodiments can be practiced, and thus embodiments can entail simply ensuring that tolerancing and material deflection do not reduce D12 to zero. That said, in some embodiments, D12 can be zero if the housing is sufficiently flexible so that the magnet can rotate in response to external forces (from the external device).

In some embodiments, where the housing is sufficiently thin and/or sufficiently flexible, the clearance could also be 0, as long as the magnet is still able to rotate in response to external forces. An exemplary embodiment of this use a vacuum in the hermetic portion of the housing, which pulls the flexible wall down on to the magnet to take up any clearances. (This could prevent or at least frustrate movement in the axial direction, but could permit movement in the radial direction.).

In an exemplary embodiment, D12 is between 50 to 750 microns. D13 can be less than, more than or equal to 0.1, 0.2, 0.3, 0.4, 0.5, 0.6, 0.7, 0.8, 0.9, 1.0, 1.1, 1.2, 1.3, 1.4, 1.5. 1.6. 1.7, 1.8. 1.9, 2.0, 2.1, 2.2, 2.3, 2.4, 2.5, 2.6. 2.7, 2.8, 2.9, 3.0, 3.5, 4, 4.5, 5, 5.5, 6, 7, 8, 9, 10, 11, or 12 or more mm, or any value or range of values therebetween in 0.01 mm increments. 1900 or 2000 microns or any value or range of values therebetween in 0.1 micron increments. D14 can be less than, more than or equal to 1, 1.1, 1.2, 1.3, 1.4, 1.5, 1.6. 1.7, 1.8, 1.9, 2, 2.1, 2.2, 2.3, 2.4, 2.5, 2.6. 2.7, 2.8, 2.9, 3.0, 3.5, 4, 4.5, 5, 5.5, 6, 7, 8, 9, or 10 or 11 or 12 mm or more any value or range of values therebetween in 0.01 mm increments. D13 can be less than, more than or equal to 0.1, 0.2, 0.3, 0.4, 0.5, 0.6, 0.7, 0.8, 0.9, 1.0, 1.1, 1.2, 1.3, 1.4, 1.5. 1.6. 1.7, 1.8. 1.9, 2.0, 2.1, 2.2, 2.3, 2.4, 2.5, 2.6. 2.7, 2.8, 2.9, 3.0, 3.5, 4, 4.5, 5, 5.5, 6, 7, 8, 9, or 10 or 11 or 12 or more mm or any value or range of values therebetween in 0.01 mm increments. 1900 or 2000 microns or any value or range of values therebetween in 0.1 micron increments. D3 can be less than, more than or equal to 0.1, 0.2, 0.3, 0.4, 0.5, 0.6, 0.7, 0.8, 0.9, 1.0, 1.1, 1.2, 1.3, 1.4, 1.5. 1.6. 1.7, 1.8. 1.9, 2.0, 2.1, 2.2, 2.3, 2.4, 2.5, 2.6. 2.7, 2.8, 2.9, 3.0, 3.5, 4, 4.5 or 5 mm or any value or range of values therebetween in 0.01 mm increments. D14 can be 5, 5.5, 6, 7, 8, 9, 10, 11, 12, 13, 14, 15, 16, 17, 18, 19 or 20 mm or any value or range of values therebetween in 0.01 mm increments.

And it is noted that any of the dimensions / features of detailed for one embodiment can be for another embodiment provided that the art enables such, in some embodiments.

It is noted that any of the given dimensions applied to any given embodiment can be applicable to any of the other embodiments providing that the art enables such laws otherwise noted. For example, the dimensions D14 and D13 can be applicable to the embodiment of figure 9 above (with the value of D2 selected accordingly). The dimensions of FIG. 9 for the gap below the magnet and the housing can be the case for the gap between the top of the magnet and the housing of FIG. 11.

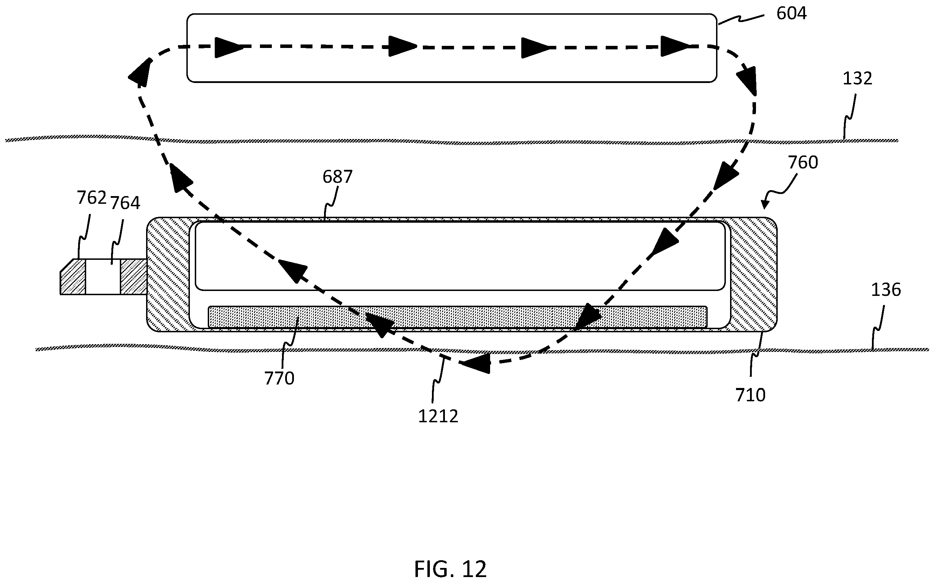

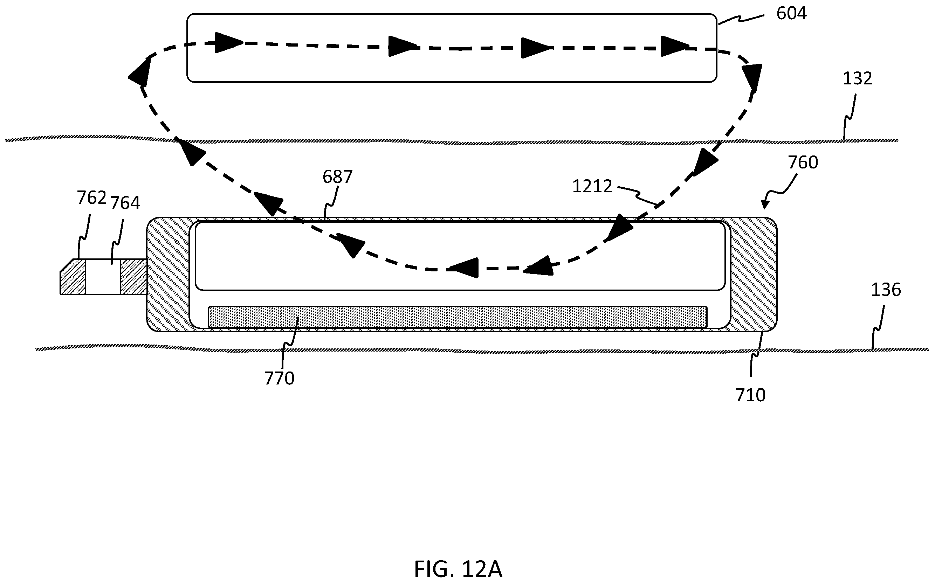

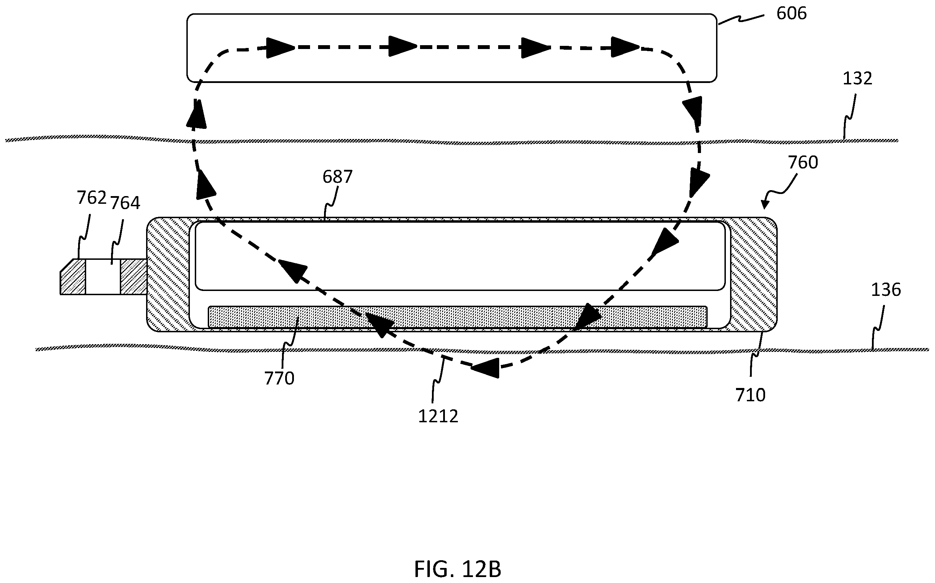

FIGs. 12 and 12A depict an exemplary scenario of use of the magnet apparatus 760 of the embodiment of figure 11 or variation(s) thereof when the external component is magnetically held against the skin of the recipient via interaction between the external magnet 604 and the implanted magnet 687 (the flux path might pop out of the bottom as seen in FIG. 12, or might stay within the implant magnet as seen in 12A. Not shown are the respective bodies of the implanted component and the external component, which bodies are omitted for purposes of clarity only. Shown is the magnetic flux 1212 having a path that results from the utilization of the four pole magnet 687 and the diametrically magnetized magnet 604. The path would be different if, for example, a four pole magnet was utilized for the external magnet 604, or if a magnet that was magnetized with a clarity direction 90° from the longitudinal axis thereof was utilized for the implanted magnet, etc. As seen, the magnetic flux 1212 that results from the interaction of the two magnets results in magnet 687 being pulled upward towards the external component in general, and the external magnet 604 in particular. FIG. 12B shows the flux when the external magnet corresponds to magnet 606 (although in other embodiments, the flux travels through the implant magnet as seen in FIG. 12A).

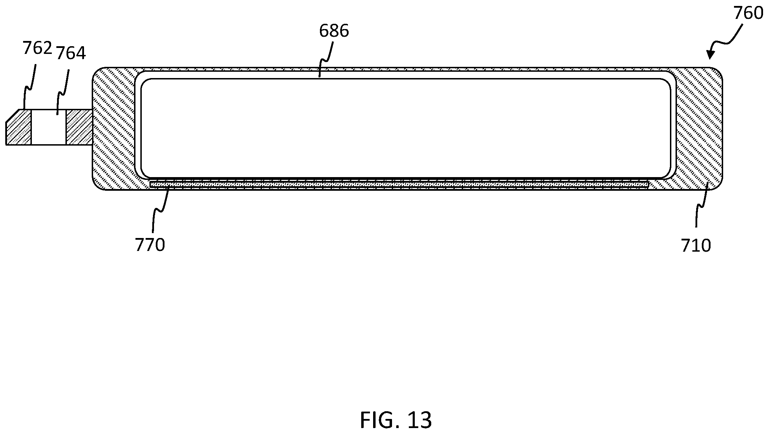

FIG. 13 depicts an alternative embodiment where the shim 770 is embedded in the housing 710 as can be seen. Here, diametrically magnetized magnet 686 is utilized.

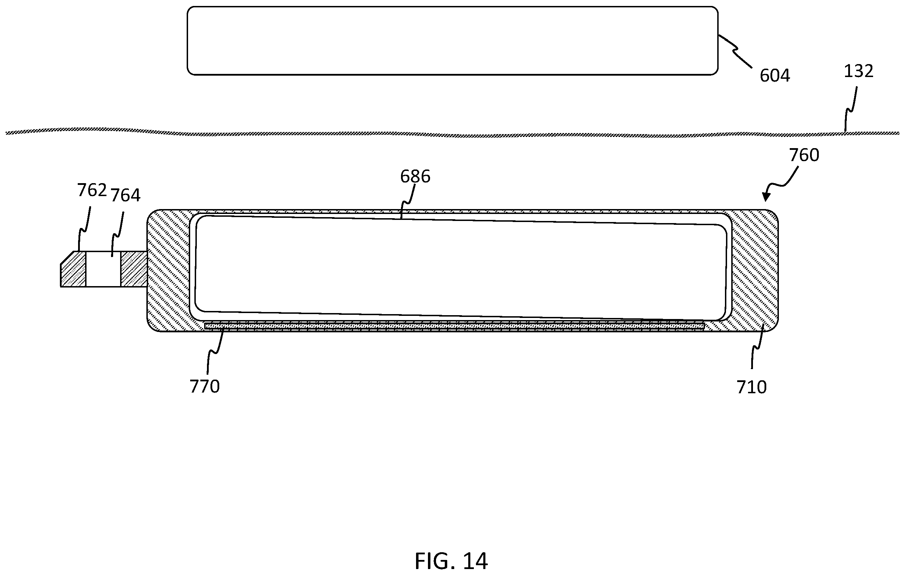

As with the embodiments detailed above, the magnet apparatus 760 of figure 11 is configured so that the implanted magnet 687 will rotate about the longitudinal axis 799 to generally magnetically align the poles of the magnet 687, or more accurately, the plane upon which those poles lie, with the poles of the external magnet, or more accurately, the plane upon which those poles lie. In an exemplary embodiment, this occurs when the implanted magnet moves in space from the location where the implanted magnet is in direct contact with the shim 770, or otherwise is closest to the shim 770 (as in the embodiment of FIG. 13). That is, in an exemplary embodiment, the implanted magnet 687 or 686 is essentially floating in space for a brief period of time. In a sense, to the extent that friction forces prevent or otherwise limit rotation of the implanted magnet while the implanted magnet is in contact with the housing and/or the shim, while the magnet travels in the space of the cavity towards the top of the housing, those friction forces are essentially relieved, were certainly reduced (there could be some friction forces with respect to the side wall if the magnet 686 or 687 is in contact with the sidewall - recall that in reality, the magnet apparatuses are utilized in a manner such that they are rotated about 90° from the views shown in figure 13 for example. Thus, gravity could pull the implanted magnet to one side of the sidewall. That said, owing to the circular nature of the magnet and/or the interior of the cavity, the magnet might roll as it is moving towards the external component. And even if the magnet does not lose total contact with the walls of the housing, the movement of the magnet along the surfaces of the walls will reduce the effects of friction and thus permit the magnet to rotate a certain amount at least. In a sense, the gap within the housing that permits the magnet to move from the bottom of the housing to the top of the housing provides an unlocking feature to enable more thorough rotation of the magnet. Put another way, the scenario depicted in FIG. 13 shows the magnet 686 in the locked position, where unlocking would correspond to the magnet 686 being subjected to a force in a direction away from the bottom surface of the housing wall sufficient to reduce the friction forces between the wall surface and the bottom surface of the magnet, which could correspond to the movement of the magnet in space completely off of the bottom wall surface. FIG. 14 shows an unlocked position of the magnet 686 in an exemplary scenario, where the upward force is sufficient to reduce the friction between the bottom surface of the housing and the magnet to enable utilitarian rotation.

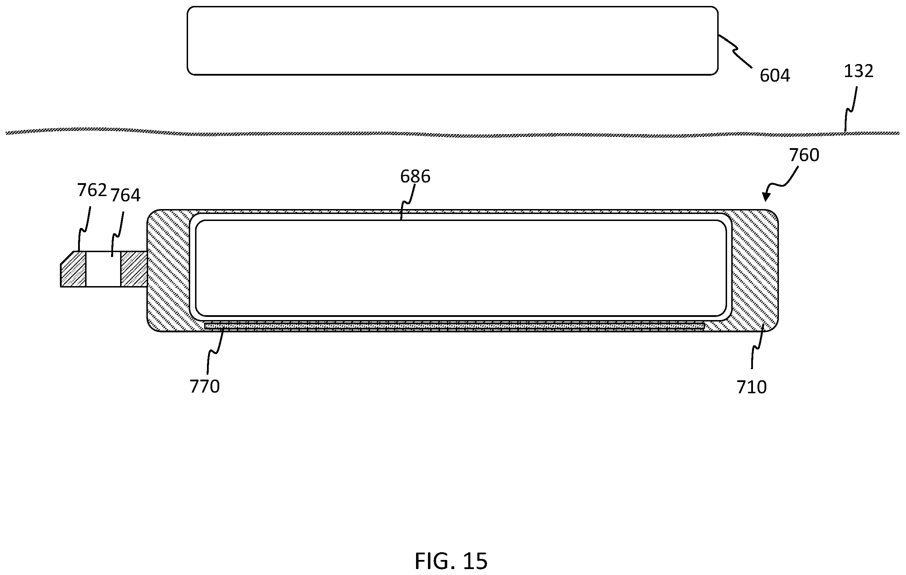

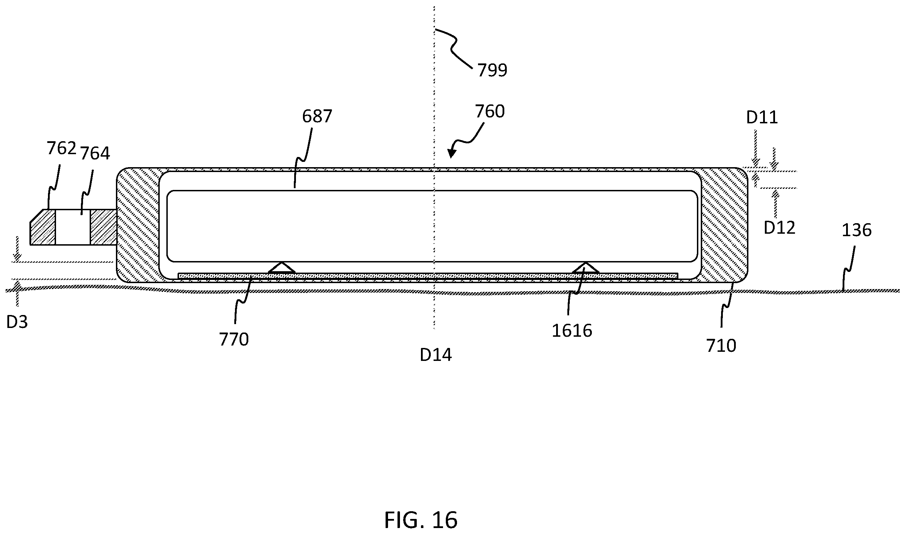

FIG. 15 shows another example of an unlocked position (where the magnet 686 is moving upwards towards the magnet 604). And to be clear, the concept of unlocking does not mean that the magnet must necessarily move in the vertical direction with reference to figure 13. Embodiments can exist where the magnet is always in an unlocked position even without any exterior magnetic force (or more accurately, a magnetic force that does not pull the magnet upwards - of course the magnet turns owing to a magnetic force - in this regard, a magnetic torque would be applied that would be comparable to the torque applied to a misaligned external magnet but would not create a pulling force). For example, the wall of the housing or the side of the shim that contacts the magnet could be coated with Teflon or the like or some other low friction material or the cavity could be filled with (or not filled, instead, a sufficient quantity is present) a low viscosity fluid. Moreover, the surface of the housing or the surface of the magnet could be configured to reduce the total friction force that might otherwise exist with respect to the magnetization forces between the magnet in the shim. Figure 16 depicts an exemplary embodiment where a band 1616 is located on top of the shim 770. The band has a triangular cross-section as can be seen, although in other embodiments, the band could have a rectangular cross-section. The effect of this is to reduce the total surface area of the fixed components that support the magnet 687 and the magnet itself. Here, instead of almost all of the bottom surface of the magnet 687 contacting the nonmoving component (the shim 770, and this feature can be utilized instead on top of the housing wall where the shim is located below the housing wall such as in the embodiments described above), only a very limited surface area of the nonmoving component contacts magnet 687 (the ridge of the band 1616). With reference to the phrase "nonmoving component," it is meant the component that is nonmoving relative to the remainder of the housing and/or the remainder of the magnet apparatus 760. Alternatively, it could be meant everything other than the magnet. And note that while the embodiment shown in figure 16 has the band being a fixed component relative to the remainder of the housing, the band could instead be fixed to the magnet 687. There can be utilitarian value with respect to inverting the band 1616 so the point / ridge of the band 1616 now contacts the shim 770, thus reducing the surface area of the moving part that contacts the nonmoving part (where the moving part is now the magnet and the band 1616). The band could be a monolithic part of the magnet or could be a separate component that is physically attached to the magnet, such as with adhesive. The band could be magnetized or not magnetized. The band could be a metal or could be a nonmetal, such as a polymer. The band could be an oil impregnated material, such as OilLite or some other material that has a very low viscous surface.

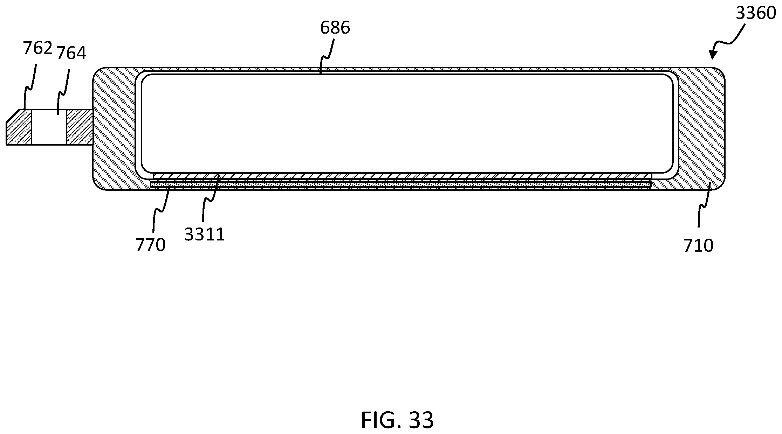

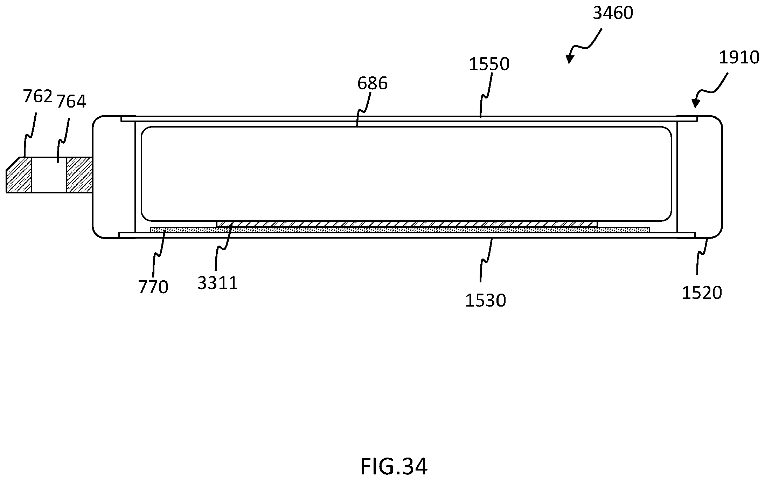

FIGs. 33 and 34 provide another embodiment related to friction management. Additional details of this will be described below, but briefly, a plastic disk 3311, which can be a drop-in insert, can be used to manage friction. Again, additional details of this will be described below.

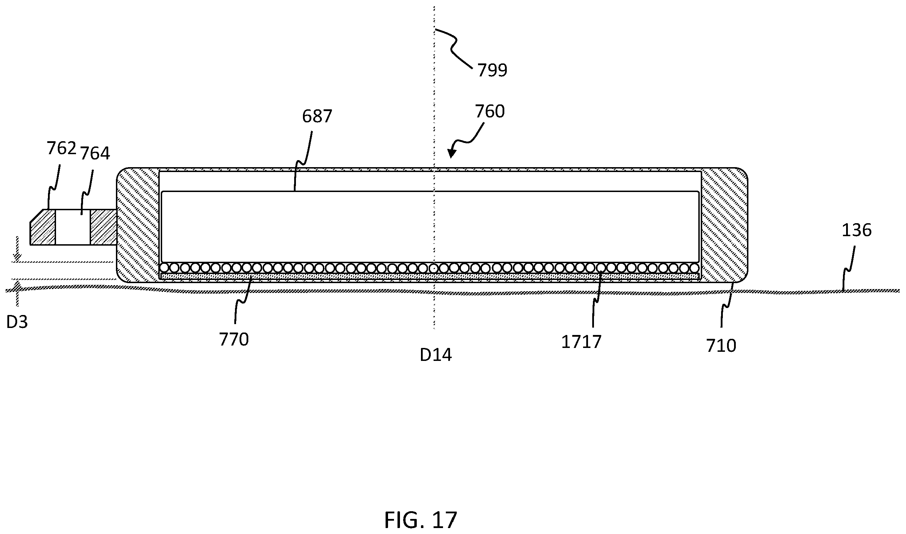

Figure 17 presents another exemplary embodiment where ball bearings 1717 support the magnet 687 over the shim 770. A race could be located with the bearings, thus holding the bearings in place. The race could be connected to the magnet or could be connected to the shim or the housing. In an exemplary embodiment, the bearings can be made of a magnetic material that are thus the magnet 687 attracts the bearings 1717 and thus would move upward with movement of the magnet. By sizing the magnet and the housing (and the shim if the shim is in the cavity), and by providing a sufficient number of bearings to densely pack the bearings, the lateral movement of the bearings could be limited.

In an exemplary embodiment, the force of the implanted magnet against the shim or the housing wall or whatever support structure is positioned between the shim and the magnet, or the force between the rotating component and the non-rotating component, in the complete absence of any external magnetic force (such as the external magnet), when the longitudinal axis of the magnet is 90 normal to the direction of gravity, is less than or equal to 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.35, 0.4, 0.45, 0.5, 0.6, 0.7, 0.8, 0.9, 1.0, 1.1, 1.2, 1.3, 1.4, 1.5, 1.6, 1.7, 1.8. 1.9 or 2.0 Newtons or any value or range of values therebetween in 0.001 Newton increments (note that the values above 0.7 Newtons or so are likely for larger prostheses). (As will be detailed below, these forces can also be in the direction of gravity, such as where the magnetic component is laterally located, or not - more on this below). In an exemplary embodiment, an acceleration applied to the magnet in a direction opposite of the location of the shim or the housing wall or whatever support structure is positioned between the shim and the magnet, or the acceleration applied to the rotating component and the non-rotating component, in the complete absence of any external magnetic force (such as the external magnet), when the longitudinal axis of the magnet is 90 normal to the direction of gravity, is less than, equal to or greater than 0.1, 0.2, 0.3, 0.4, 0.5, 0.6, 0.7, 0.8, 0.9, 1, 1.25, 1.5, 1.75, 2, 2.25, 2.5, 2.75, 3, 3.25, 3.5, 3.75, 4, 4.25, 4.5, 4.75, 5, 5.25, 5.5, 5.75, 6, 6.5, 7 or 8 Gs, or any value or range of values therebetween in 0.05G increments, to have the magnet and/or center of gravity of the magnet move and/or have the magnet move from its zero G attracted position (otherwise, the magnet does not move owing to the friction forces).

And note that these values can also be the case for accelerations in a direction normal to the aforementioned direction of acceleration (e.g., in the direction of gravity when the magnet is held as just detailed - thus the friction between the major surfaces of the magnet and the housing assembly is sufficient to hold the magnet in place when subjected to one or more of these accelerations). And to be clear, the friction forces can be balanced so that rotation of the magnet a certain amount for magnetic alignment with the magnet of the external component occurs even if the magnet is in contact with the housing during the rotation. Embodiments include preventing and/or limiting the movements in the direction of the longitudinal axis and normal thereto while permitting rotation upon magnetic coupling of the external component with the implantable component. In an embodiment where there is movement, movement in a direction normal to the longitudinal axis is limited to no more than 80, 70, 60, 50, 40, 30, 25, 20, 19, 18, 17, 16, 15, 14, 13, 12, 11, 10, 9, 8, 7, 6, 5, 4, 3, 2, 1.5, 1, 0.9, 0.8, 0.7, 0.6, 0.5, 0.4, 0.3 or 0.2 % of a maximum clearance between the magnet and the housing measured normal to the longitudinal axis (both the diameter and the value).

In an exemplary embodiment, the force of the implanted magnet against the shim or the housing wall or whatever support structure is positioned between the shim and the magnet, or the force between the rotating component and the non-rotating component, in the complete absence of any external magnetic force (such as the external magnet), when the longitudinal axis of the magnet is normal to the direction of gravity, is less than or equal to 5, 6, 7, 8, 9, 10, 11, 12, 13, 14, 15, 16, 17, 18, 19, 20 25, 30, 35, 40, 45, 50, 55, 60, 65, 70, 75, or 80% of the force of the implanted magnet against the housing wall or the shim or whatever support structure is positioned between the magnet and the housing wall or the rotating component and the non-rotating component, all in the direction of the external magnet from the implanted magnet (which could be the same direction as the shim relative to the implanted magnet) due to the external magnet used with the external component being located against the skin facing housing wall (this could be tested by removing the magnet of the external component, and taking the housing out of the implanted component, and placing the external magnet against the housing with polarity being that which is the case during normal use) and/or with the external device being used with the implant with a simulated skin flap thickness and/or any one or more of the other control variables detailed above. In an exemplary embodiment, the force of the implanted magnet against the shim or the housing wall or whatever support structure is positioned between the shim and the magnet, or the force between the rotating component and the non-rotating component, in the complete absence of any external magnetic force (such as the external magnet), when the longitudinal axis of the magnet is 90 normal to the direction of gravity, is less than or equal to or greater than 50, 55, 60, 65, 70, 75, 80, 85, 90, 95, 100, 105, 110, 125, 150, 175 or 200% of the force of the implanted magnet against the housing wall or the shim or whatever support structure is positioned between the magnet and the housing wall or the rotating component and the non-rotating component, all in the direction of the external magnet from the implanted magnet (which could be the same direction as the shim relative to the implanted magnet) due to the external magnet used with the external component being located against the skin facing housing wall (this could be tested by removing the magnet of the external component, and taking the housing out of the implanted component, and placing the external magnet against the housing with polarity being that which is the case during normal use) and/or with the external device being used with the implant with a simulated skin flap thickness and/or any one or more of the other control variables detailed above.

In an embodiment, an attractive force between the magnet 686 and the shim 770 remains substantially the same (which includes the same) or otherwise effectively the same or otherwise the force remains utilitarian regardless of the distance of the magnet from the shim (within the confines of the housing). For example, with respect to the arrangement of FIG. 10, the attractive force would be the same if the magnet 686 was as shown or if the magnet was "on the bottom" / on the opposite side of the housing from that shown in FIG. 10. In an embodiment, this is the case irrespective of whether the shim is in the compartment with the magnet, or outside the compartment. In an embodiment, this is the case irrespective of whether there is a component (e.g., a portion of the hosing wall as in FIG. 10) or multiple components between the magnet and the shim. Corollary to this is that the attractive force between the magnet 686 and the shim 770 is the same if the component(s) between the shim and the magnet were there or not there (all other things being equal). That is, as a control, if the distance is kept the same, and the component(s) are removed (which can be by destruction, as this is simply to demonstrate that his is the case) the force remains the same.

In an exemplary embodiment, the magnetic strength between the implanted magnet and the external magnet with a skin flap thickness of 3, 4, 5, 6, 7, 8, 9, or 10 mm is between 100, 150, 200, 250, 300, 350, 400, 450, 500, 550, or 600 Gauss. (Note that all examples herein can be with respect to the implanted component and the external component, so the magnets would be spaced even further owing to the silicone of the implant and the base of the external component, while all examples can be the magnet apparatus of the implant and the magnet (whether in a housing or not) of the external component.)

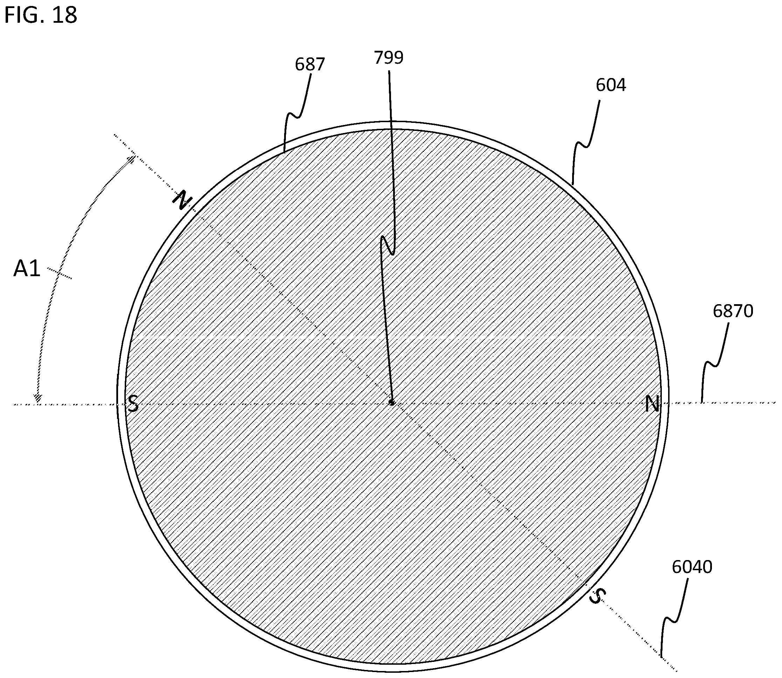

FIG. 18 presents an exemplary schematic detailing the angular orientations of magnets for explanation purposes. With respect to the orientation of FIG. 12, the view seen in FIG. 18 is looking towards the implant magnet from the bottom of FIG. 12; that is from the perspective inside the head looking outward, where the external magnet is on the opposite side of the implanted magnet from the perspective of the viewer. The external magnet 604 is shown as being slightly larger with respect to its outer diameter than the implanted magnet 687. This can be the case in some embodiments, and indeed, the external magnet could be more than slightly larger than the implanted magnet 687. In other embodiments, the outer diameters can be the same or otherwise the diameter of the external magnet could be smaller than that of the implanted magnet. Also, the thicknesses could be different and could instead be the same. Moreover, it is noted that the polarization geometries of the magnets need not necessarily be the same. By way of example only and not by way of limitation, with respect to the embodiment of figure 18, magnet 687 is a four pole magnet having a flux path that changes direction at the longitudinal axis and magnet 604 is a two pole magnet that is diametrically magnetized where the magnetic flux is normal to the longitudinal axis thereof.

Figure 18 depicts an exemplary scenario where the magnetic flux paths of the respective magnets are angled relative to one another with respect to planes in which those paths travel and lying on the longitudinal axes 799 of those magnets. Figure 18 presumes that the magnets are concentric with one another. As seen, the flux paths are misaligned by an angle A1. For example, A1 could be 63 degrees. This could be a result of the external component, which could be or otherwise could include a transcutaneous RF inductance coil communicates with the implanted RF inductance coil of the implantable component, being placed on the side of the head at an angle different than that which was the case last time that the external component was placed against the head or otherwise interacted with the implanted magnet. Alternatively, and/or in addition to this, this could be a result of the magnet 687 having rotated about the longitudinal axis 799 subsequent the removal of the external component from the head, which could occur in very close temporal proximity to the action of removal. By way of example only and not by way of limitation, in the scenario where the external component pulls the implanted magnet towards the skin and away from the skull when the external component is attached to the side of the head, as the magnetic attraction between the external magnet in the implanted magnet weakens with movement of the external component away from the implanted magnet, the implanted magnet will pull itself back towards the middle shim for example, and while the magnet is in free space, the magnet could rotate. Alternatively, and/or in addition to this, the recipient could experience G forces that cause the magnet to move, such as, for example, by walking or by running or by walking downstairs, or with respect to the action of placing one's head down on a pillow when getting into bed, etc. In any event, regardless of the causation or the scenario, when the external magnet is placed against the head of the recipient, without the rotational features detailed herein, the flux path of the external magnet would be angularly misaligned with respect to the flux path of the implanted magnet, and in figure 18, that misalignment is angle A1, which is noted above, is 63° in this exemplary scenario.

Magnets according to the embodiments of the teachings detailed herein will be arranged with respect to their polarity so as to attract each other during normal standard intended use. That is, the implanted magnet will be attracted to the external magnet, and *vice versa.* This will have the utilitarian effect of holding the external component, or at least the RF inductance coil thereof, against the skin of the recipient owing to the attraction between the two magnets. This is opposed to a scenario where the external RF inductance coil is placed upside down against the skin, where the magnets would repel each other.

According to embodiments of the teachings detailed herein, the rotational features permit the implanted magnet to rotate so that the magnet field thereof will align with the magnetic field of the external magnet. Granted, the external magnet may rotate itself owing to the movement of the person's hand as he or she places the external coil onto the skin / side of the head. The idea with the rotating magnet of the implant is that the magnet will rotate and otherwise align itself with the magnetic field of the external component. According to the embodiments detailed herein, the magnet can unlock or otherwise the friction forces that exist owing to the attraction of the implanted magnet with the shim can be sufficiently reduced if not completely eliminated so as to enable the rotation of the implanted magnet so that the magnetic flux thereof aligns with the external magnet or otherwise more closely aligns with the external magnet than that which would otherwise have been the case. In some embodiments or otherwise exemplary scenarios of use, the resulting angle A1 will be *de minimis* or otherwise can be zero. In some embodiments or at least some scenarios of use, the resulting angle A1 will always be less than 5 degrees. In some embodiments or at least some scenarios of use, resulting angle A1 will always be less than 10 degrees. In an exemplary embodiment, the resulting angle after rotation A1 will be less than and/or equal to 0, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 110, 120, 130, 140, 150 160, 170 or potentially 179 or degrees or any value or range of values therebetween in 0.1° increments. In an exemplary embodiment, the resulting reduction in the angle A1 will be at least, using the initial angle prior to alignment as the base angle, 10, 15, 20, 25, 30, 35, 40, 45, 50, 55, 60, 65, 70, 75, 80, 85, 86, 87, 88, 89, 90, 91, 92, 93, 94, 95, 96, 97, 98, 99 or 100% or any value or range of values therebetween in 0.1 % increments, owing solely to the rotation of the implanted magnet (that is, the external component does not rotate / the angle is measures relative to a dynamic system where the external component (or more accurately, the external magnet) is treated as the fixed element in the dynamic system vis-à-vis rotation. In an exemplary embodiment, the aforementioned percentages are what happens when there is a skin flap is 5 mm think, and the magnetic retention force is 0.25, 0.5, 0.75, 1, 1.25 or 1.5 Newtons or any value or range of values therebetween in 0.01 Newton increments, and the two magnets are aligned with their longitudinal axes perpendicular to the direction of gravity. Again, the external magnet does not rotate in this example. (The above performance features can be determined using a test rig, for example, with a simulated skin flap thickness, for example.) In an exemplary embodiment, the initial misalignment of the magnets (initial A1) is greater than and/or equal to 20, 25, 30, 35, 40, 45, 50, 55, 60, 65, 70, 75, 80, 85, 90, 95, 100, 105, 110, 115, 120, 125, 130, or 135, or any value or range of values therebetween in 0.1 degree increments, and the external magnet causes the implanted magnet to rotate by the aforementioned values. In an exemplary embodiment, there will be no rotation or at least there will be limited rotation if angle A1 is, for example, greater than 135, 140, 145, 150, 155, 160, 165, 170 or 175, depending on the embodiment, and then when A1 would be measured from the other side and again, greater than 135, 140, etc. The limited rotation could be no more than 5, 10, 15, 20, 25 or 30 degrees, depending on the embodiment.

The aforementioned performance features can be for one or more or all of the embodiments detailed herein in some embodiments (e.g., for the embodiment where the shim is located on the skull facing side of the housing, for the embodiment where the shim is located on the skin facing side of the housing, for an external and an implanted diametrically magnetized magnet where the magnetic flux through the disk magnets is normal to the longitudinal axes, for the magnet arrangement of FIG. 12, for the magnet arrangement of FIG. 9, etc.).

In an exemplary embodiment, when the magnet 686 (or 687) is in the position shown in FIG. 13 (where the longitudinal axis is normal to the direction of gravity, in the absence of all outer forces, where no external magnet is influencing the magnet 686, and the magnet 686 is completely separated from both sidewalls of the housing 710, the minimum torque that is required to rotate the magnet at least 10° about the longitudinal axis is 0.01, 0.0125, 0.015, 0.0175, 0.02, 0.025, 0.03, 0.04, 0.05, 0.06, 0.07, 0.08, 0.09, 0.1, 0.125, 0.15, 0.2, 0.25, 0.3, 0.4, 0.5, 0.6, 0.7, 0.8, 0.9, 1, 1.1, 1.2, 1.3, 1.4, or 1.5 N-mm, or any value or range of values therebetween in 0.001 N-mm increments. Note that if a lubricant is used, such as a lubricating fluid, the torque approaches zero to obtain rotation. In this regard, with respect to the concept of the magnet being in the locked configuration, for example only, a magnet in the locked configuration would require a threshold torque to be applied such as one of those just detailed, to rotate magnet. That does not mean that the magnet is unlocked. That only means that the magnet can rotate when exposed to the minimum torque. In an exemplary embodiment, the threshold for the magnet to be "unlocked" could be a reduction in the torque required to rotate the magnet by at least 35, 40, 45, 50, 55, 60, 65, 70, 75, 80, 85, 86, 87, 88, 89, 90, 91, 92, 93, 94, 95, 96, 97, 98, 90, or 100%, or any value or range of values therebetween in 0.1% increments. In theory, this would happen at some point just before the magnet is at least partially lifted from the bottom wall of the housing. Again, in an exemplary embodiment, the interaction of the external component, or more particularly, the magnet of the external component with the magnet of the implanted component to pull the magnet away from the bottom wall of the housing corresponds to unlocking the implanted magnet.

A utilitarian feature of utilizing the magnetic body / shim is that it can, in some embodiments, tend to hold the implant magnet in a utilitarian orientation between coil-off events (i.e., when the external component is removed, the magnet will likely couple in the correct orientation the next time, barring any large head accelerations and/or that the external component was significantly misaligned the last time or presently). In this regard, there could be scenarios where there are "dead spots." For example, it could be that there are angular orientations of the magnet of the external component and the implant magnet where the implanted magnet will not rotate. For example, in an extreme case where the poles of the external magnet are aligned with the poles of the implant magnet (e.g., North over North and South over South when looking down the longitudinal axis), sufficient torque is not generated to cause the implanted magnet to rotate. If the shim prevents or otherwise limits the intra-external device use rotation of the implanted magnet (i.e., times between when the external components are attached to the implant, where there is no magnetic attraction from the external component on the implant magnet). The ideas that by limiting or otherwise preventing the rotation of the magnet, the rotational orientation of the magnet that was utilitarian with respect to establishing the magnetic coupling between the external component in the implant that holds the external component against the skin will be maintained between uses. Thus, the likelihood that a deleterious orientation between the external magnet and the implant magnet will occur is reduced because it is unlikely that the implant magnet will rotate between the times that the external component are attached to the side of the head of the recipient. Thus, embodiments can limit the rotation between uses to less than 30, 25, 20, 15, 10, 9, 8, 7, 6, 5, 4, 3, 2, or 1 degrees, and/or limit rotation to 30, 25, 20, 15, 10, 9, 8, 7, 6, 5, 4, 3, 2, or 1% of the rotation that would otherwise occur without the magnetic component, and/or improve concentricity between the external magnet and the implanted magnet by 30, 25, 20, 15, 10, 9, 8, 7, 6, 5, 4, 3, 2, or 1% that would otherwise be the case without the magnetic component, all other things being equal, or any value or range of values therebetween in 0.1 increments and this can occur over 1, 5, 10, 15, 20, 25, 30, 35, 40, 45 or 50 or more intra-external device usages, which can include recipient exercising and one or more the rhythmic activities detailed herein during those intra-external device usages, and thus there are methods that include such.

Thus, in an embodiment, there is utility in having a shim where the implant magnet is held in the correct or at least utilitarian orientation for the external device that includes the sound processor between coil-off events. This as opposed to the absence of the shim(s) where the implant magnet changes orientation when the external coil is removed. Embodiments can thus include methods where the recipient need not be instructed on having to move the external component in order to re-orient the external component, such as that which may be utilitarian with respect to orienting the microphones of the off-the-ear sound processor, or for BTE devices so the coil cable is oriented properly, given the limited length of the coil cable.

Embodiments such as that of figure 9 where the implanted magnet is always in contact with the shim 770 are not unlocked by the external component, at least not when the external component is utilized in its normal position where the external magnet and implanted magnet interact to hold the external component to the implant.

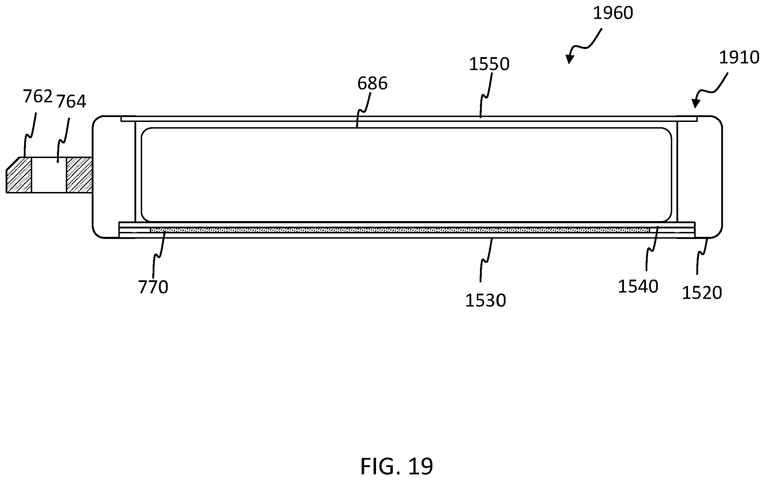

FIG. 19 presents another exemplary embodiment of a magnet apparatus of the implantable component 1960 according to an exemplary embodiment. In this exemplary embodiment, the magnet apparatus 1960 includes a housing 1910 that is made from a plurality of components that are ultimately adhered to one another. In this exemplary embodiment the shim 770 is sandwiched between two titanium plates 1540 and fifteen thirty, respectively. In this exemplary embodiment, the housing 1910 includes a sidewall apparatus 1520 that has a hollow section that extends from one side to the other, and if viewed without the walls 1530, 1540 and 1550 would appear as a circular through hole. This circular through hole is sized and dimensioned in a manner corresponding to, for example, any of the embodiments detailed above with respect to the cavity of the above embodiments. As seen, the circular through hole is sized and dimensioned to accept the magnet 686 or any of the other magnets detailed herein or any other magnets that have utilitarian value. The sidewall apparatus 1520 includes a stepped section top and a step section at the bottom so as to receive the wall sections so that when the wall sections are received in the sidewall apparatus 1520, the tops and bottoms of the sidewall apparatus are generally flush with respective walls as can be seen in figure 19. This can have utilitarian value with respect to manufacturing the magnet apparatus, such as enabling the placement of the magnet 686 and the hollow portion without having to mold the housing about the magnet. Further, this enables the shim 7702B located in the magnet apparatus with a barrier between the shim and the magnet 686 as can be seen (the barrier being the wall 1540). In an exemplary embodiment, the walls are circular plates made of titanium or titanium alloy. In an exemplary embodiment, the plate 1540 is inserted into the sidewall apparatus 1520, and then the shim 770 is placed onto the plate 1540, although in another embodiment the shim 770 can be previously attached to the plate 1540. Then, plate 1530 is placed over the shim 770 and welded to the sidewall apparatus. Note that in an exemplary embodiment, the plate 1540 is also welded to the sidewall apparatus. In another embodiment, crimping or an adhesive or any other connecting regime that can have utilitarian value can be utilized.

Note that in at least some exemplary embodiments, there are only two plates or even only one plate. Plate 1550 could instead be replaced by a housing wall that is monolithic with the sidewall component 1520 of the housing. Alternatively and/or in addition to this, only one of plates 1530 or 1540 are present. One plate / shell can have a recess machined / punched into the plate/shell to accept the shim or otherwise provide clearance for the shim. This could be on the outside or on the inside (or both, such as in embodiments where there are two or more shims, such as shims that are overlapping with respect to the longitudinal axis, which can be spaced away from each other by a plate 1540 or plate 1530 (and in some embodiments, a plurality of shims can be stacked one upon another directly in contact with each other - 1, 2, 3, 4, 5, 6, 7, 8, 9 or 10 or more shims can be used). In an exemplary embodiment, the sidewall component 1520 can have steps or graduations and the shells/plates can have different diameters. In an exemplary embodiment, only one of the shells/plates are welded or otherwise adhered to the remainder of the housing body. In some embodiments, two or more or all of the plates can be welded to the remainder of the housing body.

In this exemplary embodiment, at least wall 1540 is sized and dimensioned so as to enable utilitarian magnetic attraction of the magnet 686 with the shim 770. In an exemplary embodiment, the thickness of the walls 1540, 1530, and/or 1530 can correspond to the values of D11 above (the wall thicknesses can be different for two or all three walls). Different values can be utilized as well. While the embodiment just described utilizes titanium and/or titanium alloys to establish the walls, in another embodiment, a polymer such as PEEK or the like could be utilized to make one or more of the walls and/or the sidewall apparatus 1520. In an exemplary embodiment, wall 1540 can be made of a low friction material which could be different than the material of the other walls. While the embodiment described above indicated that the shim 770 could be adhered to the wall 1540 at the time of manufacture and/or before, in another embodiment, shim 770 could be instead adhered to the wall 1530. Also, while the embodiment shown utilizes flat circular plates, in an alternate embodiment, plate 1530 and/or 1540 could have a contoured arrangement that would accept the shim 770 and thus hold it in place with respect to the lateral direction. Conversely, crimping or the like can be utilized to secure the shim 770.

Further by way of example, plate 1540 by way of example could have some form of ridged structure such as that detailed above so as to reduce the surface area of the magnet that contacts the housing when the magnet 686 is attracted to the shim 770.

The dimensions and/or features detailed above with respect to the above embodiments can be applicable to the similarly situated features of the embodiment of figure 19 and thus will not be repeated here in the interests of textual economy. Still, it is briefly noted that in an exemplary embodiment, the performance features of the magnet and the shim can correspond to any one or more those detailed above with respect to rotation and/or attraction of the magnet to the shim 770.

In view of the above, embodiments include a device, such as the implantable portion of a cochlear implant or an implantable portion of an active transcutaneous bone conduction device or the implantable portion of a middle ear implant, with the implantable portion of a retinal prosthesis for example (the device can be any sensory prosthesis in some embodiments that has an implant, for example), etc., comprising a housing, such as housing assembly 1910, or housing 710, and a magnet such as magnet 686 or 687, etc., in the housing. Collectively, this can establish a magnet apparatus, such as magnet apparatus 1960 or 760, etc. In this exemplary embodiment, consistent with the concept that the implantable component is in the implantable component of a hearing prosthesis, the device is an implantable medical device. Consistent with the embodiments above, there is a clearance (e.g., D12) between an interior of the housing and the magnet in a direction of a longitudinal axis of the housing. In this embodiment, the device is configured to at least independently (e.g., without another device such as the external component) temporarily hold the magnet in direct contact with a portion of the housing and/or a body connected to or otherwise in direct contact with the portion of the housing (shim 770 or the ridge or bearings, etc.), the portion being made of the same material and/or having a configuration and/or structure that is at least similar (e.g., similar wall thickness, similar diameter in the case of the wall plates 1530, 1540 and 1550, etc.) as a portion of the housing on an opposite side of the portion of the housing. Note that the shim is not part of the housing. Thus, in the embodiment where the shim is in the cavity of the housing, the portion would be the housing wall that supports the shim. In this embodiment, the device is configured to enable the magnet to rotate inside the housing when the magnet is subjected to a torque from a magnetic field.

In an embodiment, the device is configured to enable the magnet to rotate while holding the magnet in direct contact with the portion of the housing or the body connected to the portion of the housing. This can correspond to the embodiment of FIG. 9. In an embodiment, the device is configured so that the magnet decontacts with the portion of the housing or the shim and then rotates when an external device (e.g., the external sound processor of the cochlear implant) that is used in conjunction with the device is placed into magnetic communication with the magnet to hold the external device against skin of the recipient via magnetic communication between the external component and the magnet.

In an embodiment, the device is configured to hold the magnet in direct contact with the portion of the housing. In an embodiment, the device is configured to prevent the magnet from directly contacting the portion of the housing.

In an embodiment, the device includes a magnetic material, such as the shim, to which the magnet is attracted, thus holding the magnet in contact with the portion. In an exemplary embodiment, the magnetic material is a magnet, while in another embodiment, the magnetic material is a non-magnet.

In an embodiment, the device includes a magnetic component, such as the shim 770, and the magnetic component is one of located outside an interior of the housing (e.g., such as the embodiment of FIG. 19) or located inside the housing (e.g., FIG. 9). In this device, at least one of: (1) the device is configured to at least temporarily hold the magnet in direct contact with a portion of the housing if the magnetic component is located outside the interior of the housing; or (2) the device is configured to at least temporarily hold the magnet in direct contact with the body, wherein the magnetic component is the body. In an embodiment, the "temporary" is halted upon movement of the magnet towards the magnet of the external component as detailed above.

The magnetic material results in an application of a force that biases the implant magnet so that it is always pulled or pushed against one of the internal surfaces of the magnet casing or the material or a component between the casing and the magnet, so as to prevent free movement of the magnet in the casing in a longitudinal direction but enabling rotation (in some embodiments, rotation is also prevented) at least in the absence of the external component. The magnetic material can be a ferromagnetic material (for example, iron, nickel, steel, etc., any material to which the magnet can attract that will not have a deleterious result can be used in some embodiments).

Moreover, consistent with the use of the device in the head, of a human, the device has a skull facing side and a skin facing side and the portion is on the skull facing side. In an embodiment, where the device includes a magnetic material, such as the shim, in addition to the magnet, the magnet is attracted (or the magnet is repelled form the magnetic material, in another embodiment), thus holding the magnet in contact with the portion, wherein the magnetic material is unmagnetized and/or does not impart a significant rotational alignment force onto the magnet. With regard to the latter feature, in the absence of other magnetic fields, such as the external magnet, the magnet can be oriented at any orientation in the housing. Put another way, if the magnet apparatus is shaken, permitting the magnet to unlock, when the magnet is relocked at the end of the shaking, the angular orientation of the magnet at the end of the shaken the will be determined by chaos theory. That is, no specific orientation will be favored over any other orientation at least with respect to the effects of the magnetic component.

In an exemplary embodiment, there is a device, such as any of the medical devises detailed herein. The device comprises a housing and a planar magnet located in the housing (ideally, hermetically sealed in the housing), establishing a magnet-housing assembly. The planar magnet can be a disk magnet as seen above. A planar magnet is a magnet that has a surface that has a major surface that has a plane (and thus, for example, not a spherical magnet or a cylindrical magnet). The magnet could be another type of magnet, as long as it has a plane (in other embodiments, the magnet is not a planar magnet). Consistent with the embodiments above, the device is an implantable medical device, and the magnet-housing assembly is configured with an anti-rattle apparatus to reduce a rattling effect of the magnet vis-à-vis contact with the magnet and the housing while enabling the magnet to rotate about a structural longitudinal axis (an axis defined by the structure (and is thus "imaginary"), as opposed to a magnetic axis for example - the magnetic axis can correspond / be concentric and be parallel with the structural longitudinal axis in some embodiments - embodiments do not require a spindle on which the magnet rotates, and this is not to what a structural longitudinal axis refers (but embodiments can include such)) of the magnet (e.g., axis 799) while the magnet is in the housing (albeit when the magnet is unlocked in some embodiments). In this regard, the shim 770 and the magnetic attraction of the magnet to the shim will cause the magnet to be held in place against the housing or the shim or the component between the housing. This will prevent the magnet from moving around in the housing, at least with respect to a direction parallel to the longitudinal axis (as will be detail below, embodiments also include magnetic attraction in the lateral direction as will be described below, although as noted above, in some embodiments, the housing sidewalls can be magnetic material). In any event, the idea is that when the magnet apparatus is exposed to a force, or more accurately, an acceleration and then deceleration / reverse acceleration, etc., if there is a clearance between the magnet and the housing according to one or more of the clearances detailed herein, and there is no shim or otherwise no material to which the magnet can be attracted, the magnet could move back and forth and otherwise bounce against the walls of the housing (side and/or lateral wall(s)) establishing the interior cavity. Conversely, if there is magnetic attraction of the magnet to the shim (or repulsion if the shim is magnetized for example), a higher acceleration will be required to result in the magnet moving away from the housing or otherwise away from whatever component the magnet is directly contacting owing to the magnetic attraction (or repulsion). The idea is that by establishing a sufficient attractive force that requires a higher acceleration relative to general accelerations that might be experienced during normal life (e.g., walking, running, standing up from sitting, sitting from standing up, shaking one's head in disagreement, nodding one's head in agreement, etc.), the magnet will remain in contact with the wall of the housing (or whatever component is there supporting / that contacts the magnet), and thus the magnet will not rattle. Of course, a sufficient amount of acceleration would cause at least a tick (a characterization of the sound of the magnet striking the wall of the housing if the magnet is free to move in the lateral direction for example). Thus, for example, say someone jumps into bed and lands on his or her side, there might be a tick-tick as the magnet moves away from the shim, hits the far wall of the housing, and then is reattracted back towards the shim, and hits the shim or the wall of the housing between the shim and the magnet, etc. Say someone engages in parachuting, or jumps from a tree limb 5 or 6 or 7 feet above the ground. There might be a tick-tick as the person lands (owing to the lateral movement of the magnet). However, typically, there will not be a repeated ticking /rattling. By way of example only and not by way of limitation, say a recipient is walking. Without the antirattle feature, every step might cause a tick. With the antirattle feature, there might never be any ticks, but in some embodiments or otherwise some scenarios, the only time there might be tick is when the recipient takes a hard step, such as when the recipient reaches the bottom of a flight of stairs, and then there might be the recovery tick, but that would be the end. This as compared to a magnet in a housing, where there is sufficient clearance for the magnet to rotate, where every time the person moves a sufficient amount / experiences an acceleration or deceleration of a sufficient amount, there is a tick. This tick would be noticeable if the recipient has normal hearing in the contra-lateral ear. The tick could be heard by a third party and/or be picked up by a microphone of the prosthesis (implanted or external microphone).

To be clear, the tick / rattle can occur owing to the movement of the magnet in the lateral direction and/or in the longitudinal direction.

Accordingly, in an exemplary embodiment, the devices systems and methods disclosed herein can include reducing the number of ticks and/or energy generated by the magnet apparatus owing to movement of the magnet by at least 70, 75, 80, 85, 90, 95 or 100% or any value or range of values therebetween in 1% increments over a given period of time where an activity is continuously executed. By way of example only and not by way of limitation, a period of time could be at least 1, 2, 3, 4, 5, 6, 7, 8, 9 or 10 minutes and the activity could be walking on flat asphalt while wearing tennis shoes at a rate of 1, 2, 3 or 4 miles per hour. Subjectively, the number of ticks could be reduced by 95% or for example or any of the aforementioned percentages in other scenarios or other embodiments, for that person, and objectively, the number of ticks / energy could be reduced by that percentage or any of the percentages just detailed for a person who is walking across asphalt that is flat wearing tennis shoes who is a 20, 25, 30, 35, 40, 45, 50, 55, 60, 65, 70, 75 or 80 percentile human factors engineering (or any percentage therebetween or range of percentages in 1 percentage increments) male or female of 5, 10, 15, 20, 25, 30, 35, 40, 45, 50, 55, 60, 65, 70, 75 or 80 years of age or any value or range of values therebetween in one year increments as of November 11, 2021, which person was born in the United States, European Union (or a current member state as of November 11, 2021, or the predecessor of the current member state(s)), Australia, or the People's Republic of China. In an exemplary embodiment, the aforementioned performance features can be for any of the human factors engineering humans just detailed who is running (or jogging) at a rate of 3, 4, 5, 6, 7 or 8 miles per hour (all at a steady pace).

In an exemplary embodiment, the aforementioned rhythmic activity, such as walking, imparts lateral forces onto the magnet, which would cause the magnet to move in the lateral direction (normal to the longitudinal axis of the magnet), and thus cause the magnet to strike the lateral sides of the housing, and thus cause a ticking every time or most every time that the magnet strikes the housing. By designing the housing and the magnet and the shim so that the magnetic attraction between the shim and the magnet is such that there are sufficient friction forces on the magnet so as to prevent the magnet from moving in the lateral direction, the ticking can be reduced and/or eliminated. (This can be accomplished by, for example, limiting / reducing the movement(s) as noted above. by way of example only and not by way of limitation.) In this regard, the phenomenon is akin to how a magnet placed on a refrigerator maintains its position and does not fall downward owing to gravity. The magnetic attraction and the size and the shape of the magnet and the overall contact surface area between the magnet and the refrigerator and the friction coefficients of the material can prevent the magnet from sliding downward in the face of a 1G environment. (Indeed, every time a refrigerator door is shut, hard, the magnet may move slightly downwards, but over a year of opening and closing, the movement may be de minimus.) In some embodiments where the magnet can unlock, the magnet would be in the locked configuration. When the magnet is unlocked, and thus is free at least in part of contact between the surface to which it previously abutted owing to the magnetic attraction with the shim, the magnet can then rotate and align with the external device to the extent such rotation is needed, at least until the magnet reaches the opposite side of the housing. But note also that embodiments herein can have utilitarian value to non-rhythmic events as well, such as an activity that creates a repeated forces but is irregular/arhythmic (basketball would be an example, another example would be driving on a rough or unpaved road). Thus, embodiments can be directed to dynamic activities by way of example. Accordingly, the disclosure herein relating to rhythmic activities corresponds to an alternate disclosure of dynamic activities that are arhythmic, providing that the art enables such. And to be clear, the teachings herein are applicable to one off dynamic events (with respect to a given temporal period, such as 2, 3, 4 or 5 seconds).

Consistent with the teachings above, the anti-rattle apparatus can enable the magnet to move in the direction of the structural longitudinal axis (e.g., axis 799). And in some embodiments, the rattle / ticking is caused by movement of the magnet in that direction. This fact is not contradictory. The antirattle apparatus also temporarily prevents the magnet from moving in that direction as noted above, hence establishing the antirattle feature. The anti-rattle apparatus can enable the magnet to move in the direction of the structural longitudinal axis away from a bone facing side of the housing when the device is implanted in a human when an external device that transcutaneously interacts with the device is held against the human via magnetic interaction between the planar magnet and the external device.

Note that in at least some exemplary embodiments where magnetic material is located between the implanted magnet and the external component, the magnet will always be biased towards the skin, and thus upon placing the external component against the skin, there will be no tick because the magnet will not have to move from one side of the housing to the other side of the housing (away from the bone towards the skin) because the magnet will already be on the side of the housing that faces the skin. Conversely, there might be a slight tick when the magnetic material is located on the skull facing side of the housing when the external component is coupled to the implant owing to movement of the magnet from the skull facing side toward the skin facing side. Of course, this may not be noticeable to the person and certainly will not be rhythmic during almost all circumstances (other than playing around, one is not going to remove and then replace the external component against the head of the recipient in a manner that has a recognizable frequency).

Owing to the use of the magnetic body (shim), the anti-rattle apparatus relies on a magnetic attraction to pull the magnet towards a bone facing side of the housing when the device is implanted in a human, and/or the anti-rattle apparatus is configured to unlock the magnet upon an external device that transcutaneous interacts with the device coming into magnetic communication with the planar magnet. The anti-rattle apparatus enables the magnet to move in the direction of the structural longitudinal axis only upon an application of a minimum force of the values noted above in the longitudinal direction. And in some embodiments where the anti-rattle apparatus includes a magnetic material, the magnet interacts with the magnetic material to establish an anti-rattle feature of the anti-rattle apparatus and the magnetic material is physically separated from contact from the magnet. (Physical separation, as that phrase is utilized herein is not established by mere coating on the magnet or the magnetic material or because a thin layer of fluid might be located between the two. Physical separation can be established by the housing wall or by the ring that establishes the ridge or by the bearings.)

Note that the concept of antirattle does not mean that there is never any rattle. As used herein, antirattle is rattle prevention/limitation in a manner that is significant enough to eliminate most rattles that would otherwise exist during common experiences.

Consistent with the teachings above regarding diametrically polarized magnets, etc., the planar magnet in at least some embodiments has a magnetic polarity axis that is offset from the structural longitudinal axis. Still, embodiments can be utilized with an axial magnetized magnet. Embodiments can utilize a magnet that is not a spherical magnet.

Continuing with an explanation regarding a device that has a housing and a magnet, wherein the magnet is hermetically sealed in the housing, the magnet and the housing establishing a magnet-housing assembly, in an embodiment, the device is configured to provide relievable contact of the magnet relative to a portion of the magnet-housing assembly via magnet attraction between the magnet and a magnetic component of the device. Here, the shim or the ring with the ridge or the bearings, etc., can be part of the magnet-housing assembly. (Magnet-housing assembly can be the magnet apparatus 1960 for example, or can be magnet apparatus 760 of FIG. 16, for example.) Again, where the device is configured to retain an external component via transcutaneous magnetic attraction established at least in part by the magnet, in an embodiment, the device is configured so that the relievability of the contact is established upon the establishment of the transcutaneous magnetic attraction. For example, the magnet moves from the location seen in FIG. 11 to the location seen in FIG. 12 because the magnet 604 pulls the magnet 687 away from the shim 770 upwards (or horizontally when the device is implanted in the head - still the direction of axis 799).

In an embodiment, the magnet is a non-spherical magnet that has a magnetic polarity axis that is more diametrically magnetized than it is axially magnetized (e.g., as measured from the longitudinal axis, the magnetic flux enters and leaves the magnet at an angle 50 degrees or 60 degrees therefrom). The magnet-housing assembly can have an overall thickness that is no greater than 8 mm or 7 mm or 6 mm or 5 mm or 4 mm or 3 mm or 2 mm. And referring back to the portion of the magnet-housing assembly, the portion of the magnet-housing assembly is a wall of the housing or a component of the magnet-housing assembly separate from the magnet and stationary relative to the housing, the component of the magnet-housing assembly corresponding to the magnetic component. And further to the above, the magnetic component has a thickness that is less than 250, 200, 150, 100, 90, 80, 70, 60, 50, 40, 30 or 20 or 10 microns, and a diameter of less than 15, 14, 13, 12, 11, 10, 9, 8, 7, 6, or 5 mm.

In an exemplary embodiment, the magnetic component is a circular plate having a rectangular cross-section in a plane lying on and parallel to the longitudinal axis, and a circular cross-section in a plane normal to the longitudinal axis. In an exemplary embodiment, the total mass of the magnetic component is less than, or equal to 20, 19, 18, 17, 16, 15, 14, 13, 12, 11, 10, 9, 8, 7.5, 7, 6.5, 6, 5.5, 5, 4.5, 4, 3.5, 3, 2.5, 2, 1.5, 1, 0.9, 0.8, 0.7, 0.6, 0.5, 0.4, 0.3, 0.2, 0.1, 0.09, 0.08, 0.07, 0.06, 0.05 or 0.04 grams or any value or range of values therebetween in 0.001 gram increments. Embodiments typically have at least one flat surface. Different shaped magnetic components can be used, as will be describe below, but briefly, elliptical shaped plates / shims / bodies, triangular, hoop, square, rectangular, pentagon shaped plates / shims bodies can be used.

In an exemplary embodiment, the total mass of the magnet (or compilation of magnets) of the external device is less than, or equal to 20, 19, 18, 17, 16, 15, 14, 13, 12, 11, 10, 9, 8, 7.5, 7, 6.5, 6, 5.5 or 5 grams or any value or range of values therebetween in 0.01 gram increments. In an exemplary embodiment, the total mass of the implanted magnet (or complication of implanted magnets) less than, or equal to 7, 6.5, 6, 5.5, 5, 4.5, 4, 3.5, 3, 2.5, 2, 1.5, 1, 0.9, 0.8, 0.7, 0.6, 0.5, 0.4, 0.3, 0.2 or 0.1 grams or any value or range of values therebetween in 0.01 gram increments.

In an exemplary embodiment, there is only one magnetic component in the magnet apparatus. As will be described below, there can be two or more magnetic components. In an exemplary embodiment, the magnet apparatus is devoid of magnetic material other than the magnet and the magnetic component(s). In an exemplary embodiment, the aforementioned masses constitute all of the magnetic material of the magnet apparatus other than the magnet. That is, outside of the magnet, there is no more magnetic material than the aforementioned masses.

As noted above, FIGs. 33 and 34 provide additional features of friction management. The embodiment of FIG. 33 parallels the embodiment of FIG. 7A noted above. In this regard, there is a magnet apparatus 3360, which can correspond to magnet apparatus 760 noted above, with the inclusion therein of a body 3310, which can be a polymer insert / plastic insert. In this embodiment, the body 3311 is a PTFE insert located inside the housing 710, and is in direct contact with the magnet 686 (where the magnetic material thereof is a magnetic body coated with a metal, such as platinum or gold, and thus the body 3311 is in direct contact with the gold coating when the body 331 is in direct contact with the magnet 686). In an embodiment, the body 3310 is a PTFE disk with a circular outer circumference and to flat planar sides on the top and the bottom. The concept here is that the PTFE disk 3311 has known surface features vis-à-vis friction (and note that the features on one side can be different from those of the other side). These friction features are different from the friction features of the inside of the housing that would otherwise contact the magnet 686. The housing structure and design can be implemented in a utilitarian manner for the general desired purposes of being a housing (e.g., to provide a barrier between the inside of the housing and the outside of the housing), free of design constraints related to friction (at least with respect to friction between the magnet and the housing), and then the body 3311 can be designed to achieve the desired friction features.

In an embodiment, the body 3311 is adhered to the housing 686 with an adhesive or by welding. Corollary to this is that instead, the body 3311 can be adhered to the magnet 686. In an embodiment, the body 3311 is not adhered to the housing or the magnet, and can "float" in a manner that would be analogous to how the magnet 686 floats (if the shim 770 was not there at least) in the housing 710.

The diameter (or more accurately, surface area) of the body 3311 (or the shim in an embodiment where the body has a larger diameter than the shim, and the shim is what creates the support area, where the body does not have sufficient rigidity to provide meaningful support outside the perimeter of the shim) can be set by design to set the desired friction effect, although alternatively, there could be cancellation because force per unit area increases as area decreases, and there is thus a cancellation effect. The material and/or the surface roughness can be set by design to set the desired friction effect. The combination of the two can be implemented to set the friction effect. Indeed, in an embodiment, the magnetization force between the shim and the magnet is also set to achieve a given friction feature. Thus, three variables (surface roughness, material characteristic and force) can be set by design to achieve a desired friction feature. That said, the magnetization force will, in at least some embodiments, be set for purposes of anti-rattle, etc. (anti-rattle will drive the magnetization design) as opposed to the friction feature, and other variables will be varied to achieve the friction feature.

FIG. 34 shows another embodiment that parallels the embodiment of FIG. 19 in some ways, where magnet apparatus 3460 includes the walls 1530 and 1550 used to seal the housing, but where shim 770 is located inside the compartment of the housing with the magnet 686 (there is no wall 1540 in this embodiment, but concomitant with the disclosure herein of embodiments where any feature herein can can be combined with any one or more other features herein providing that the art enables such, the body 3311 could be used with the embodiment of FIG. 19, where wall 1540 separates the body 3311 from the shim 770, in a manner analogous to the separation of the body 3311 from the shim 770 vis-à-vis the embodiment of FIG. 33). Also in this embodiment, the diameter of the body 3311 is different from the embodiment of FIG. 33. As with the embodiment of FIG. 33, the body 3311 can be adhered to wall 1530 or not, or to magnet 686 or not.

A diameter of the body 3311 (the horizontal distance of FIG. 33 and FIG. 34 of the body) can be the value of D2 or D6 or D6+D4 or any value in between any of those values, or less than any of those values. In an embodiment, the diameter of the body 3311 is less than or equal to or greater than, if applicable, to 140, 135, 130, 125, 120, 115, 110, 109, 108, 107, 106, 105, 104, 103, 102, 101, 100, 99, 98, 97, 96, 95, 94, 93, 92, 91, 90, 89, 88, 87, 86, 85, 80, 75, 70, 65, 60, 55, 50, 45 or 40% or any value or range of values therebetween in 0.01% increments of the value of D2, D6 or D6+D4 (the diameter would not be larger than D6+D4, hence the "if applicable" - these numbers are provided in this format for textual economy - and note that in some embodiments, the diameter would be slightly less than the combined D6+D4, so as to avoid buckling / to ensure that the body can be laid totally flat). In an embodiment, a thickness of the body 3311 (the distance in the vertical direction) is less than or equal to 5, 10, 15, 20, 25, 30, 35, 40, 45, 50, 55, 60, 65, 70, 85, 80, 85, 90, 95, 100, 110, 120, 130, 140, 150, 160, 170, 180, 190, 200, 225, 250, 275 or 300 microns or any value or range of values therebetween in 1 micron increments. The thickness can be linked to the thickness of the shim D1, which can be greater than, less than or equal to 500, 450, 400, 350, 300, 250, 200, 150, 140, 130, 120, 110, 100, 90, 80, 70, 60, 50, 40, 30 or 20% or any value or range of values therebetween in 1% increments of the value of D1.

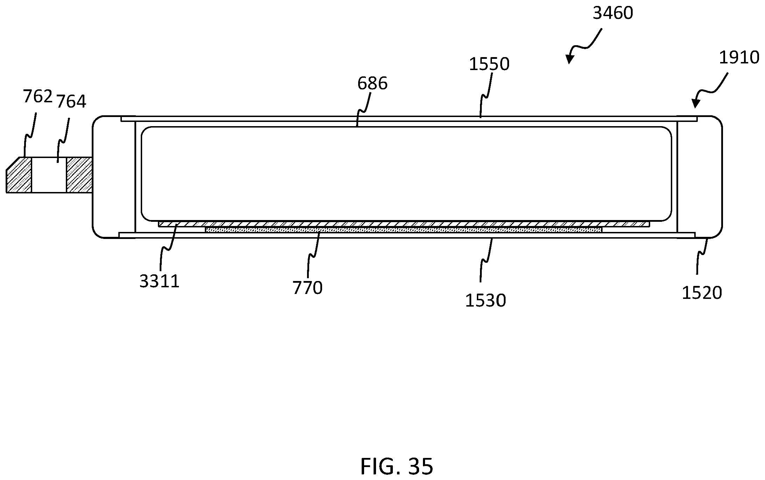

FIG. 35 shows an alternate embodiment where the body extends past the outer diameter of the shim 770. Here, the shim 770 is smaller than the shim 770 presented above in FIGs. 34 and 33, but this arrangement could be applicable to a larger shim as well.

In an embodiment, the body 3311 is made by punching out a disk / from a thin sheet (a sheet having the thickness of the end body). In an embodiment, the disk is a solid body, while in other embodiments, there can be one or more holes therethrought, such as a hole in the center of the body. And note that while a body with a circular outer profile is presented herein, in an alternate embodiment, a different shape can be used, such as a square or an octagon or some form of polygon shape (the shape when viewed looking down the longitudinal axis of the magnet 686).

In an embodiment, the surface that contacts the magnet 686 is smooth so as to reduce friction.

In an embodiment, the body 3311 is made of PTFE or PEEK or Polyamide. Non-polymers with utilitarian friction properties that are non-magnetic can be utilized, such as phosphor bronze, etc. The material can be selected based on the desired friction features (some embodiments may want increased friction as a result of the use of the body relative to that which would otherwise be the case, as noted above and as will be detailed below). The body 3311 can be any polymer that can have utilitarian value, or any other material that can have utilitarian value. In an embodiment, a low friction material is used. Moreover, the body 3311 can be a body that will not cause "wear" on the magnet 686 (raw magnetic material or the coating thereof), which will create "dust" (wear particles) inside the housing.

The use of the body 3311 can provide a "tuned" arrangement that can enable the magnet 686 to rotate when exposed to an MRI field so that the magnet 686 can magnetically align with the magnetic field of the MRI when a recipient of a device having the magnet apparatus is exposed thereto, while also preventing or at least limiting the propensity of the magnet to move laterally (left-right / into-out of the page of FIG. 33 and FIG. 34) when the magnet 686 is exposed to forces in the lateral direction (such as when the recipient is walking - recall that the alignment of the magnet apparatus is typically 90 degrees from that shown in FIGs. 33 and 34, because the magnet apparatus is implanted flush with the skull and/or skin on a side of the recipient's head). This can have utilitarian value with respect to establishing MRI compatibility and preventing "rattle" or "ticking" of the magnet during life activities.

In at least some embodiments, the attraction force between the magnet 686 and the shim 770 is sufficiently high and the surface(s) at issue are such that the friction between the body 3311 and the magnet 686 or between the body 3311 and an inside of the housing wall (or both for a floating body 3311) prevents the magnet 686 from moving (or at least limits the movement) with respect to the G-forces noted herein. In an embodiment, the body 3311 creates an increased friction relative to that which would be the case if the magnet 686 was against the shim 770 or against the housing. In an embodiment, the friction force (or alternatively, a force needed to be applied to move the magnet in the housing) is increased by at least and/or equal to 10, 15, 20, 25, 30, 35, 40, 45, 50, 55, 60, 65, 70, 75, 80, 85, 90, 95, 100, 110, 120, 130, 140, 150, 200, 250, 300, 350, 400, 450, 500, 550 or 600% or more or any value or range of values therebetween in 1% increments relative to that which would be the case in the absence of the body 3311, all other things being equal (such as, for example, the distance between the shim 770 and the magnet 686 is the same). That said, in an embodiment, the friction force (or the force needed to move the magnet in the housing) is decreased by at least and/or equal to 10, 15, 20, 25, 30, 35, 40, 45, 50, 55, 60, 65, 70, 75, 80, 85 or 90% or more or any value or range of values therebetween in 1% increments relative to that which would be the case in the absence of the body 3311, all other things being equal.

While the embodiments shown have the body 3311 located on one side of the magnet 686, in other embodiments, there is a body 3311 located on both sides of the magnet 686, and the bodies can be identical or can be different from each other (different diameter, different thickness or different shape for example). Note further that embodiments can include layered bodies as well (two or more bodies can be stacked one on top of the other, and here too the bodies can be different from each other (different thickness and/or different diameter and/or different shape).

In view of the above, in an embodiment, there is a device, such as the implantable magnet apparatus detailed herein, that includes a friction body that is located between the magnet and the housing, in direct contact with the magnet. In an embodiment, there is a non-magnetic component that is located between the magnet 686 and the magnetic component (e.g., the shim 770), located inside the housing, that one of increases or decreases a force needed to be applied to the magnet to move the magnet in the housing relative to that which would be the case in the absence of the non-magnetic component. In some embodiments, it increases the force, and in other embodiments, it decreases the force.

In some exemplary embodiments, there is a method where the magnet remains stationary within the housing during the action of executing the rhythmic activity, and this is owing to a friction surface (e.g., established by the body for example) within the implanted housing. As used herein, a friction surface or friction component or friction body is a surface that is used to achieve a friction result (whether that be higher friction or lower friction). A friction surface / component / body does not mean high friction. In this embodiment, the magnet would not remain stationary within the housing during the action of executing the rhythmic activity in the absence of the friction surface, all other things being equal. In an embodiment, the rhythmic activity is any one or more of those detailed herein. In an embodiment, the rhythmic activity imparts an acceleration according to any one or more of the values detailed herein.

In an embodiment, the magnet remains stationary within the housing during the action of executing the rhythmic activity owing to a friction surface within the implanted housing, and the magnet would rotate within the housing when exposed to a torque of a certain value but would not rotate within the housing in the absence of the friction surface when exposed to that same torque.

In an embodiment, the portion of the magnet-housing assembly described herein is a polymer body located within the housing but separate from the housing. In an embodiment, it is part of the housing. In an embodiment, the portion of the magnet-housing assembly is a friction component (e.g., the body) of the magnet-housing assembly separate from the magnet and separate from a wall of the housing, the friction component of the magnet-housing assembly being located between the magnet and the magnetic component. As seen above, in an embodiment, the compartment containing the magnet has a "stack" of the shim, the body and the magnet, from bottom to top, and the shim can be adhered to the interior of the housing in an exemplary embodiment. In this embodiment, the magnet is in direct contact with a polymer body.

As noted above, in some embodiments, there is a physical separation present between the shim (or whatever magnetic material component is the case) and the magnet. here, this can be established by a non-magnetic body located in the housing, such as the body 3311. In an embodiment, the shim (or whatever magnetic material component is the case) is located in a compartment of the housing in which the magnet is located.

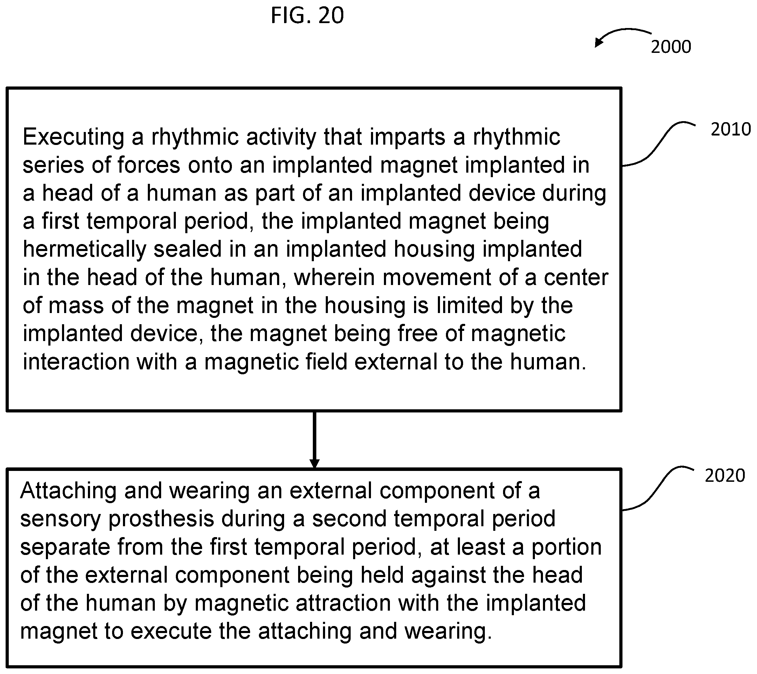

Embodiments include methods. FIG. 20 shows an exemplary flowchart for an exemplary method 2000 according to an exemplary embodiment. Method 2000 includes method 2010, which includes executing a rhythmic activity that imparts a rhythmic series of forces onto an implanted magnet implanted in a head of a human as part of an implanted device during a first temporal period, the implanted magnet being hermetically sealed in an implanted housing implanted in the head of the human, wherein movement of a center of mass of the magnet in the housing is limited by the implanted device, the magnet being free of magnetic interaction with a magnetic field external to the human. This can be walking, running for example. But it is noted that exemplary methods are not limited to vertical rhythmic activities. Horizontal rhythmic activities can also be executed. The rhythmic activities imparts an oscillating or varying acceleration / deceleration on the magnet. In this method, there is no external component attached to the head. That is, the implanted device is an implanted component of a hearing prosthesis, and there is no external component that cooperates with the implanted component during the rhythmic activity (it can be used later and/or before). In an embodiment, the implanted device is a total implantable hearing prosthesis, and includes an implanted microphone, so the device can evoke a hearing percept based on ambient sound without the external device. (This is another reason to avoid the rattle - the rattle could be picked up by the implanted microphone.) Of course, the person could simply not want to hear anything and/or use the prosthesis while engaging in the rhythmic activity in the case where the implanted device is a partially implanted sensory prosthesis.

In an embodiment, the rhythmic activity is walking at least 50, 75, 100, 125, 150, 200, 250, 300, 350, 400, 450 or 500 feet on a level surface, walking up or down a flight of stairs or two or three or four flights of stairs. In an embodiment, the rhythmic activity is running instead of walking. In an embodiment, the rhythmic activity is any of those detailed herein with the given limitations.

In an embodiment, during the rhythmic activity, the implanted magnet is held away from a side of the housing facing outer skin of the human.

Method 2000 also includes method 2020, which includes attaching and wearing an external component of a sensory prosthesis during a second temporal period separate from the first temporal period, at least a portion of the external component being held against the head of the human by magnetic attraction with the implanted magnet to execute the attaching and wearing.

In some embodiments, during the action of attaching, the magnet rotates about a longitudinal axis due to interaction with a magnet of the external component. In an embodiment, the magnet rotates at least 30 degrees about its longitudinal axis. In an embodiment, the center of mass of the magnet moves relative to the housing during the action of attaching towards the external component and away from where the center of mass was located when movement of the center of mass was limited. This is the movement from FIG. 11 to FIG. 12, for example.

And note that rotation is not movement of the center of mass relative to the housing, at least not for symmetrical magnets, as a center of mass is a point, and the magnet would rotate about that point.

In an embodiment, the movement is greater than and/or less than and/or equal to 5, 10, 15, 20, 30, 40, 50, 60, 70, 80, 90, 100, 125, 150, 175, 200, 225, 250, 275, 300, 325, 350, 375, 400, 450 or 500 microns or any value or range of values therebetween in 0.1 micron increments.

In an embodiment, rotation is solely about one axis. There is no rotation about another axis. In an embodiment, any rotation about any other axis is *de minimis.*

In an embodiment, there can be rocking of the magnet about an axis normal to the longitudinal axis of the magnet. In an embodiment, there can also be rotation about the longitudinal axis of the magnet. Further, there can be rocking about a second axis normal to the axis normal to the longitudinal axis, where the second axis is also normal to the longitudinal axis.

In an embodiment, the magnet cannot rotate about an axis normal to the axis of rotation of the magnet / the longitudinal axis / the axis normal to the skull / skin / the external component when used, more than 30, 25, 20, 15, 14, 13, 12, 11, 10, 9, 8, 7, 6, 5, 4, 3, 2 or 1 degrees or any value or range of values therebetween in 0.1 degree increments (rotation being relative to the housing - the housing might rotate / twist / turn in some embodiments).

In some scenarios, during the rhythmic activity, the implanted magnet is held away from a side of the housing facing outer skin of the human and an opposite side of the housing from the side of the housing facing outer skin of the human. This can be done according to an embodiment further described below. However, in some scenarios, during the rhythmic activity, the implanted magnet is held away from a side of the housing facing outer skin of the human and/or held against an opposite side of the housing (or at least held against a component located on an opposite side of the housing) from the side of the housing facing outer skin of the human. In some scenarios, during the rhythmic activity, the implanted magnet is held away from a side of the housing facing outer skin of the human. In some embodiments, the holding is achieved by attraction of the magnet to a magnetic material at the side of the magnet.

In an exemplary embodiment, an acoustic energy level radiating from the housing is less than 40, 35, 30, 25, 20, 19, 18, 17, 16, 15, 14, 13, 12, 11, 10, 9, 8, 7, 6 or 5 Decibels SPL or any value or range of values therebetween in 0.1 dB SPL increments during execution of the rhythmic activity. This can be a mean, median and/or mode value. This can be a maximum value. In an embodiment, the energy level is zero (e.g., because the magnet does not move - note that in some scenarios, the magnet moves but does not release and then contact from the interior of the cavity - the magnet could slide along the contacting surface and/or rotate, and that would not be zero dB, but would be a low energy level).

In an embodiment, the shim(s) 770 or whatever magnetic body is being utilized is of a structure (sized and dimensioned and made of a given material) that results in the shim being magnetically saturated by the magnet 686 (and corollary to this, the magnet 686 is such that the magnet magnetically saturates the shim). This is the case irrespective of the distance of the magnet from the shim and/or the material between the shim and the magnet. Thus, embodiments include a ferrous shim that is magnetically saturated by the magnet 686 (and is such without any other magnet being present - a magnet can be present, but the saturation is due entirely due to the magnet 686).

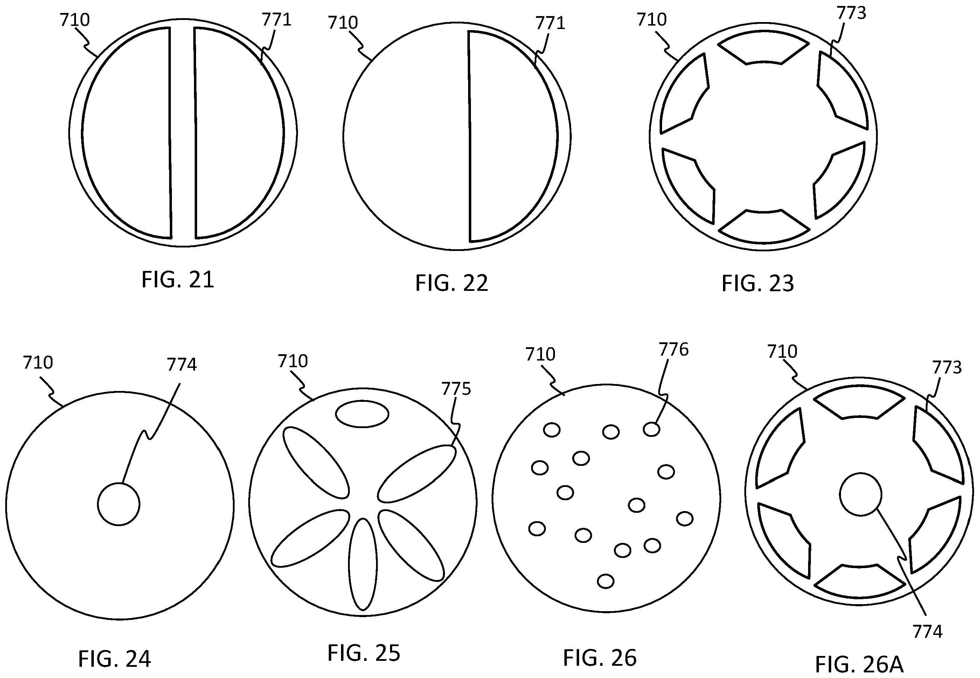

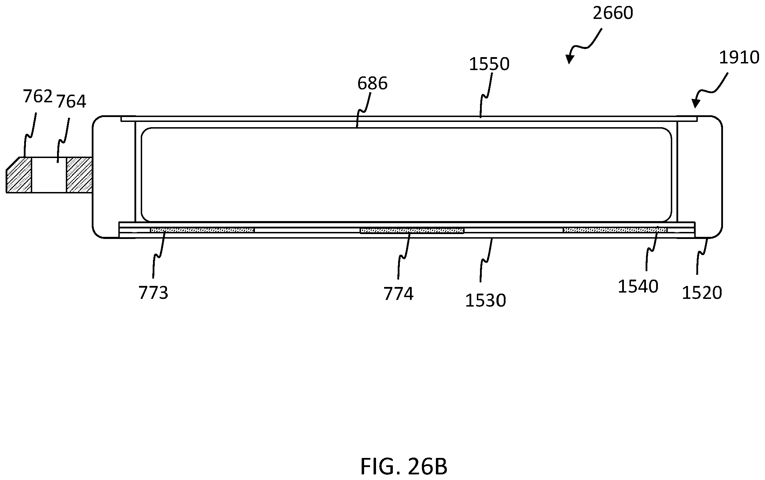

Figures 21-26 present exemplary alternative embodiments of utilizing shims. These are all views from the top and should be considered drawn to scale, although in other embodiments, those features can be of a different size and shape and otherwise not drawn to scale. The inner diameter of the cavity is shown as element 710 (interior of the housing). It is noted that the shims can be in the cavity, or can be below or above the cavity. The shims can be place in a manner concomitant with the placements disclosed above. Indeed, the shims above can be replaced with those as seen. Shim 771 is a half-moon cross-section, with flat tops and bottoms. As seen in figure 21 and 22, in some embodiments there is only one shim while in other embodiments there to shims. Figure 23 shows six shims 773 evenly dispersed relative to the interior of the housing 710, although it is noted that in alternate embodiments, the shims can be located to one side or could be more densely packed. More or fewer shims 773 can be utilized. Figure 24 shows a micro shim 774. And note that the embodiments can be combined so shim 774 can be utilized with the shims of figure 23. Figure 25 shows oval-shaped shims 775, and shows that different shims can be used / different orientations can be used, and figure 26 shows a plurality of micro shims 776 randomly distributed relative to the cavity. In these embodiments, all of the shims are plates and can have one or more of the dimensional features detailed above. FIG. 26A shows the combination of the embodiment of FIG. 23 and FIG. 24 (FIG. 26B shows the sideview of an exemplary embodiment of a magnet apparatus 2660). Note that another example can be that element 774 could instead be a ring instead of a flat plate as shown.

With respect to the cavity, in at least some exemplary embodiments, the profile the cavity is essentially a slightly larger version of the outer profile of the magnet in the cavity. That is, if the magnet is a disk shaped magnet, the interior of the cavity will be negatively disk shaped. Thus, for textual economy, the various dimensions detailed above with respect to the magnet can correspond to the dimensions of the cavity, save for the maximum dimensions of the magnet (and for those, the dimensions of the cavity can be plussed up from those by 1, 2, 3, 4, 5, 6, 7, 8, 9 or 10% or any value or range of those therebetween in 0.1% increments - indeed, this can be the case for all of the dimensions disclosed for the magnet).

With respect to the magnet and the plates in the cavity, there is an upper surface and a lower surface that is normal to the longitudinal axis of those components. For example, the upper surface of the cavity can be formed by the housing wall at the top, and the lower surface of the cavity can be formed by the housing wall at the bottom. These are the major surfaces of the cavity, and corresponding / facing surfaces of the shim and the magnet are also the major surfaces, at least when the magnet is a disk. The major surfaces have feature that have certain geometric values. For example, all or at least 90, 95 or 99% of the surfaces can be within two parallel planes that are 25 microns from each other, or 10 or 20, or 30, or 40, 50, 60, 70, 80, 90, 100, 125, 150, 175 or 200 microns from each other. Also, the top and bottom surfaces are parallel to each other within any of those values when taken at the outermost portions of the surfaces. Also as can be seen, with respect to the longitudinal axes, no part of the shim overlaps any part of the magnet. This is also the case with respect to the lateral axes. Also as can be seen, at least one of the major surfaces the shim are parallel to at least one of the major surfaces of the magnet (or both can be parallel) when the magnet is at its closest position relative to the shim. This is opposed to an arrangement where the magnet where the shim has a curved surface. In an exemplary embodiment, when the magnet in shim or at their closest approach, the aforementioned parallel features are present (e.g., one end of the bottom surface of the magnet has a distance to the closest portion of the shim, and another end of the bottom surface of the magnet furthest from the one and has a distance to the closest portion of the shim, and those two distances do not have a difference greater than the various values detailed above with respect to the parallel planes (this is geometric dimensioning and tolerancing).

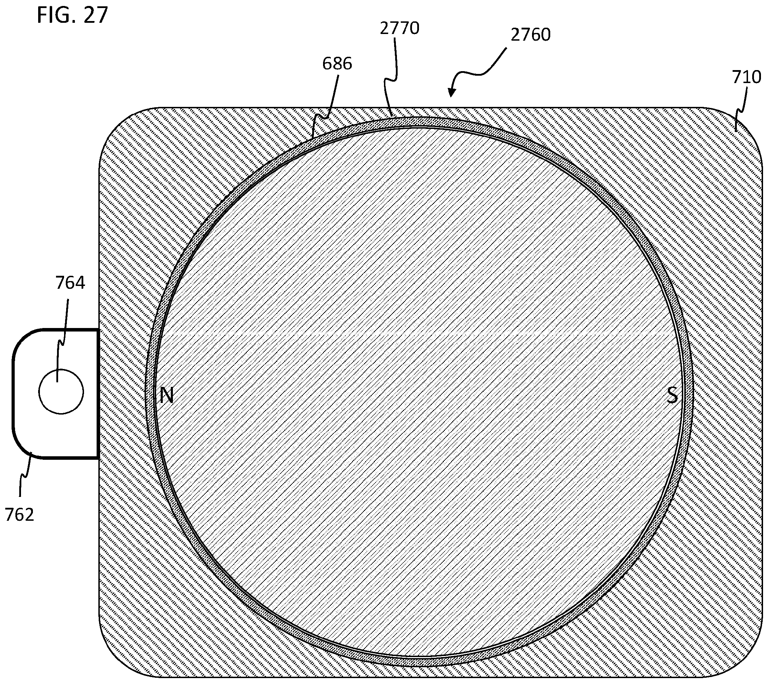

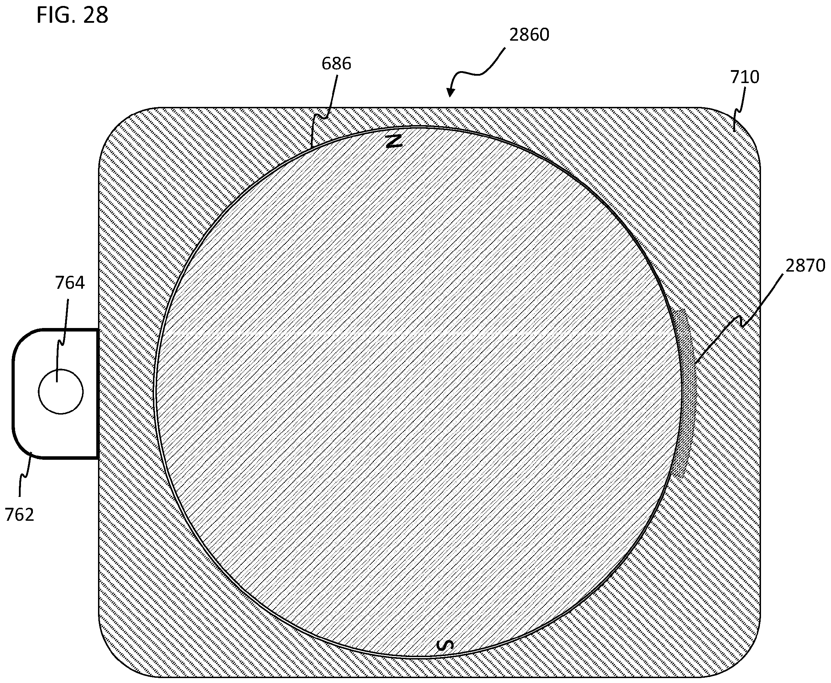

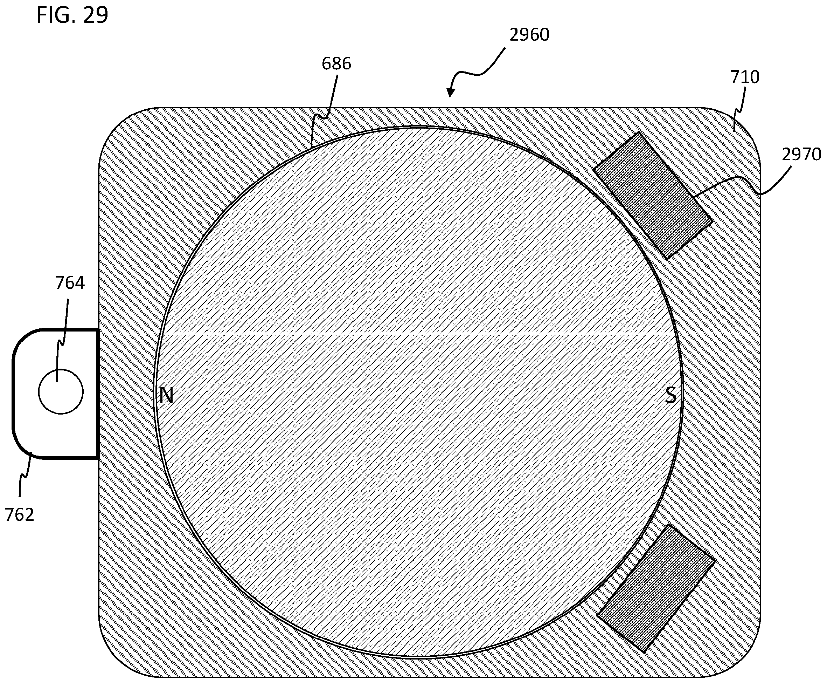

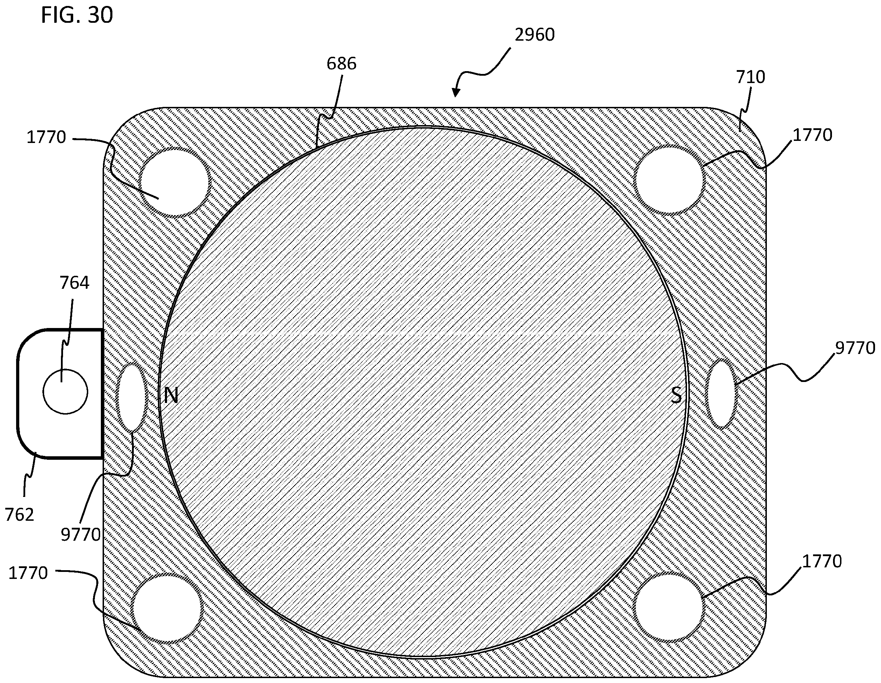

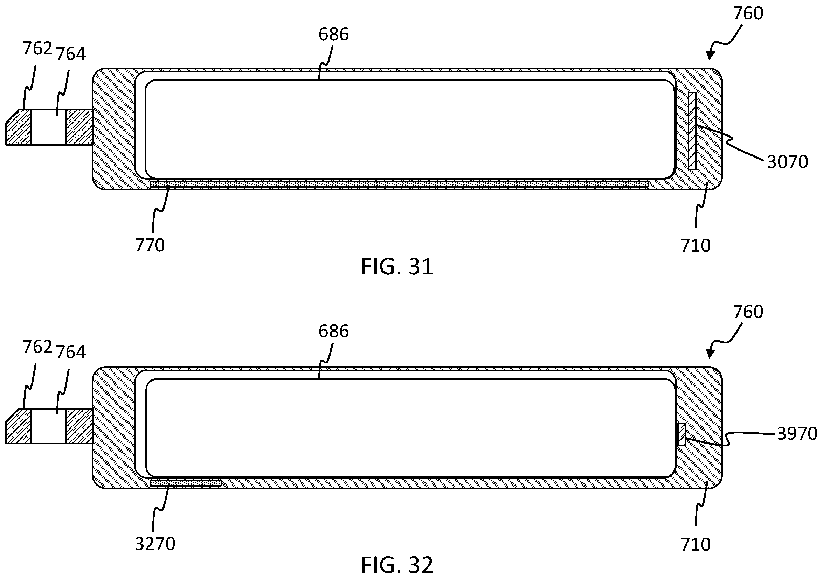

While the embodiments described above all have a shim that does not overlap with the magnet in the lateral direction, other embodiments of the shim that does overlap in the longitudinal direction. By way of example only and not by way of limitation, figure 27 shows a magnet apparatus 2760 with a hoop 2770 of magnetic material that is inside the cavity, while in other embodiments, it can be outside the cavity and surround the cavity. In an exemplary embodiment, the hoop is a ring, while in other embodiments, the hoop has a cross-section of the wall that extends from the bottom of the cavity to the top of the cavity, while in other embodiments, it is somewhere in between. The pertinent dimensions for components situated proximate the hoop can be utilized for the hoop albeit adjusted to accommodate spacing and will not be detailed for the purposes of textual economy. Moreover, the thicknesses of the shim can be utilized for the thicknesses of the hoop for example. Jumping ahead to figure 30, figure 30 shows a cross-section of a magnetic component 3070 that is a rectangular plate that extends into and out of the page. It is noted that the positioning of that component can correspond to the positioning of the hoop 2770 by way of example. It is also the case for the components shown in FIG. 31 and 32, and the hoop can be positioned elsewhere. Figure 28 presents another exemplary magnet apparatus 2860 with a magnetic component 2870 that is embedded in the wall of the housing that establishes the cavity on a lateral side of the magnet 686. The magnet 686 is pulled to the right (with respect to the view of FIG. 28, as noted above, in practice, when implanted, and the human is standing erect, the magnet would be magnetically pulled downward or upward or to the side, depending on the orientation of the implant - embodiments are envisioned where the magnet is pulled downward parallel to a direction of gravity or where the main vector of attraction is between 45, 40, 35, 30, 25, 20, 19, 18, 17, 16, 15, 14, 13, 12, 11, 10, 9, 8, 7, 5, 4, 3, 2 or 1 degrees or any value or range of values therebetween in 0.1 degree increments from the direction of gravity or from a direction normal to a direction of gravity when the person is standing erect) via the magnetic attraction. The arc of the component 2870 (which could be a bent beam of rectangular cross-section with a radius of curvature that is complementary to the cavity and/or the magnet) can be varied depending on the desired attraction. Figure 29 presents to magnetic components in the form of rectangular blocks 2970 spaced as shown, which results in attraction of the magnet to the right. While the embodiment of figure 29 shows the magnet's spaced symmetrically with respect to a plane that bifurcate the magnet apparatus 2960 horizontally, in some embodiments, the components 2970 may not be symmetrical, and in other embodiments there might be only one which can be symmetrically located or can be located is shown or any other location depending on where the magnet is desired to be held in the absence of interaction with the external magnet of the external component. Figure 30 presents an exemplary magnet apparatus 3060 that utilizes different magnetic components 1770 and 9770 as shown. These are extrusions in the direction in and out of the page having the cross-section shown in an exemplary embodiment.

In the embodiments described above with respect to the laterally positioned magnetic components, the magnetic attraction is such that the magnet is pulled to one side or otherwise is pulled in the lateral direction (and thus downward or upward or forward or backward, depending on the orientation of implantation when the human is standing erect) as opposed to the longitudinal direction (and thus to the side when the human is standing erect). That is, instead of a major surface being held against a major surface of the shim were a major surface of the cavity, a lateral surface, or more accurately, a portion of a lateral surface is held against a portion of a lateral surface of the cavity or the magnetic component or both. It is noted that while in some embodiments, the magnetic components, or more specifically, the center of masses of the magnetic components, are positioned equidistant with respect to the top and bottom of the cavity, while in other embodiments, the magnetic component, or more accurately, the center of mass of the magnetic component opposition an offset manner relative to the center of the cavity. This can have the effect of pulling the magnet in the lateral direction while also pulling the magnet in the longitudinal direction (or, more accurately, the magnet pulling itself in those directions).

As noted above, figure 31 presents an exemplary magnet apparatus that utilizes shim 770 and curved magnetic component 3070 as shown. This can have the effect of having a force that is downward and also to the right. Figure 32 presents an exemplary embodiment where there is a magnetic component that has a rectangular cross-section 3270 as shown, which can be a straight extrusion or can be an arcuate component with respect to in and out of the page, and then a magnetic component 3970 which again can be a straight extrusion or can be an arcuate component.

In an embodiment, lateral movement of the magnet is restricted, either by friction as noted above, or by laterally spaced magnetic shims (or both). For example, in an embodiment, when implanted, the magnet does not move up and down (or down and up) in the case of a bottom mounted shim (or top mounted ship - with respect to the direction of gravity) and/or forward or backward (or backward of forward) in the case of a backward/rearward located (or forward/ frontward located) shim (or magnetic material). In an embodiment, the magnet can "rock" within the housing. For example, if the shim is located at the bottom of the housing, and the cavity in which the magnet is located is circular, and the magnet is circular, and the diameter of the cavity is larger than the diameter of the magnet in the lateral direction (the housing has a greater radius of curvature than the magnet), the magnet can rock in the housing while being held against the wall. For example, the magnet could move upward and forward by "rolling" along the housing wall, and could roll back, thus rolling backwards and downward and then upward (while still rolling backwards). The magnetic attraction may permit this movement, albeit the movement might be more limited than that which would otherwise be the case without the shim.

And it reiterated that the embodiments where the magnetic material is located at longitudinal locations relative to the magnet (and thus between the skull and the magnet or between the outer surface of the skin and the magnet), the above movements and restrictions of movement can also be applicable, with or without the shim that is located at lateral sides. In an embodiment, the friction force between the major surfaces of the magnet and the housing (or a component attached to the housing / a component of the housing assembly) is sufficiently strong that the magnet will not move laterally under certain accelerations, such as any one or more of those detailed herein, in the vertical direction / the direction of gravity. Conversely, such embodiments can also still permit the magnet to rotate for alignment with the external component when the external component is placed in proximity to the implanted magnet / placed against the skin. Moreover, the lateral movement can exist, even if the magnet does not become separated from the housing or a component of the housing assembly. That is, the entire magnet can slide laterally along the surface of the housing / housing assembly, for alignment with the external component magnet. The magnet can slide and rotate as well. Embodiments can include balancing the force adhering the magnet to the housing assembly with the utilitarian desires for the magnet to be able to rotate for alignment purposes with the external magnet. That said, embodiments include an arrangement where the magnet does not rotate for alignment purposes in some other embodiments (at least for some scenarios where the external magnet is misaligned by a certain amount) when the external component is magnetically coupled to the implantable portion, but will rotate when exposed to an MRI magnetic field of 1.5, 2, 2.5, 3, 3.5 or 4 T or more at a specific angle that will cause rotation.

At least some exemplary embodiments take into account the management of the magnetic flux created by the magnet. For example, segmented plates and/or plates of different patterns, such as those of FIGs. 21, 22, 23, 25, 26 and/or plates of tiny geometry, such as that of FIG. 24. Different patterns can be used to tune for different properties. The embodiment of FIG. 22, for example, could allow the diametric magnet of the implant to prefer a certain rotational orientation, which would be utilitarian for orienting the external device (because the external deice, or at least the head piece, may have a limited range of orientations owing to the cable from the headpiece to the Behind-The-Ear device, or the orientations of the microphones of an Off- The-Ear device. Multiple axisymmetric elements can be used to disrupt the magnetic flux, limiting the amount of shunting and or reducing / minimizing any deleterious effect of the magnetic material on retention of the external component to the head. The segments can be relatively tightly spaced to minimize and/or eliminate any "cogging" effect that would impact the implant magnet (i.e., snapping to discrete angular positions). The pattern could be radial or non-radial or circumferential or non-circumferential or evenly distributed or randomly / unevenly distributed. Also, segmenting the shim is also beneficial for reducing eddy current losses in the RF link between the external device and the implanted device.

With regard to retention, it can be that in at least some embodiments, by placing the magnetic material on the skull facing side, retention of the external component is increased / is greater than that which would be the case if the magnetic material were on the skin facing side. That is, there could be a disadvantage to placing the material on the skin facing side as such might reduce retention force of the external device due to magnetic flux shunting, which can be problematic at least for recipients with high skin flap thicknesses. Conversely, placing the ferromagnetic / magnetic material on the skull side can have less of a reduction effect on retention (because it may shunt less of the magnetic flux from coupling to the external magnet). Accordingly, in an exemplary embodiment, all things being equal, for a spacing of the implant magnet and the external magnetic of the same distance, a retention force of the external magnet to the implant magnet when the implant magnet is in the magnet apparatus is 1.05, 1.1, 1.15, 1.2, 1.25, 1.3, 1.35, 1.4, 1.45 or 1.5 or more or any value or range of values therebetween in 0.01 increments greater for the same shim located on the skull facing side than for the same shim located on the skin facing side (between the two magnets). Moreover, putting the ferromagnetic / magnetic material around the periphery has the advantage of having less effect on retention.

And the embodiments that have the magnetic material at the lateral location(s) also can have utilitarian value with respect to avoiding / minimizing / reducing cogging. And these too can be tuned to achieve such.

The utilization of different size magnetic material bodies and/or a different distribution of material between the central axis of the implanted magnet and the periphery of the implant magnet can be used to tune for the amount of torque needed to rotate the magnet. For example, a central dot vs. around ring could both provide similar amounts of attraction, but it may be easier for the magnet to rotate with the central dot design, making it easier for external SP alignment. Accordingly, embodiments include a tuned magnetic material arrangement that optimizes two or more of rotatability, antirattle and external component retention.

It is again noted that while the phrase shim has been utilized herein quite frequently, embodiments are not so limited. Any disclosure of a shim herein corresponds to a disclosure of a component that has flat parallel major surfaces.

In an embodiment, there is no viscous damping fluid and/or damping material in the housing. Indeed, the fluid is a low viscosity fluid if present, so as to enhance the rotation of the magnet.

Any disclosure of a device and/or system detailed herein also corresponds to a disclosure of otherwise providing that device and/or system and/or utilizing that device and/or system.

Any disclosure of an embodiment that has a functionally corresponds to a device configured to have that functionality, and also corresponds to a method that results in the functionality / includes the actions associated with the functionality, and *vice versa.*

Any embodiment or any feature disclosed herein can be combined with any one or more or other embodiments and/or other features disclosed herein, unless explicitly indicated and/or unless the art does not enable such. Any embodiment or any feature disclosed herein can be explicitly excluded from use with any one or more other embodiments and/or other features disclosed herein, unless explicitly indicated that such is combined and/or unless the art does not enable such exclusion.

Any function or method action detailed herein corresponds to a disclosure of doing so an automated or semi-automated manner.

Any disclosure herein of any component and/or feature can be combined with any one or more of any other component and/or feature disclosure herein unless otherwise noted. Providing that the art enables such. Any disclosure herein of any component and/or feature can be explicitly excluded from combination with any one or more or any other component and/or feature disclosed herein unless otherwise noted, providing that the art enables such. Any disclosure herein of any method action includes a disclosure of a device and/or system configured to implement that method action. Any disclosure herein of a device and/or system corresponds to a disclosure of a method of utilizing that device and/or system. Any disclosure herein of a manufacturing method corresponds to a disclosure of a device and/or system that results from the manufacturing method. Any disclosure of a device and/or system corresponds to a disclosure of a method of making a device and/or system.

While various embodiments of the present invention have been described above, it should be understood that they have been presented by way of example only, and not limitation. The scope of the invention is defined by the appended claims.

## Claims

1. A device, comprising:
a housing (710, 1910), the housing (710, 1910) including a cylindrical hollow portion; and
a magnet (686, 687) in the cylindrical hollow portion, establishing a magnet-housing assembly, wherein
the device is an implantable medical device,
there is a clearance between an interior of the housing (710, 1910) and the magnet (686, 687) in a direction of a longitudinal axis of the housing (710, 1910) and/or in a direction normal to the longitudinal axis, and
the device is configured to enable the magnet (686, 687) to rotate inside the housing (710, 1910) when the magnet (686, 687) is subjected to a torque from a magnetic field;
**characterized in that**
the magnet-housing assembly includes a shim (770, 771, 774, 775, 776) made of magnetic material, the shim (770, 771, 774, 775, 776) located closer to one flat side of the cylindrical hollow portion than an opposite flat side of the cylindrical hollow portion,
the device is configured to bias the magnet (686, 687) towards the one flat side owing to magnetic attraction between the magnet (686, 687) and the shim, and
the magnetic attraction is an anti-rattle feature of the device.

2. The device of claim 1, wherein:
the device is configured to enable the magnet (686, 687) to rotate while holding the magnet (686, 687) in direct contact with the one flat side of the cylindrical hollow portion.

3. The device of claim 2, wherein the device is configured to independently permanently hold the magnet (686, 687) in direct contact with the one flat side of the cylindrical hollow portion.

4. The device of any one of the preceding claims, wherein:
the magnetic material of the shim (770, 771, 774, 775, 776) is unmagnetized and/or does not impart a significant rotational alignment force onto the magnet (686, 687).

5. The device of any one of the preceding claims, wherein;
the magnetic material of the shim (770, 771, 774, 775, 776) is located adjacent a lateral side of the magnet (686, 687).

6. The device of any one of the preceding claims, wherein
the magnetic material of the shim (770, 771, 774, 775, 776) is magnetically saturated by the magnet (686, 687).

7. The device of any one of the preceding claims, wherein:
the shim (770, 771, 774, 775, 776) is located inside the housing (710, 1910); and
a non-magnetic component (1616, 1717, 3311) is located between the magnet (686, 687) and the shim (770), inside the housing (710, 1910), that one of increases or decreases a force needed to be applied to the magnet (686, 687) to move the magnet (686, 687) in the housing (710, 1910) relative to that which would be the case in the absence of the non-magnetic component (1616, 1717).

8. The device of any one of the preceding claims, wherein:
the device is an implantable component of a sensory prosthesis, in particular of a cochlear implant, a middle ear implant, an active transcutaneous bone conduction device, a retinal implant or a vestibular implant.

9. The device of any one of the preceding claims, wherein the device has a skull facing side and a skin facing side; and
the one flat side of the cylindrical hollow portion is on the skull facing side.

10. The device of claim 1, wherein:
the magnet (686, 687) is a non-spherical magnet that has a magnetic polarity axis that is more diametrically magnetized than it is axially magnetized.

11. The device of claim 1, wherein:
a friction body is located between the magnet (686, 687) and the housing (710, 1910), in direct contact with the magnet (686, 687) in the absence of an external magnetic field.

12. The device of claim 1, wherein:
the one flat side of the cylindrical hollow portion is formed by a polymer body located within the housing (710, 1910).

13. The device of claim 1, wherein the one flat side of the cylindrical hollow portion is a low friction component of the magnet-housing assembly separate from the magnet (686, 687) and separate from a wall of the housing (710, 1910), the low friction component of the magnet-housing assembly being located between the magnet (686, 687) and the magnetic component.

14. The device of any one of the preceding claims, further comprising an RF inductance coil concentric with the magnet (686, 687).

## Patentansprüche

1. Vorrichtung, die umfasst:
ein Gehäuse (710, 1910), wobei das Gehäuse (710, 1910) einen zylindrischen hohlen Abschnitt aufweist; sowie
einen Magneten (686, 687) in dem zylindrischen holen Abschnitt, durch den eine Magnet-Gehäuseanordnung gebildet wird, wobei
die Vorrichtung eine implantierbare medizinische Vorrichtung ist,
ein Zwischenraum zwischen einer Innenseite des Gehäuses (710, 1900) und dem Magneten (686, 687) in einer Richtung einer Längsachse des Gehäuses (710, 1910) und/oder in einer Richtung senkrecht zu der Längsachse vorhanden ist, und
die Vorrichtung so ausgeführt ist, dass sie es dem Magneten (686, 687) ermöglicht, sich im Inneren des Gehäuses (710, 1910) zu drehen, wenn der Magnet (686, 687) einem Drehmoment von einem Magnetfeld ausgesetzt wird;
**dadurch gekennzeichnet, dass**
die Magnet-Gehäuseanordnung eine Scheibe (770, 771, 774, 775, 776) einschließt, die aus magnetischem Material besteht, wobei sich die Scheibe (770, 771, 774, 775, 776) näher an einer flachen Seite des zylindrischen holen Abschnitts befindet als an einer gegenüberliegenden flachen Seite des zylindrischen holen Abschnitts,
die Vorrichtung so ausgeführt ist, dass sie den Magneten (686, 687) aufgrund von magnetischer Anziehung zwischen dem Magneten (686, 687) und der Scheibe in Richtung der einen flachen Seite vorspannt, und
die magnetische Anziehung eine Klapperschutzfunktion der Vorrichtung erfüllt.

2. Vorrichtung nach Anspruch 1, wobei:
die Vorrichtung so ausgeführt ist, dass sie es dem Magneten (686, 687) ermöglicht, sich zu drehen, und dabei den Magneten (686, 687) in direktem Kontakt mit der einen flachen Seite des zylindrischen holen Abschnitts hält.

3. Vorrichtung nach Anspruch 2, wobei die Vorrichtung so ausgeführt ist, dass sie den Magneten (686, 687) unabhängig dauerhaft in direktem Kontakt mit der einen flachen Seite des zylindrischen holen Abschnitts hält.

4. Vorrichtung nach einem der vorangehenden Ansprüche, wobei:
das magnetische Material der Scheibe (770, 771, 774, 775, 776) nicht magnetisiert ist und/oder keine nennenswerte Dreh-Ausrichtkraft auf den Magneten (686, 687) ausübt.

5. Vorrichtung nach einem der vorangehenden Ansprüche, wobei:
das magnetische Material der Scheibe (770, 771, 774, 775, 776) an eine Längsseite des Magneten (686, 687) angrenzend angeordnet ist.

6. Vorrichtung nach einem der vorangehenden Ansprüche, wobei:
das magnetische Material der Scheibe (770, 771, 774, 775, 776) durch den Magneten (686, 687) magnetisch gesättigt wird.

7. Vorrichtung nach einem der vorangehenden Ansprüche, wobei:
sich die Scheibe (770, 771, 774, 775, 776) im Inneren des Gehäuses (710, 1910) befindet; und sich eine nicht-magnetische Komponente (1616, 1717, 3311) zwischen dem Magneten (686, 687) und der Scheibe (770) im Inneren des Gehäuses (710, 1910) befindet, durch die eine Kraft, die auf den Magneten (686, 687) ausgeübt werden muss, um den Magneten (686, 687) in dem Gehäuse (710, 1910) zu bewegen, relativ zu der, die beim Nichtvorhandensein der nichtmagnetischen Komponente (1616, 1717) wirken würde, zunimmt oder abnimmt.

8. Vorrichtung nach einem der vorangehenden Ansprüche, wobei:
die Vorrichtung eine implantierbare Komponente einer sensorischen Prothese, insbesondere eines Cochlea-Implantats, eines Mittelohrimplantats, einer aktiven transkutanen Knochenleitungsvorrichtung, eines Retina-Implantats oder eines Vestibularimplantats, ist.

9. Vorrichtung nach einem der vorangehenden Ansprüche, wobei die Vorrichtung eine dem Schädel zugewandte Seite sowie eine der Haut zugewandte Seite hat; und
die eine flache Seite des zylindrischen holen Abschnitts an der dem Schädel zugewandten Seite liegt.

10. Vorrichtung nach Anspruch 1, wobei:
der Magnet (686, 687) ein nicht sphärischer Magnet ist, der eine magnetische Polachse hat, die stärker diametral magnetisiert als axial magnetisiert ist.

11. Vorrichtung nach Anspruch 1, wobei:
ein Reibungskörper sich zwischen dem Magneten (686, 687) und dem Gehäuse (710, 1910) in direktem Kontakt mit dem Magneten (686, 687) beim Nichtvorhandensein eines externen Magnetfeldes befindet.

12. Vorrichtung nach Anspruch 1, wobei:
die eine flache Seite des zylindrischen hohlen Abschnitts durch einen Polymer-Körper gebildet wird, der sich im Inneren des Gehäuses (710, 1910) befindet.

13. Vorrichtung nach Anspruch 1, wobei die eine flache Seite des zylindrischen holen Abschnitts eine reibungsarme Komponente der Magnet-Gehäuseanordnung separat von dem Magneten (686, 687) und separat von einer Wand des Gehäuses (710, 1910) ist, wobei sich die reibungsarme Komponente der Magnet-Gehäuseanordnung zwischen dem Magneten (686, 687) und der magnetischen Komponente befindet.

14. Vorrichtung nach einem der vorangehenden Ansprüche, die des Weiteren eine HF-Induktionsspule konzentrisch zu dem Magneten (686, 687) umfasst.

## Revendications

1. Dispositif comprenant:
un boîtier (710, 1910), le boîtier (710, 1910) comprenant une partie cylindrique creuse; et
un aimant (686, 687) situé dans la partie cylindrique creuse, formant un ensemble aimant-boîtier, dans lequel
le dispositif est un dispositif médical implantable,
il y a un jeu entre l'intérieur du boîtier (710, 1910) et l'aimant (686, 687) dans la direction de l'axe longitudinal du boîtier (710, 1910) et/ou dans une direction normale à l'axe longitudinal, et
le dispositif est configuré pour permettre à l'aimant (686, 687) de tourner à l'intérieur du boîtier (710, 1910) lorsque l'aimant (686, 687) est soumis à un couple provenant d'un champ magnétique;
**caractérisé en ce que**
l'ensemble aimant-boîtier comprend une cale (770, 771, 774, 775, 776) en matériau magnétique, la cale (770, 771, 774, 775, 776) étant située plus près d'un côté plat de la partie creuse cylindrique que d'un côté plat opposé de la partie creuse cylindrique,
le dispositif est configuré pour solliciter l'aimant (686, 687) vers ledit côté plat grâce à l'attraction magnétique entre l'aimant (686, 687) et la cale, et
l'attraction magnétique constitue une fonction anti-vibration du dispositif.

2. Dispositif selon la revendication 1, dans lequel:
le dispositif est configuré pour permettre à l'aimant (686, 687) de tourner tout en maintenant l'aimant (686, 687) en contact direct avec ledit côté plat de la partie creuse cylindrique.

3. Dispositif selon la revendication 2, dans lequel le dispositif est configuré pour maintenir de manière indépendante et permanente l'aimant (686, 687) en contact direct avec la face plate de la partie cylindrique creuse.

4. Dispositif selon l'une quelconque des revendications précédentes, dans lequel:
le matériau magnétique de la cale (770, 771, 774, 775, 776) n'est pas magnétisé et/ou n'exerce pas de force d'alignement en rotation significative sur l'aimant (686, 687).

5. Dispositif selon l'une quelconque des revendications précédentes, dans lequel :
le matériau magnétique de la cale (770, 771, 774, 775, 776) est situé à proximité d'un côté latéral de l'aimant (686, 687).

6. Dispositif selon l'une quelconque des revendications précédentes, dans lequel
le matériau magnétique de la cale (770, 771, 774, 775, 776) est saturé magnéti-quement par l'aimant (686, 687).

7. Dispositif selon l'une quelconque des revendications précédentes, dans lequel :
la cale (770, 771, 774, 775, 776) est située à l'intérieur du boîtier (710, 1910); et
un composant non magnétique (1616, 1717, 3311) est situé entre l'aimant (686, 687) et la cale (770), à l'intérieur du boîtier (710, 1910), ce composant augmentant ou diminuant la force devant être appliquée à l'aimant (686, 687) pour déplacer l'aimant (686, 687) dans le boîtier (710, 1910) par rapport à celle qui serait nécessaire en l'absence du composant non magnétique (1616, 1717).

8. Dispositif selon l'une quelconque des revendications précédentes, dans lequel:
le dispositif est un composant implantable d'une prothèse sensorielle, en particulier d'un implant cochléaire, d'un implant de l'oreille moyenne, d'un dispositif de conduction osseuse transcutanée active, d'un implant rétinien ou d'un implant vestibulaire.

9. Dispositif selon l'une quelconque des revendications précédentes, dans lequel le dispositif comporte une face tournée vers le crâne et une face tournée vers la peau; et le côté plat de la partie cylindrique creuse se trouve sur le côté tourné vers le crâne.

10. Dispositif selon la revendication 1, dans lequel:
l'aimant (686, 687) est un aimant non sphérique qui présente un axe de polarité magnétique qui est davantage magnétisé diamétralement qu'axialement.

11. Dispositif selon la revendication 1, dans lequel:
un corps de friction est situé entre l'aimant (686, 687) et le boîtier (710, 1910), en contact direct avec l'aimant (686, 687) en l'absence de champ magnétique externe.

12. Dispositif selon la revendication 1, dans lequel:
le côté plat de la partie cylindrique creuse est formé par un corps en polymère situé à l'intérieur du boîtier (710, 1910).

13. Dispositif selon la revendication 1, dans lequel le côté plat de la partie cylindrique creuse est un composant à faible frottement de l'ensemble aimant-boîtier, séparé de l'aimant (686, 687) et séparé d'une paroi du boîtier (710, 1910), le composant à faible frottement de l'ensemble aimant-boîtier étant situé entre l'aimant (686, 687) et le composant magnétique.

14. Dispositif selon l'une quelconque des revendications précédentes, comprenant en outre une bobine d'inductance RF concentrique avec l'aimant (686, 687).
